(19) Europäisches Patentamt / European Patent Office / Office européen des brevets

(11) **EP 3 371 324 B1**

(12) **EUROPEAN PATENT SPECIFICATION**

(45) Date of publication and mention
of the grant of the patent:
**18.08.2021 Bulletin 2021/33**

(21) Application number: **16861122.6**

(22) Date of filing: **04.11.2016**

(51) Int Cl.:
*C12Q 1/70* *(2006.01)*          *C12Q 1/6883* *(2018.01)*
*G01N 33/50* *(2006.01)*

(86) International application number:
**PCT/AU2016/051052**

(87) International publication number:
**WO 2017/075666 (11.05.2017 Gazette 2017/19)**

(54) **VIRAL BIOMARKERS AND USES THEREFOR**

VIRALE BIOMARKER UND VERWENDUNGEN DAFÜR

BIOMARQUEURS VIRAUX ET UTILISATIONS ASSOCIÉES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **06.11.2015 AU 2015904558**

(43) Date of publication of application:
**12.09.2018 Bulletin 2018/37**

(73) Proprietor: **ImmuneXpress Pty Ltd
Boonah, Queensland 4310 (AU)**

(72) Inventors:
• **BRANDON, Richard Bruce
Boonah, Queensland 4310 (AU)**
• **SAMPSON, Dayle Lorand
Shoreline
Washington 98133 (US)**

(74) Representative: **Vossius & Partner
Patentanwälte Rechtsanwälte mbB
Siebertstrasse 3
81675 München (DE)**

(56) References cited:
WO-A1-2014/209238     WO-A1-2015/117204
WO-A1-2015/117204     WO-A1-2015/121605
WO-A2-2012/174282     WO-A2-2014/201516
WO-A2-2014/201516     US-A1- 2004 097 460
US-A1- 2008 070 235     US-A1- 2015 086 570

• KAWADA J. et al.: "Evaluation of Systemic Inflammatory Responses in Neonates with Herpes Simplex Virus Infection", The Journal of Infectious Diseases., vol. 190, 2004, pages 494-8, XP055371378,
• RAYBIOTECH: "RayBio® Label-based (L-Series) Human Antibody Array 1000 Membrane Kit: A combination of Human L-507 and Human L-493 Arrays - User Manual", , February 2014 (2014-02), page 15, 16, 18, 19, XP055379361, Retrieved from the Internet: URL:http://www.raybiotech.com/files/manual/Antibody-Array/AAH-BLM-1000.pdf [retrieved on 2017-02-20]
• TSALIK E.L. et al.: "An integrated transcriptome and expressed variant analysis of sepsis survival and death", Genome Medicine., vol. 6 2014, pages 3-4, XP021207766, Retrieved from the Internet: URL:https://genomemedicine.biomedcentral.com/track/pdf/10.1186/s13073-014-0111-5?site=genomemedicine.biomedcentral.com [retrieved on 2017-02-21]
• JAMEHDAR S.A. et al.: "Herpes Simplex Virus Infection in Neonates and Young Infants with Sepsis", Iran Red Crescent Medical Journal., vol. 16, no. 2 2014, XP055379371, Retrieved from the Internet: URL:https://www.ncbi.nlm.nih.gov/pmc/articles/PMC3965875/pdf/ircmj-16- 14310.pdf [retrieved on 2017-02-21]
• WALTON A.H. et al.: "Reactivation of Multiple Viruses in Patients with Sepsis", PlosOne., vol. 9, no. 6, 1 June 2014 (2014-06-01), pages 1-13, XP055540517,

**(Cont. next page)**

• **HU X. et al.: "Gene expression profiles in febrile children with defined viral and bacterial infection", Proceedings of the National Academy of Sciences of the United States of America ., vol. 110, no. 31, 2013, pages 12792-97, XP055379373,**

Remarks:
The complete document including Reference Tables and the Sequence Listing can be downloaded from the EPO website

**Description**

**FIELD OF THE INVENTION**

This invention relates generally to methods for diagnosing and/or monitoring a herpesvirus-associated systemic inflammation by measurement of a host immune response. The invention can be used for diagnosis including early diagnosis, monitoring, making treatment decisions, or management of subjects suspected of having systemic inflammation associated with a herpesvirus infection. More particularly, the present invention relates to peripheral blood RNA and protein biomarkers that are useful for specifically distinguishing between the host systemic immune response to herpesviruses as compared to the host immune response to other causes of systemic inflammation, including other types of viruses.

**BACKGROUND OF THE INVENTION**

Fever and clinical signs of systemic inflammation (or SIRS) are commonly seen in patients presenting to medical services; either in general practice clinics, outpatient clinics, emergency rooms, hospital wards or intensive care units (Rangel-Frausto et al. (1995). The natural history of the systemic inflammatory response syndrome (SIRS). A prospective study. JAMA : The Journal of the American Medical Association, 273(2), 117-123; McGowan et al. (1987). Fever in hospitalized patients. With special reference to the medical service. The American Journal of Medicine, 82(3 Spec No), 580-586; Bor et al. (1988). Fever in hospitalized medical patients: characteristics and significance. Journal of General Internal Medicine, 3(2), 119-125; Finkelstein et al. (2000). Fever in pediatric primary care: occurrence, management, and outcomes. Pediatrics, 105(1 Pt 3), 260-266).

When SIRS is the result of a confirmed infectious process it is called infection-positive SIRS (ipSIRS), otherwise known as sepsis. Within this definition lies the following assumptions; the infectious process could be local or generalized; the infection could be bacterial, fungal/yeast, viral or parasitic; the infectious process could be in an otherwise sterile body compartment. Such a definition has been updated in Levy et al. 2003 ("2001 SCCM/ESICM/ACCP/ATS/SIS International Sepsis Definitions Conference," Critical Care Medicine 31, no. 4: 1250-1256) to accommodate clinical and research use of the definition. The revised definition allows that the infection be in a sterile or non-sterile site (*e.g.*, overgrowth of a pathogen / commensal in the intestine) and that the infection can be either confirmed or suspected.

In many instances the use of the terms SIRS and sepsis, and what clinical conditions they do or do not include, are confusing in clinical situations. Such confusion leads to difficulties in clinical diagnosis and in making decisions on subsequent patient treatment and management. Difficulties in clinical diagnosis are based on the following questions: 1) what constitutes a "suspected" infection given that many body organs / sites are naturally colonized by microbes (*e.g., Escherichia coli* in the intestines, *Staphylococcus epidermidis* in skin), viruses (*e.g.,* latent viruses such as herpes) or parasites *(e.g.,* Toxoplasma, Giardia); 2) what constitutes a pathological growth of an organism in a normally non-sterile body site?; 3) what contributions to SIRS are made by a viral / microbial / parasitic co-infection in a non-sterile body site (*e.g.*, upper respiratory tract), and if such an infection is suspected then should the patient be put on antibiotics, anti-fungal, anti-viral or anti-parasitic compounds?

Patients with fever and SIRS need to be carefully assessed, and tested, to determine the cause of the presenting clinical signs as there are many possible differential diagnoses (Munro, N. (2014). Fever in acute and critical care: a diagnostic approach. AACN Adv Crit Care 25: 237-248). Possible differential diagnoses include infection (bacterial, fungal, viral, parasitic), trauma, allergy, drug reaction, autoimmunity, surgery, neutropenia, cancer, metabolic disorders, clotting disorders. Patients with fever and SIRS caused by bacterial or fungal infection often require immediate medical attention and it is therefore important to quickly differentiate such patients. Patients with fever and SIRS caused by viral infection need to be further assessed to determine 1) the degree of systemic inflammation due to viral infection, 2) the degree of involvement of microbes (commensals, microbiome, pathogens) to systemic inflammation 3) contributions that each of viruses, microbes and sterile injury are making to systemic inflammation 4) likelihood of the patient rapidly deteriorating. The results of such an assessment aids clinicians in making appropriate management and treatment decisions. Furthermore, the use of biomarkers has been recently suggested for analyzing SIRS and sepsis and providing more detailed differential diagnoses (WO/2014/201516, WO/2015/117204, WO/2015/121605, and WO/2014/209238).

Herpesviruses are a phylogenetically ancient family of large DNA viruses for which there is a wealth of scientific literature because they cause widespread disease in both humans and animals. The spread of herpesviruses from one generation to the next would appear to have occurred successfully for tens to hundreds of millions of years and the three sub-families of herpesviridae arose 180-220 million years ago (McGeoch DJ., Cook S., Dolan A., Jamieson FE., Telford EA. Molecular phylogeny and evolutionary timescale for the family of mammalian herpesviruses. 1995. Journal of Molecular Biology. 247: 443). This co-existence of virus with host involves a close accommodation with the host immune system (Hill, AB. Mechanisms of interference with the MHC class I-restricted pathway of antigen presentation by herpesviruses. Immunology and Cell Biology. 1996. 74: 523-526).

There are more than 80 known types of herpesvirus, but only eight are known to cause disease in humans. These are divided into three sub-families listed below:

Alphaherpesvirinae: human herpesvirus 1, 2 and 3 (HHV-1, -2, -3);

Betaherpesvirinae: human herpesvirus 5, 6 and 7 (HHV-5, -6, -7); and

Gammaherpesvirinae: human herpesvirus 4 and 8 (HHV-4, -8).

Of these, the most important herpesviruses with respect to human disease are: *Herpes simplex virus* (HSV1 and 2, or HHV1 and 2), *Varicella-Zoster virus* (VZV, or HHV3), *Epstein-Barr virus* (EBV, or HHV4) and *cytomegalovirus* (CMV, or HHV5).

Key features of all herpesvirus infections and diagnosis include:

- Most people are exposed to, and become infected with, at least one herpesvirus type early in life;

- An infection with a herpesvirus is permanent for which there is no known cure;

- Following an initial infection, to which the host immune system responds and recovers, herpesviruses become latent (that is, exist in a dormant form in particular cells);

- Latent herpesvirus infections can become active herpesvirus infections (active meaning the immune system is actively and demonstrably responding to the presence of a herpesvirus) when the immune system of the host is compromised; and

- Diagnosis of a herpesvirus infection is often based on the presence of pathognomonic clinical signs by which time the efficacy of treatment with anti-viral compounds is diminished.

Confirmatory diagnosis of a herpesvirus infection is usually achieved by measurement of an IgM / IgG serum ratio, comparison of IgG serum concentrations from two sampling time points, or determination of IgG avidity (poor avidity correlates to an early infection), and / or the detection of herpes-virus specific DNA or mRNA transcripts. Differentiating latent herpesvirus infection from active herpesvirus infection is more challenging and methods to achieve this include; detection of "late" virus-specific mRNA transcripts, and comparison of the levels of virus antigens able to be detected in whole blood versus plasma. In the latter instance, latent infection will result in restriction of antigen to whole blood rather than plasma (Ross SA, Novak Z, Pati S, Boppana SB. Diagnosis of Cytomegalovirus Infections. Infectious disorders drug targets. 2011;11(5):466-474).

Oral anti-viral medications such as acyclovir, famciclovir, or valacyclovir, which are effective and specific inhibitors of herpes viral DNA polymerase, have been developed to ameliorate the symptoms of herpes infections. These medications can also be used to treat an outbreak, or for suppressing herpesvirus recurrences. Lower doses may be helpful in reducing the number of herpesvirus attacks in people with frequent recurrences. Such medications are most effective early in the course of an infection and before peak clinical signs occur.

Physiological stress (such as heavy exercise, concurrent disease, mental stress), or where the immune system is compromised (for example HIV infection, immunosuppressive therapy (anti-organ-rejection therapy, steroids, anti-cancer therapy etc.), sepsis, other viral infections, pregnancy, can lead to susceptibility to primary infection or reactivation of herpesviruses leading to chronic symptoms of infection, or crossing of the placenta by herpesviruses to infect the fetus (Bustamante, C. I., & Wade, J. C. (1991) Herpes simplex virus infection in the immunocompromised cancer patient. Journal of Clinical Oncology : Official Journal of the American Society of Clinical Oncology, 9(10), 1903-1915; Walton, A. H., Muenzer, J. T., Rasche, D., Boomer, J. S., Sato, B., Brownstein, B. H., et al. (2014). Reactivation of multiple viruses in patients with sepsis. PLoS ONE, 9(2), e98819; Blyth, W. A., Harbour, D. A., & Hill, T. J. (1980). Effect of immunosuppression on recurrent herpes simplex in mice. Infection and Immunity, 29(3), 902-907; Muller, W., Jones, C., & Koelle, D. (2010). Immunobiology of Herpes Simplex Virus and Cytomegalovirus Infections of the Fetus and Newborn. Current Immunology Reviews, 6(1), 38-55).

With respect to the host immune response to herpesvirus infection, it is known that an innate immune response is elicited followed by an adaptive immune response. The innate immune response is antigen-independent and consists primarily of cytokine production, complement activation, macrophage and natural killer cell activation and apoptotic cell death. A critical element in both the innate and adaptive immune response to viral infection is the production of interferons (IFNs) by a variety of cell types and in response to a variety of viral antigens (Mossman KL. Activation and inhibition of virus and interferon: The herpesvirus story. 2002. Viral Immunology. 15(1): 3-15.; Lebel F and Hirsch MS. 1985. The role of interferon in immunity and prophylaxis. Pp 371-393, in Roizman, B., and Lopez, C. (eds.), The Herpesviruses, vol 4, Plenum Press, New York.). The main inducing element of IFNs is double-stranded RNA (dsRNA) which is also produced by DNA viruses. However, viral glycoproteins have also been demonstrated to induce specialised leukocytes (interferon producing cells, IPC) to produce IFNs. The IFNs themselves are not antiviral but they induce numerous interferon-stimulated genes (ISGs) that effectively limit virus replication and spread. Such genes, amongst hundreds, include the Janus kinase and Stat families of signal transducers and activators of transcription (de Veer MJ., Holko M., Frevel M., Walker E., Der S., Paranjape JM., Silverman RH., Williams BRG. Functional classification of interferon-stimulated genes identified using microarrays. 22001. Journal of Leukocyte Biology. 69: 912-920). However, such a host interferon response is not specific to viruses since it is known that other pathogens, including parasites and bacteria, can elicit a similar response (Herrera, V., Perry, S., Parsonnet, J., & Banaei, N. (2011). Clinical Application and Limitations of Interferon- Release Assays for the Diagnosis of Latent Tuberculosis Infection. Clinical Infectious Diseases : An Official Publication of the Infectious Diseases Society of America, 52(8), 1031-1037; Yarovinsky, F. (2014). Innate immunity to Toxoplasma gondii infection. Nature Reviews Immunology, 14(2), 109-121).

The analysis of gene expression products for diagnostic purposes requires identification of one or more genes that can be used to generate a signature for use in distinguishing between different conditions. However, such identification can require the analysis of many gene expression products, which can be mathematically complex, computationally expensive and hence difficult to implement. Much of the biomarker discovery process is devoted to identifying a subset of the data that may have relevant import, from which a signature is derived using a combination of these values to produce a model for diagnostic or prognostic use.

Measurement of specific host immune responses to herpesviruses using gene expression is known (Hu, X., Yu, J., Crosby, S. D., & Storch, G. A. (2013). Gene expression profiles in febrile children with defined viral and bacterial infection. Proceedings of the National Academy of Sciences, 110(31), 12792-12797; Halminen, M., Ilonen, J., Julkunen, I., Ruuskanen, O., Simell, O., & Makela, M. J. (1997). Expression of MxA protein in blood lymphocytes discriminates between viral and bacterial infections in febrile children. Pediatric Research, 41(5), 647-650; WO/2006/015452). However, conventional combinations of biomarkers use a relatively large number of biomarkers, which in turn makes tests expensive to perform, limiting their use in practice.

A need, therefore, exists for better ways of detecting the presence of herpesvirus infections, particularly systemic inflammation associated with herpesvirus infections, to permit early diagnosis, monitoring, making treatment decisions, or management of subjects having, or suspected of having, a systemic inflammation.

## SUMMARY OF THE INVENTION

The present invention arises from the determination that certain host response peripheral blood RNA transcripts (RNA markers) are commonly, specifically and differentially expressed across different herpesvirus infections in the presence of systemic inflammation. Such RNA transcripts (biomarkers) are useful for diagnosis early in the infective process and over the course of infection. These biomarkers are useful therefore in early diagnosis, diagnosis, monitoring, prognosis and determination of severity of a systemic inflammatory response associated with a herpesvirus infection. In particular, based on the demonstrated specificity to herpesvirus-associated systemic inflammation, such biomarkers are useful in determining the etiology of a systemic inflammatory response when caused by a herpesvirus infection.

Based on this determination, the present inventors have developed various methods, apparatus, compositions, and kits, which take advantage of differentially expressed biomarkers, including ratios thereof (derived biomarkers), to determine the presence, absence or degree of herpesvirus-associated systemic inflammatory response syndrome (HVaSIRS) in subjects presenting with fever or clinical signs of systemic inflammation. These methods, apparatus, compositions, and kits may represent a significant advance over prior art processes and products, which have not been able to: 1) distinguish HVaSIRS from other etiologies of systemic inflammation, including other viruses, bacteria, trauma, autoimmune disease; and/or 2) determine the contribution of a herpesvirus infection (if any) to the presenting clinical signs and pathology.

Accordingly, the present invention relates to a method for determining an indicator used in assessing a likelihood of a subject having a presence, absence or degree of herpesvirus-associated systemic inflammatory response syndrome (HVaSIRS), wherein the subject has at least one clinical sign of SIRS, the method comprising: (a) determining a pair of biomarker values, each biomarker value being a value measured or derived for at least one corresponding HVaSIRS biomarker and that is at least partially indicative of a concentration of the HVaSIRS biomarker in a sample taken from the subject; (b) determining a derived biomarker value using the pair of biomarker values, the derived biomarker value being indicative of a ratio of concentrations of the pair of HVaSIRS biomarkers; and (c) determining the indicator using the derived biomarker value, wherein one HVaSIRS biomarker of the biomarker pair is selected from Group A HVaSIRS biomarkers and the other is selected from Group B HVaSIRS biomarkers, wherein an individual Group A HVaSIRS biomarker is an expression product of a gene selected from the group consisting of: CCL5, GZMA, TGFBR3, CD8A, PABPC4, CHST12, NKG7, SYT11, RARRES3, RPL12, KIF13B, ICAM2, ADRB2, MRPS18B, RPL8, RPS11, SLAMF7, PRKD2, FDFT1, ATP2B4, RPL38, BTG1, DNPEP, NAP1L1, RABGAP1L and ACAT1 and wherein an individual Group B HVaSIRS biomarker is an expression product of a gene selected from the group consisting of: FLNB, EPHX2, CCR7, MAL, LEF1, TRAF3IP3, PIK3IP1, ITM2C, LDLRAP1, NOSIP, ARHGEF18, SATB1, ITPKB, TBC1D4, FLT3LG, DHRS3, RBM4B, HSD17B8, MLLT11, TMC6, PLEKHB1, ALDOC, RPL22, EEF1G, TOP2B, BACH2, BNIP3, ADSL, GRWD1, RPL10A, TMEM134, ETS1, CRY2, SERBP1, RPS5, ZW10, EIF4B, PEX11B, PHB2, SERPINE2, SRM, LSM7, RPL15, RBM17, DGKA, PTOV1, HIC2, NET1, XPC, SLC5A6, SERTAD2, TMEM39B, RPL27, RPL11, C6orf48, WDR61, PFKP, EIF2S3, NECAP2, RPL30, NACA, NGFRAP1, RPS9, RALA, COX4I1, UBE2D2 and VPS35.

Thus, in one aspect, the present invention provides methods for determining an indicator used in assessing a likelihood of a subject having a presence, absence or degree of HVaSIRS. These methods generally comprise, consist or consist essentially of: (1) determining biomarker values that are measured or derived for at least two (e.g., 2, 3, 4, 5, 6, 7, 8, 9, 10, or more) corresponding HVaSIRS biomarkers in a sample taken from the subject and that are at least partially indicative of respective levels of the HVaSIRS biomarkers in the sample; and (2) determining the indicator using the biomarker values. Suitably, the subject has at least one clinical sign of SIRS. The at least two HVaSIRS biomarkers are suitably not biomarkers of at least one other SIRS condition (e.g., 1, 2, 3, 4 or 5 other SIRS conditions) selected from the group consisting of: bacterium associated SIRS (BaSIRS), autoimmune disease associated SIRS (ADaSIRS), cancer associated SIRS (CaSIRS), trauma associated SIRS and a virus-associated SIRS other than HVaSIRS (also referred to herein as a non-HVVaSIRS virus associated SIRS) (nHVVaSIRS). The sample is suitably a biological sample, representative examples of which include blood samples including peripheral blood samples, and leukocyte samples.

The at least two HVaSIRS biomarkers may be expression products of genes selected from the group consisting of: *ACAT1, ACSL1, ADCY3, ADCY7, ADRB2, ADSL, AKAP11, ALDOC, ARHGEF18, ARNTL, ARSB, ATP2B4, BACH2, BCL11A, BNIP3, BTG1, BTG3, C1QB, C6orf48, CAMK1D, CASP4, CASZ1, CCL5, CCR7, CD300A, CD37, CD79A, CD79B, CD8A, CD93, CHST12, COL17A1, COX4I1, CRY2, CST7, CYB561, CYLD, DGKA, DHRS3, DNPEP, DPEP2, EEF1G, EHD1, EIF2AK2, EIF2S3, EIF4B, EPHX2, ETS1, FAM129A, FDFT1, FLNB, FLT3LG, GALNT10, GDPD5, GFI1, GNG7, GPR162, GPR56, GRWD1, GZMA, HERC5, HERC6, HIC2, HSD17B8, ICAM2, IFI16, IFI44, IFITM1, IKBKG, IL4R, ITM2C, ITPKB, KIF13B, KPNB1, LAMA5, LBH, LDLR, LDLRAP1, LEF1, LRMP, LSM7, LTBP3, MAL, MED25, MLLT11, MRPS18B, MZF1, NAALADL1, NACA, NAP1L1, NECAP2, NET1, NGFRAP1, NKG7, NOSIP, OASL, PABPC4, PARP3, PEX11B, PFKL, PFKP, PHB2, PIK3IP1, PLEKHB1, POP5, PPP2R2B, PRDM1, PRKD2, PTOV1, PXN, RAB3GAP1, RABGAP1L, RALA, RARRES3, RBM17, RBM4B, REC8, RPAIN, RPL10A, RPL11, RPL12, RPL15, RPL22, RPL27, RPL30, RPL36, RPL38, RPL8, RPS11, RPS5, RPS9, RUNX3, SAC3D1, SATB1, SERBP1, SERPINE2, SERTAD2, SLAMF7, SLC5A6, SPINT2, SQRDL, SREBF1, SRM, SVIL, SYNE2, SYPL1, SYT11, TANK, TAP1, TBC1D4, TBK1, TCF4, TCL1A, TDRD7, TGFBR3, TMC6, TMEM134, TMEM39B, TMUB2, TOP2B, TPK1, TPST2, TRAF3IP3, TXN, UBE2D2, UBE2L6, UBR2, VASH1, VPREB3, VPS35, WDR61, XPC, ZBP1 and ZW10.* Non-limiting examples of nucleotide sequences for these HVaSIRS biomarkers are listed in SEQ ID NOs: 1-174 (see, Table 11). Non-limiting examples of amino acid sequences for these HVaSIRS biomarkers are listed in SEQ ID NOs: 175-348 (see, Table 12). In illustrative examples, an individual HVaSIRS biomarker is selected from the group consisting of: (a) a polynucleotide expression product comprising a nucleotide sequence that shares at least 70% (or at least 71% to at least 99% and all integer percentages in between) sequence identity with the sequence set forth in any one of SEQ ID NO: 1-174, or a complement thereof; (b) a polynucleotide expression product comprising a nucleotide sequence that encodes a polypeptide comprising the amino acid sequence set forth in any one of SEQ ID NO: 175-348; (c) a polynucleotide expression product comprising a nucleotide sequence that encodes a polypeptide that shares at least 70% (or at least 71% to at least 99% and all integer percentages in between) sequence similarity or identity with at least a portion of the sequence set forth in SEQ ID NO: 175-348; (d) a polynucleotide expression product comprising a nucleotide sequence that hybridizes to the sequence of (a), (b), (c) or a complement thereof, under medium or high stringency conditions; (e) a polypeptide expression product comprising the amino acid sequence set forth in any one of SEQ ID NO: 175-348; and (f) a polypeptide expression product comprising an amino acid sequence that shares at least 70% (or at least 71% to at least 99% and all integer percentages in between) sequence similarity or identity with the sequence set forth in any one of SEQ ID NO: 174-348.

The HVaSIRS biomarkers of the present invention have strong diagnostic performance when combined with one or more other HVaSIRS biomarkers. In some embodiments, pairs of biomarkers are used to determine the indicator. In illustrative examples of this type, one biomarker of a biomarker pair is selected from Group A HVaSIRS biomarkers and the other is selected from Group B HVaSIRS biomarkers, wherein an individual Group A HVaSIRS biomarker is an expression product of a gene selected from the group consisting of: *GZMA, TGFBR3, CD8A, PABPC4, CHST12, NKG7, SYT11, RARRES3, RPL12, KIF13B, ICAM2, ADRB2, MRPS18B, RPL8, RPS11, SLAMF7, PRKD2, FDFT1, ATP2B4, RPL38, BTG1, DNPEP, NAP1L1, RABGAP1L and ACAT1* and wherein an individual Group B HVaSIRS biomarker is an expression product of a gene selected from the group consisting of: *EPHX2, CCR7, MAL, LEF1, TRAF3IP3, PIK3IP1, ITM2C, LDLRAP1, NOSIP, ARHGEF18, SATB1, ITPKB, TBC1D4, FLT3LG, DHRS3, RBM4B, HSD17B8, MLLT11, TMC6, PLEKHB1, ALDOC, RPL22, EEF1G, TOP2B, BACH2, BNIP3, ADSL, GRWD1, RPL10A, TMEM134, ETS1, CRY2, SERBP1, RPS5, ZW10, EIF4B, PEX11B, PHB2, SERPINE2, SRM, LSM7, RPL15, RBM17, DGKA, PTOV1,*

*HIC2, NET1, XPC, SLC5A6, SERTAD2, TMEM39B, RPL27, RPL11, C6orf48, WDR61, PFKP, EIF2S3, NECAP2, RPL30, NACA, NGFRAP1, RPS9, RALA, COX4I1, UBE2D2 and VPS35.*

In other illustrative examples, one biomarker of a biomarker pair is selected from Group C HVaSIRS biomarkers and the other is selected from Group D HVaSIRS biomarkers, wherein an individual Group C HVaSIRS biomarker is an expression product of a gene selected from the group consisting of: *RUNX3, GPR56, LBH, POP5, SYNE2, TPST2, RPL36, GALNT10, GFI1, MZF1, SREBF1, AKAP11, CAMK1D, CYB561, BTG3, CST7, SVIL, EIF2AK2, ARNTL, ZBP1, ADCY7, SYPL1 and HERC6,* and wherein an individual Group D HVaSIRS biomarker is an expression product of a gene selected from the group consisting of: *CD79B, CD79A, VPREB3, TCL1A, LAMA5, BCL11A, TCF4, DPEP2, RPAIN, RAB3GAP1, MED25 and LRMP.*

In other illustrative examples, one biomarker of a biomarker pair is selected from Group E HVaSIRS biomarkers and the other is selected from Group F HVaSIRS biomarkers, wherein an individual Group E HVaSIRS biomarker is an expression product of a gene selected from the group consisting of: *TMUB2, ARSB, C1QB, PFKL, EHD1, PRDM1, IKBKG, TAP1, TANK, PARP3, OASL, ADCY3, FAM129A, SQRDL, UBR2, IFITM1, CYLD, ACSL1, PXN, UBE2L6, IL4R, COL17A1, TXN, CD300A, LDLR, TBK1, HERC5, TDRD7, KPNB1, IFI44, CASP4 and IFI16* and wherein an individual Group F HVaSIRS biomarker is an expression product of a gene selected from the group consisting of: *GPR162, NAALADL1, LTBP3, REC8, CD37, TPK1, GDPD5, SAC3D1, SPINT2 and CD93.*

Biomarker values may be measured or derived for a Group A HVaSIRS biomarker and for a Group B HVaSIRS biomarker, and the indicator may be determined by combining the biomarker values. Furthermore, biomarker values may be measured or derived for a Group C HVaSIRS biomarker and for a Group D HVaSIRS biomarker, and the indicator may be determined by combining the biomarker values. Furthermore, biomarker values may be measured or derived for a Group E HVaSIRS biomarker and for a Group F HVaSIRS biomarker, and the indicator may be determined by combining the biomarker values. Furthermore, biomarker values may be measured or derived for a Group A HVaSIRS biomarker, for a Group B HVaSIRS biomarker, for a Group C HVaSIRS biomarker and for a Group D HVaSIRS biomarker, and the indicator may be determined by combining the biomarker values. Furthermore, biomarker values may be measured or derived for a Group A HVaSIRS biomarker, for a Group B HVaSIRS biomarker, for a Group C HVaSIRS biomarker, for a Group D HVaSIRS biomarker, for a Group E HVaSIRS biomarker, for a Group F HVaSIRS biomarker, and the indicator may be determined by combining the biomarker values. Suitably, in the above embodiments, the methods comprise combining the biomarker values using a combining function, wherein the combining function is at least one of: an additive model; a linear model; a support vector machine; a neural network model; a tree-learning method (*e.g.*, random forest model); a regression model; a genetic algorithm; an annealing algorithm; a weighted sum; a nearest neighbor model; an ensemble method (*e.g.*, bagging, boosting weighted averaging); and a probabilistic model.

In some embodiments, the methods comprise: (a) determining a pair of biomarker values, each biomarker value being a value measured or derived for at least one corresponding HVaSIRS biomarker; (b) determining a derived biomarker value using the pair of biomarker values, the derived biomarker value being indicative of a ratio of concentrations of the pair of HVaSIRS biomarkers; and determining the indicator using the derived marker value. In illustrative examples of this type, biomarker values are measured or derived for a Group A HVaSIRS biomarker and for a Group B HVaSIRS biomarker to obtain the pair of biomarker values and the derived biomarker value is determined using the pair of biomarker values. In other illustrative examples, biomarker values are measured or derived for a Group C HVaSIRS biomarker and for a Group D HVaSIRS biomarker to obtain the pair of biomarker values and the derived biomarker value is determined using the pair of biomarker values. In other illustrative examples, biomarker values are measured or derived for a Group E HVaSIRS biomarker and for a Group F HVaSIRS biomarker to obtain the pair of biomarker values and the derived biomarker value is determined using the pair of biomarker values.

In some embodiments, the methods comprise: (a) determining a first derived biomarker value using a first pair of biomarker values, the first derived biomarker value being indicative of a ratio of concentrations of first and second HVaSIRS biomarkers; (b) determining a second derived biomarker value using a second pair of biomarker values, the second derived biomarker value being indicative of a ratio of concentrations of third and fourth HVaSIRS biomarkers; (c) determining a third derived biomarker value using a third pair of biomarker values, the third derived biomarker value being indicative of a ratio of concentrations of fifth and sixth HVaSIRS biomarkers; and (d) determining the indicator by combining the first, second and third derived biomarker values. Suitably, the first HVaSIRS biomarker is selected from Group A HVaSIRS biomarkers, the second HVaSIRS biomarker is selected from Group B HVaSIRS biomarkers, the third HVaSIRS biomarker is selected from Group C HVaSIRS biomarkers, the fourth HVaSIRS biomarker is selected from Group D HVaSIRS biomarkers, the fifth HVaSIRS biomarker is selected from Group E HVaSIRS biomarkers, and the sixth HVaSIRS biomarker is selected from Group F HVaSIRS biomarkers. In illustrative examples of this type, the methods comprise combining the biomarker values using a combining function, wherein the combining function is at least one of: an additive model; a linear model; a support vector machine; a neural network model; a tree-learning method (*e.g.*, random forest model); a regression model; a genetic algorithm; an annealing algorithm; a weighted sum; a nearest neighbor model; an ensemble method (*e.g.*, bagging, boosting weighted averaging); and a probabilistic model.

Suitably, in embodiments that utilize pairs of HVaSIRS biomarkers as broadly described above and elsewhere herein, an individual pair of HVaSIRS biomarkers has a mutual correlation in respect of HVaSIRS that lies within a mutual correlation range, the mutual correlation range being between $\pm0.9$ (or between $\pm0.8$, $\pm0.7$, $\pm0.6$, $\pm0.5$, $\pm0.4$, $\pm0.3$, $\pm0.2$ or $\pm0.1$) and the indicator has a performance value greater than or equal to a performance threshold representing the ability of the indicator to diagnose the presence, absence or degree of HVaSIRS, wherein the performance threshold is indicative of an explained variance of at least 0.3. In illustrative examples of this type, an individual HVaSIRS biomarker has a condition correlation with the presence, absence or degree of HVaSIRS that lies outside a condition correlation range, wherein the condition correlation range is between $\pm 0.3$. In other illustrative examples, an individual HVaSIRS biomarker has a condition correlation with the presence, absence or degree of HVaSIRS that lies outside a condition correlation range, wherein the condition correlation range is at least one of $\pm0.9$, $\pm0.8$, $\pm0.7$, $\pm0.6$, $\pm0.5$ or $\pm0.4$. In specific embodiments, the performance threshold is indicative of an explained variance of at least one of 0.4, 0.5, 0.6, 0.7, 0.8 and 0.9.

In certain embodiments that utilize pairs of HVaSIRS biomarkers as broadly described above and elsewhere herein the Group A HVaSIRS biomarker is suitably an expression product of *CCL5*, the Group B HVaSIRS biomarker is suitably an expression product of *FLNB*, the Group C HVaSIRS biomarker is suitably an expression product of *PPP2R2B*, the Group D HVaSIRS biomarker is suitably an expression product of *GNG7*, the Group E HVaSIRS biomarker is suitably an expression product of *CASZ1*, and the Group F HVaSIRS biomarker is suitably an expression product of *VASH1*.

The herpesvirus associated with the HVaSIRS is suitably selected from any herpesvirus that preferably affects mammals, which is capable of inducing at least one of the clinical signs of SIRS, illustrative examples of which include human herpesviruses 1-8 (HHV1-8) (*Herpes simplex virus* 1 and 2, Varicella Zoster *virus, Epstein-Barr virus, Cytomegalovirus, Roseolovirus, Herpes simplex virus 7,* and *Kaposi's sarcoma-associated virus*).

The invention further relates to the use of an apparatus for determining an indicator used in assessing a likelihood of a subject having a presence, absence or degree of HVaSIRS, the apparatus comprises at least one electronic processing device that:

- determines a pair of biomarker values, each biomarker value being a value measured or derived for at least one corresponding HVaSIRS biomarker of a sample taken from the subject and being at least partially indicative of a concentration of the HVaSIRS biomarker in the sample, wherein one HVaSIRS biomarker of the biomarker pair is selected from Group A HVaSIRS biomarkers and the other is selected from Group B HVaSIRS biomarkers;

- determines a derived biomarker value using the pair of biomarker values, the derived biomarker value being indicative of a ratio of concentrations of the pair of HVaSIRS biomarkers; and

- determines the indicator using the derived biomarker value.

Disclosed in the context of the present invention is an apparatus for determining an indicator used in assessing a likelihood of a subject having a presence, absence or degree of HVaSIRS. This apparatus generally comprises at least one electronic processing device that:

determines a pair of biomarker values, each biomarker value being a value measured or derived for at least one corresponding HVaSIRS biomarker, as broadly described above and elsewhere herein, of a sample taken from the subject and being at least partially indicative of a concentration of the HVaSIRS biomarker in the sample;

determines a derived biomarker value using the pair of biomarker values, the derived biomarker value being indicative of a ratio of concentrations of the pair of HVaSIRS biomarkers; and

determines the indicator using the derived biomarker value.

Further disclosed in the context of the present invention are compositions for determining an indicator used in assessing a likelihood of a subject having a presence, absence or degree of HVaSIRS. These compositions generally comprise, consist or consist essentially of at least one pair of cDNAs and at least one oligonucleotide primer or probe that hybridizes to an individual one of the cDNAs, wherein the at least one pair of cDNAs is selected from pairs of cDNAs including a first pair, a second pair and a third pair of cDNAs, wherein the first pair comprises a Group A HVaSIRS biomarker cDNA and a Group B HVaSIRS biomarker cDNA, and wherein the second pair comprises a Group C HVaSIRS biomarker cDNA and a Group D HVaSIRS biomarker cDNA, and wherein the third pair comprises a Group E HVaSIRS biomarker cDNA and a Group F HVaSIRS biomarker cDNA. Suitably, the compositions comprise a population of cDNAs corresponding to mRNA derived from a cell or cell population. The cell may be a cell of the immune system, suitably a leukocyte. The cell population may be blood, suitably peripheral blood. The at least one oligonucleotide primer or probe may be hybridized to an individual one of the cDNAs. The composition may further comprise a labeled reagent for detecting the cDNA. In illustrative examples of this type, the labeled reagent is a labeled said at least one oligonucleotide primer or probe. Furthermore, the labeled reagent may be a labeled said cDNA. Suitably, the at least one oligonucleotide primer or probe is in a form other than a high density array.

The invention also relates to the use of a kit for determining an indicator indicative of the likelihood of the presence, absence or degree of HVaSIRS, the kit comprising at least one pair of reagents selected from reagent pairs including a first pair of reagents, a second pair of reagents and a third pair of reagents, wherein the first pair of reagents comprises (i) a reagent that allows quantification of a Group A HVaSIRS biomarker; and (ii) a reagent that allows quantification of a Group B HVaSIRS biomarker, wherein the second pair of reagents comprises: (iii) a reagent that allows quantification of a Group C HVaSIRS biomarker; and (iv) a reagent that allows quantification of a Group D HVaSIRS biomarker wherein the third pair of reagents comprises: (v) a reagent that allows quantification of a Group E HVaSIRS biomarker; and (vi) a reagent that allows quantification of a Group F HVaSIRS biomarker.

Further disclosed in the context of the present invention are kits for determining an indicator indicative of the likelihood of the presence, absence or degree of HVaSIRS. The kits generally comprise, consist or consist essentially of at least one pair of reagents selected from reagent pairs including a first pair of reagents, a second pair of reagents and a third pair of reagents, wherein the first pair of reagents comprises (i) a reagent that allows quantification of a Group A HVaSIRS biomarker; and (ii) a reagent that allows quantification of a Group B HVaSIRS biomarker, wherein the second pair of reagents comprises: (iii) a reagent that allows quantification of a Group C HVaSIRS biomarker; and (iv) a reagent that allows quantification of a Group D HVaSIRS biomarker, and wherein the third pair of reagents comprises: (v) a reagent that allows quantification of a Group E HVaSIRS biomarker; and (vi) a reagent that allows quantification of a Group F HVaSIRS biomarker.

Further disclosed are methods for managing a subject with HVaSIRS. These methods generally comprise, consist or consist essentially of: exposing the subject to a treatment regimen for treating HVaSIRS, or avoiding exposing the subject to a treatment regimen for treating a SIRS other than HVaSIRS based on an indicator obtained from an indicator-determining method, wherein the indicator is indicative of the presence of HVaSIRS in the subject, and wherein the indicator-determining method is an indicator-determining method as broadly described above and elsewhere herein. The methods may further comprise taking a sample from the subject and determining an indicator indicative of the likelihood of the presence, absence or degree of HVaSIRS using the indicator-determining method. The methods may further comprise sending a sample taken from the subject to a laboratory at which the indicator is determined according to the indicator-determining method. The methods suitably further comprise receiving the indicator from the laboratory.

Also disclosed are methods of monitoring the efficacy of a particular treatment regimen in a subject towards a desired health state (e.g., absence of HVaSIRS). These methods generally comprise, consist or consist essentially of: (1) determining biomarker values that are measured or derived for at least two (e.g., 2, 3, 4, 5, 6, 7, 8, 9, 10, or more) corresponding HVaSIRS biomarkers as broadly defined above in a sample taken from the subject and that are at least partially indicative of respective levels of the HVaSIRS biomarkers in the sample, wherein the sample is taken after treatment of the subject with the treatment regimen; (2) determining an indicator using the biomarker values, wherein the indicator is used in assessing a likelihood of the subject having a presence, absence or degree of HVaSIRS, and (3) assessing the likelihood of the subject having a presence, absence or degree of HVaSIRS using the indicator to thereby determine whether the treatment regimen is effective for changing the health status of the subject to the desired health state.

Furhter disclosed are methods of determining whether a treatment regimen is effective for treating a subject with HVaSIRS. These methods generally comprise, consist or consist essentially of: (a) correlating biomarker values that are measured or derived for at least two (e.g., 2, 3, 4, 5, 6, 7, 8, 9, 10, or more) corresponding HVaSIRS biomarkers as broadly defined above with an effective treatment regimen; (b) determining respective biomarker values that are measured or derived for the at least two corresponding HVaSIRS biomarkers in a sample taken from the subject after treatment with the treatment regimen; (c) determining an indicator using the biomarker values, wherein the indicator is used in assessing a likelihood of the subject having a presence, absence or degree of HVaSIRS, (d) assessing the likelihood of the subject having a presence, absence or degree of HVaSIRS using the indicator to thereby determine whether the treatment regimen is effective for treating HVaSIRS in the subject.

Also disclosed are methods of correlating biomarker values that are measured or derived for at least two (e.g., 2, 3, 4, 5, 6, 7, 8, 9, 10, or more) corresponding HVaSIRS biomarker as broadly defined above with a positive or negative response to a treatment regimen. These methods generally comprise, consist or consist essentially of: (a) determining respective biomarker values for the at least two corresponding HVaSIRS biomarkers in a sample taken from a subject with HVaSIRS following commencement of the treatment regimen, wherein the biomarker values are at least partially indicative of respective levels of the HVaSIRS biomarkers in the sample; and (c) correlating the sample HVaSIRS biomarker values with a positive or negative response to the treatment regimen.

Further disclosed are methods of determining a positive or negative response to a treatment regimen by a subject with HVaSIRS. These methods generally comprise, consist or consist essentially of: (a) correlating reference biomarker values that are measured or derived for at least two (e.g., 2, 3, 4, 5, 6, 7, 8, 9, 10, or more) corresponding HVaSIRS biomarker as broadly defined above with a positive or negative response to the treatment regimen; and (b) determining respective biomarker values for the at least two corresponding HVaSIRS biomarkers in a sample taken from a subject with HVaSIRS, wherein the sample biomarker values indicate whether the subject is responding to the treatment regimen.

The methods of determining a positive or negative response to a treatment regimen may further comprise: (i) determining first sample biomarker values for the at least two corresponding HVaSIRS biomarkers as broadly defined above in a sample taken from the subject prior to commencing the treatment regimen; and (ii) comparing the first sample biomarker values with second sample biomarker values for the at least two corresponding HVaSIRS biomarkers taken from the subject after commencement of the treatment regimen, to thereby determine a positive or negative response to the treatment regimen.

Also disclosed are methods of treating, preventing or inhibiting the development of HVaSIRS in a subject. These methods generally comprise, consist or consist essentially of: exposing the subject to a treatment regimen for treating HVaSIRS, or avoiding exposing the subject to a treatment regimen for treating a SIRS other than HVaSIRS based on an indicator obtained from an indicator-determining method, the indicator-determining method comprising, consisting or consisting essentially of: (a) determining a plurality of biomarker values for at least two (e.g., 2, 3, 4, 5, 6, 7, 8, 9, 10, or more) HVaSIRS biomarkers as broadly defined above of the subject, each biomarker value being indicative of a value measured or derived for a respective HVaSIRS biomarker; (b) determining an indicator using a combination of the plurality of biomarker values, the indicator being at least partially indicative of the presence, absence or degree of HVaSIRS, wherein: (i) at least two (e.g., 2, 3, 4, 5, 6, 7, 8, 9, 10, or more) HVaSIRS biomarkers have a mutual correlation in respect of HVaSIRS that lies within a mutual correlation range, the mutual correlation range being between $\pm 0.9$; and (ii) the indicator has a performance value greater than or equal to a performance threshold representing the ability of the indicator to diagnose the presence, absence or degree of HVaSIRS, the performance threshold being indicative of an explained variance of at least 0.3.

Suitably, the method further comprises: (1) determining a plurality of measured biomarker values, each measured biomarker value being a measured value of HVaSIRS biomarker of the subject; and (2) applying a function to at least two of the measured biomarker values to determine at least one derived biomarker value, the at least one derived biomarker value being indicative of a value of a corresponding derived HVaSIRS biomarker.

Suitably, the function includes at least one of: (a) multiplying two biomarker values; (b) dividing two biomarker values; (c) adding two biomarker values; (d) subtracting two biomarker values; (e) a weighted sum of at least two biomarker values; (f) a log sum of at least two biomarker values; (g) a geometric mean of at least two biomarker values; and (h) a sigmoidal function of at least two biomarker values.

Further disclosed are methods for monitoring the efficacy of a particular treatment regimen in a subject towards a desired health state. These methods generally comprise, consist or consist essentially of: (a) determining a plurality of biomarker values for at least two (e.g., 2, 3, 4, 5, 6, 7, 8, 9, 10, or more) HVaSIRS biomarkers as broadly defined above of the subject after treatment with a treatment regimen, each biomarker value being indicative of a value measured or derived for a respective HVaSIRS biomarker; (b) determining an indicator using a combination of the plurality of biomarker values, the indicator being at least partially indicative of the presence, absence or degree of HVaSIRS, wherein: (i) at least two (e.g., 2, 3, 4, 5, 6, 7, 8, 9, 10, or more) HVaSIRS biomarkers have a mutual correlation in respect of HVaSIRS that lies within a mutual correlation range, the mutual correlation range being between $\pm 0.9$; and (ii) the indicator has a performance value greater than or equal to a performance threshold representing the ability of the indicator to diagnose the presence, absence or degree of HVaSIRS, or to provide a prognosis for a HVaSIRS, the performance threshold being indicative of an explained variance of at least 0.3, and (c) determining that the treatment regimen is effective for changing the health status of the subject to the desired health state on the basis that the indicator indicates the presence of HVaSIRS or the presence of HVaSIRS of a lower degree relative to the degree of HVaSIRS in the subject before treatment with the treatment regimen.

Further contemplated is the use of the indicator-determining methods as broadly described above and elsewhere herein in methods for correlating a biomarker profile with an effective treatment regimen for HVaSIRS, or for determining whether a treatment regimen is effective for treating a subject with HVaSIRS, or for correlating a biomarker profile with a positive or negative response to a treatment regimen, or for determining a positive or negative response to a treatment regimen by a subject with HVaSIRS.

**BRI EF DESCRI PTI ON OF THE DRAWINGS**

Figure 1 is a plot of area under curve (AUC) obtained across the positive and negative validation datasets (combined) when sequentially adding a further nine derived biomarkers to the best performing single derived biomarker (CCL5 / FLNB). The combination of CCL5 / FLNB, PPP2R2B / GNG7 and CASZ1 / VASH1 is considered to have the optimal commercial utility with the lowest risk of introduction of noise.

Figure 2 is a plot of AUC obtained across the positive and negative validation datasets individually when sequentially adding a further nine derived biomarkers to the best performing single derived biomarker (CCL5 / FLNB). The combination of CCL5 / FLNB, PPP2R2B / GNG7 and CASZ1 / VASH1 is considered to have the optimal commercial utility with the lowest risk of introduction of noise and the greatest difference between positive and negative validation datasets.

Figure 3 shows receiver operating characteristic (ROC) - AUC results for the *Positive validation* sets after data was normalized and scaled to form a single dataset.

Figure 4 shows box and whisker plots of the performance of the best derived biomarker CCL5 / FLNB across each of the individual datasets. "Negative validation" datasets are depicted in the top row and "positive validation" datasets are depicted in the bottom row.

Figure 5 shows plots of the performance of the best combination of derived biomarkers CCL5 / FLNB and PPP2R2B / GNG7 across each of the individual datasets. "Negative validation" datasets are depicted in the top row and "positive validation" datasets are depicted in the bottom row.

Figure 6 shows plots of the performance of the best combination of derived biomarkers CCL5 / FLNB, PPP2R2B / GNG7 and CASZ1 / VASH1 across each of the individual datasets. "Negative validation" datasets are depicted in the top row and "positive validation" datasets are depicted in the bottom row.

Figure 7 shows a plot of the most frequently observed numerators in single ratio combinations.

Figure 8 shows a plot of the most frequently observed denominators in single ratio combinations.

Figure 9 is an example output depicting an indicator that is useful for assessing the presence of HVaSIRS in a patient.

## DETAILED DESCRIPTION OF THE INVENTION

### 1. Definitions

Unless defined otherwise, all technical and scientific terms used herein have the same meaning as commonly understood by those of ordinary skill in the art to which the invention belongs. Although any methods and materials similar or equivalent to those described herein can be used in the practice or testing of the present invention, preferred methods and materials are described. For the purposes of the present invention, the following terms are defined below.

The articles "a" and "an" are used herein to refer to one or to more than one (*i.e.,* to at least one) of the grammatical object of the article. By way of example, "an element" means one element or more than one element.

As used herein, "and/or" refers to and encompasses any and all possible combinations of one or more of the associated listed items, as well as the lack of combinations when interpreted in the alternative (or).

The term "biomarker" broadly refers to any detectable compound, such as a protein, a peptide, a proteoglycan, a glycoprotein, a lipoprotein, a carbohydrate, a lipid, a nucleic acid (*e.g.,* DNA, such as cDNA or amplified DNA, or RNA, such as mRNA), an organic or inorganic chemical, a natural or synthetic polymer, a small molecule (*e.g.,* a metabolite), or a discriminating molecule or discriminating fragment of any of the foregoing, that is present in or derived from a sample. "Derived from" as used in this context refers to a compound that, when detected, is indicative of a particular molecule being present in the sample. For example, detection of a particular cDNA can be indicative of the presence of a particular RNA transcript in the sample. As another example, detection of or binding to a particular antibody can be indicative of the presence of a particular antigen (*e.g.*, protein) in the sample. Here, a discriminating molecule or fragment is a molecule or fragment that, when detected, indicates presence or abundance of an above-identified compound. A biomarker can, for example, be isolated from a sample, directly measured in a sample, or detected in or determined to be in a sample. A biomarker can, for example, be functional, partially functional, or non-functional. In specific embodiments, the "biomarkers" include "immune system biomarkers", which are described in more detail below.

The term "biomarker value" refers to a value measured or derived for at least one corresponding biomarker of a subject and which is typically at least partially indicative of an abundance or concentration of a biomarker in a sample taken from the subject. Thus, the biomarker values could be measured biomarker values, which are values of biomarkers measured for the subject, or alternatively could be derived biomarker values, which are values that have been derived from one or more measured biomarker values, for example by applying a function to the one or more measured biomarker values. Biomarker values can be of any appropriate form depending on the manner in which the values are determined. For example, the biomarker values could be determined using high-throughput technologies such as mass spectrometry, sequencing platforms, array and hybridization platforms, immunoassays, flow cytometry, or any combination of such technologies and in one preferred example, the biomarker values relate to a level of activity or abundance of an expression product or other measurable molecule, quantified using a technique such as PCR, sequencing or the like. In this case, the biomarker values can be in the form of amplification amounts, or cycle times, which are a logarithmic representation of the concentration of the biomarker within a sample, as will be appreciated by persons skilled in the art and as will be described in more detail below.

The term "biomarker profile" refers to one or a plurality of one or more types of biomarkers (*e.g.,* an mRNA molecule, a cDNA molecule and/or a protein, *etc.*), or an indication thereof, together with a feature, such as a measurable aspect (*e.g.*, biomarker value) of the biomarker(s). A biomarker profile may comprise a single biomarker whose level, abundance or amount correlates with the presence, absence or degree of a condition (*e.g.*, a healthy condition or HVaSIRS). Alternatively, a biomarker profile may comprise at least two such biomarkers or indications thereof, where the biomarkers can be in the same or different classes, such as, for example, a nucleic acid and a polypeptide. Thus, a biomarker profile may comprise at least 2, 3, 4, 5, 6, 7, 8, 9, 10, 11, 12, 13, 14, 15, 16, 17, 18, 19, 20, 21, 22, 23, 24, 25, 30, 35, 40, 45, 50, 55, 60, 65, 70, 75, 80, 85, 90, 95, or 100 or more biomarkers or indications thereofA biomarker profile may comprise hundreds, or even thousands, of biomarkers or indications thereof. A biomarker profile can further comprise one or more controls or internal standards. The biomarker profile may comprise at least one biomarker, or indication thereof, that serves as an internal standard. Furthermore, a biomarker profile may comprise an indication of one or more types of biomarkers. The term "indication" as used herein in this context merely refers to a situation where the biomarker profile contains symbols, data, abbreviations or other similar indicia for a biomarker, rather than the biomarker molecular entity itself. The term "biomarker profile" is also used herein to refer to a biomarker value or combination of at least two biomarker values, wherein individual biomarker values correspond to values of biomarkers that can be measured or derived from one or more subjects, which combination is characteristic of a discrete condition, stage of condition, subtype of condition or a prognosis for a discrete condition, stage of condition, subtype of condition. The term "profile biomarkers" is used to refer to a subset of the biomarkers that have been identified for use in a biomarker profile that can be used in performing a clinical assessment, such as to rule in or rule out a specific condition, different stages or severity of conditions, subtypes of different conditions or different prognoses. The number of profile biomarkers will vary, but is typically of the order of 10 or less.

The terms "complementary" and "complementarity" refer to polynucleotides (*i.e.,* a sequence of nucleotides) related by the base-pairing rules. For example, the sequence "A-G-T," is complementary to the sequence "T-C-A." Complementarity may be "partial," in which only some of the nucleic acids' bases are matched according to the base pairing rules. Or, there may be "complete" or "total" complementarity between the nucleic acids. The degree of complementarity between nucleic acid strands has significant effects on the efficiency and strength of hybridization between nucleic acid strands.

Throughout this specification, unless the context requires otherwise, the words "comprise," "comprises" and "comprising" will be understood to imply the inclusion of a stated step or element or group of steps or elements but not the exclusion of any other step or element or group of steps or elements. Thus, use of the term "comprising" and the like indicates that the listed elements are required or mandatory, but that other elements are optional and may or may not be present. By "consisting of" is meant including, and limited to, whatever follows the phrase "consisting of". Thus, the phrase "consisting of" indicates that the listed elements are required or mandatory, and that no other elements may be present. By "consisting essentially of" is meant including any elements listed after the phrase, and limited to other elements that do not interfere with or contribute to the activity or action specified in the disclosure for the listed elements. Thus, the phrase "consisting essentially of" indicates that the listed elements are required or mandatory, but that other elements are optional and may or may not be present depending upon whether or not they affect the activity or action of the listed elements.

The term "correlating" refers to determining a relationship between one type of data with another or with a state.

The term "degree" of HVaSIRS, as used herein, refers to the seriousness, severity, stage or state of a HVaSIRS. For example, a HVaSIRS may be characterized as mild, moderate or severe. A person of skill in the art would be able to determine or assess the degree of a particular HVaSIRS. For example, the degree of a HVaSIRS may be determined by comparing the likelihood or length of survival of a subject having a HVaSIRS with the likelihood or length of survival in other subjects having HVaSIRS. In other embodiments, the degree of a HVaSIRS may be determined by comparing the clinical signs of a subject having a condition with the degree of the clinical signs in other subjects having HVaSIRS.

As used herein, the terms "diagnosis", "diagnosing" and the like are used interchangeably herein to encompass determining the likelihood that a subject will develop a condition, or the existence or nature of a condition in a subject. These terms also encompass determining the severity of disease or episode of disease, as well as in the context of rational therapy, in which the diagnosis guides therapy, including initial selection of therapy, modification of therapy (*e.g.*,

adjustment of dose or dosage regimen), and the like. By "likelihood" is meant a measure of whether a subject with particular measured or derived biomarker values actually has a condition (or not) based on a given mathematical model. An increased likelihood for example may be relative or absolute and may be expressed qualitatively or quantitatively. For instance, an increased likelihood may be determined simply by determining the subject's measured or derived biomarker values for at least two HVaSIRS biomarkers and placing the subject in an "increased likelihood" category, based upon previous population studies. The term "likelihood" is also used interchangeably herein with the term "probability". The term "risk" relates to the possibility or probability of a particular event occurring at some point in the future. "Risk stratification" refers to an arraying of known clinical risk factors to allow physicians to classify patients into a low, moderate, high or highest risk of developing a particular disease or condition.

The term "gene", as used herein, refers to a stretch of nucleic acid that codes for a polypeptide or for an RNA chain that has a function. While it is the exon region of a gene that is transcribed to form mRNA, the term "gene" also includes regulatory regions such as promoters and enhancers that govern expression of the exon region.

The term "high-density array" refers to a substrate or collection of substrates or surfaces bearing a plurality of array elements (*e.g.*, discrete regions having particular moieties, *e.g.,* proteins (*e.g.,* antibodies), nucleic acids (*e.g.,* oligonucleotide probes), *etc.,* immobilized thereto), where the array elements are present at a density of about 100 elements/ $cm^2$ or more, about 1,000 elements/ $cm^2$ or more, about 10,000 elements/ $cm^2$ or more, or about 100,000 elements/ $cm^2$ or more. Specifically, a "high-density array" may be one that comprises a plurality of array elements for detecting about 100 or more different biomarkers, about 1,000 or more different biomarkers, about 10,000 or more different biomarkers, or about 100,000 or more different biomarkers. In representative example, a "high-density array" is one that comprises a plurality of array elements for detecting biomarkers of about 100 or more different genes, of about 1,000 or more different genes, of about 10,000 or more different genes, or of about 100,000 or more different genes. Generally, the elements of a high-density array are not labeled. The term "low-density array" refers to a substrate or collection of substrates or surfaces bearing a plurality of array elements (*e.g.*, discrete regions having particular moieties, *e.g.,* proteins (*e.g.,* antibodies), nucleic acids (*e.g.,* oligonucleotide probes), *etc.,* immobilized thereto), where the array elements are present at a density of about 100 elements/ $cm^2$ or less, about 50 elements/ $cm^2$ or less, about 20 elements/ $cm^2$ or less, or about 10 elements/ $cm^2$ or less. Specifically, a "low-density array" may be one that comprises a plurality of array elements for detecting about 100 or less different biomarkers, about 50 or less different biomarkers, about 20 or less different biomarkers, or about 10 or less different biomarkers. In representative example, a "low-density array" is one that comprises a plurality of array elements for detecting biomarkers of about 100 or less different genes, of about 50 or less different genes, of about 20 or less different genes, or of about 10 or less different genes. Generally, the elements of a low-density array are not labeled.

The term "indicator" as used herein refers to a result or representation of a result, including any information, number, ratio, signal, sign, mark, or note by which a skilled artisan can estimate and/or determine a likelihood or risk of whether or not a subject is suffering from a given disease or condition. In the case of the present invention, the "indicator" may optionally be used together with other clinical characteristics, to arrive at a diagnosis (that is, the occurrence or nonoccurrence) of HVaSIRS or a prognosis for a HVaSIRS in a subject. That such an indicator is "determined" is not meant to imply that the indicator is 100% accurate. The skilled clinician may use the indicator together with other clinical indicia to arrive at a diagnosis.

The term "immobilized" means that a molecular species of interest is fixed to a solid support, suitably by covalent linkage. This covalent linkage can be achieved by different means depending on the molecular nature of the molecular species. Moreover, the molecular species may be also fixed on the solid support by electrostatic forces, hydrophobic or hydrophilic interactions or Van-der-Waals forces. The above described physico-chemical interactions typically occur in interactions between molecules. In particular, all that may be required is that the molecules (*e.g.*, nucleic acids or polypeptides) remain immobilized or attached to a support under conditions in which it is intended to use the support, for example in applications requiring nucleic acid amplification and/or sequencing or in in antibody-binding assays. For example, oligonucleotides or primers are immobilized such that a 3' end is available for enzymatic extension and/or at least a portion of the sequence is capable of hybridizing to a complementary sequence. Immobilization can occur via hybridization to a surface attached primer, in which case the immobilized primer or oligonucleotide may be in the 3'-5' orientation. Immobilization can also occur by means other than base-pairing hybridization, such as the covalent attachment.

The term "immune system", as used herein, refers to cells, molecular components and mechanisms, including antigen-specific and non-specific categories of the adaptive and innate immune systems, respectively, that provide a defense against damage and insults resulting from a viral infection. The term "innate immune system" refers to a host's non-specific reaction to insult to include antigen-nonspecific defense cells, molecular components and mechanisms that come into action immediately or within several hours after exposure to almost any insult or antigen. Elements of the innate immunity include for example phagocytic cells (monocytes, macrophages, dendritic cells, polymorphonuclear leukocytes such as neutrophils, reticuloendothelial cells such as Küpffer cells, and microglia), cells that release inflammatory mediators (basophils, mast cells and eosinophils), natural killer cells (NK cells) and physical barriers and molecules such as keratin, mucous, secretions, complement proteins, immunoglobulin M (IgM), acute phase proteins, fibrinogen and molecules of the clotting cascade, and cytokines. Effector compounds of the innate immune system include chemicals such as lysozymes, IgM, mucous and chemoattractants (*e.g.*, cytokines or histamine), complement and clotting proteins. The term "adaptive immune system" refers to antigen-specific cells, molecular components and mechanisms that emerge over several days, and react with and remove a specific antigen. The adaptive immune system develops throughout a host's lifetime. The adaptive immune system is based on leukocytes, and is divided into two major sections: the humoral immune system, which acts mainly *via* immunoglobulins produced by B cells, and the cell-mediated immune system, which functions mainly *via* T cells.

Reference herein to "immuno-interactive" includes reference to any interaction, reaction, or other form of association between molecules and in particular where one of the molecules is, or mimics, a component of the immune system.

The term "label" is used herein in a broad sense to refer to an agent that is capable of providing a detectable signal, either directly or through interaction with one or more additional members of a signal producing system and that has been artificially added, linked or attached via chemical manipulation to a molecule. Labels can be visual, optical, photonic, electronic, acoustic, opto-acoustic, by mass, electro-chemical, electro-optical, spectrometry, enzymatic, or otherwise chemically, biochemically hydrodynamically, electrically or physically detectable. Labels can be, for example tailed reporter, marker or adapter molecules. Specifically, a molecule such as a nucleic acid molecule may be labeled with a detectable molecule selected form the group consisting of radioisotopes, fluorescent compounds, bioluminescent compounds, chemiluminescent compounds, metal chelators or enzymes. Examples of labels include, but are not limited to, the following radioisotopes (*e.g.*, $^3$H, $^{14}$C, $^{35}$S, $^{125}$I, $^{131}$I), fluorescent labels (*e.g.*, FITC, rhodamine, lanthanide phosphors), luminescent labels such as luminol; enzymatic labels (*e.g.*, horseradish peroxidase, beta-galactosidase, luciferase, alkaline phosphatase, acetylcholinesterase), biotinyl groups (which can be detected by marked avidin, *e.g.*, streptavidin containing a fluorescent marker or enzymatic activity that can be detected by optical or calorimetric methods), predetermined polypeptide epitopes recognized by a secondary reporter (*e.g.*, leucine zipper pair sequences, binding sites for secondary antibodies, metal binding domains, epitope tags).

The term "microarray" refers to an arrangement of hybridizable array elements, *e.g.*, probes (including primers), ligands, biomarker nucleic acid sequence or protein sequences on a substrate.

The term "nucleic acid" or "polynucleotide" as used herein includes RNA, mRNA, miRNA, cRNA, cDNA mtDNA, or DNA. The term typically refers to a polymeric form of nucleotides of at least 10 bases in length, either ribonucleotides or deoxynucleotides or a modified form of either type of nucleotide. The term includes single and double stranded forms of DNA or RNA.

By "obtained" is meant to come into possession. Samples so obtained include, for example, nucleic acid extracts or polypeptide extracts isolated or derived from a particular source. For instance, the extract may be isolated directly from a biological fluid or tissue of a subject.

As used herein, the term "positive response" means that the result of a treatment regimen includes some clinically significant benefit, such as the prevention, or reduction of severity, of symptoms, or a slowing of the progression of the condition. By contrast, the term "negative response" means that a treatment regimen provides no clinically significant benefit, such as the prevention, or reduction of severity, of symptoms, or increases the rate of progression of the condition.

"Protein", "polypeptide" and "peptide" are used interchangeably herein to refer to a polymer of amino acid residues and to variants and synthetic analogues of the same.

By "primer" is meant an oligonucleotide which, when paired with a strand of DNA, is capable of initiating the synthesis of a primer extension product in the presence of a suitable polymerizing agent. The primer is preferably single-stranded for maximum efficiency in amplification but can alternatively be double-stranded. A primer must be sufficiently long to prime the synthesis of extension products in the presence of the polymerization agent. The length of the primer depends on many factors, including application, temperature to be employed, template reaction conditions, other reagents, and source of primers. For example, depending on the complexity of the target sequence, the primer may be at least about 5, 6, 7, 8, 9, 10, 11, 12, 13, 14, 15, 16, 17, 18, 19, 20, 21, 22, 23, 24, 25, 26, 27, 28, 29, 30, 35, 40, 50, 75, 100, 150, 200, 300, 400, 500, to one base shorter in length than the template sequence at the 3' end of the primer to allow extension of a nucleic acid chain, though the 5' end of the primer may extend in length beyond the 3' end of the template sequence. Primers can be large polynucleotides, such as from about 35 nucleotides to several kilobases or more. Primers can be selected to be "substantially complementary" to the sequence on the template to which it is designed to hybridize and serve as a site for the initiation of synthesis. By "substantially complementary", it is meant that the primer is sufficiently complementary to hybridize with a target polynucleotide. Desirably, the primer contains no mismatches with the template to which it is designed to hybridize but this is not essential. For example, non-complementary nucleotide residues can be attached to the 5' end of the primer, with the remainder of the primer sequence being complementary to the template. Alternatively, non-complementary nucleotide residues or a stretch of non-complementary nucleotide residues can be interspersed into a primer, provided that the primer sequence has sufficient complementarity with the sequence of the template to hybridize therewith and thereby form a template for synthesis of the extension product of the primer.

As used herein, the term "probe" refers to a molecule that binds to a specific sequence or sub-sequence or other moiety of another molecule. Unless otherwise indicated, the term "probe" typically refers to a nucleic acid probe that binds to another nucleic acid, also referred to herein as a "target polynucleotide", through complementary base pairing. Probes can bind target polynucleotides lacking complete sequence complementarity with the probe, depending on the stringency of the hybridization conditions. Probes can be labeled directly or indirectly and include primers within their scope.

The term "prognosis" as used herein refers to a prediction of the probable course and outcome of a clinical condition or disease. A prognosis is usually made by evaluating factors or symptoms of a disease that are indicative of a favorable or unfavorable course or outcome of the disease. The skilled artisan will understand that the term "prognosis" refers to an increased probability that a certain course or outcome will occur; that is, that a course or outcome is more likely to occur in a subject exhibiting a given condition, when compared to those individuals not exhibiting the condition.

The term "sample" as used herein includes any biological specimen that may be extracted, untreated, treated, diluted or concentrated from a subject. Samples may include, without limitation, biological fluids such as whole blood, serum, red blood cells, white blood cells, plasma, saliva, urine, stool (*i.e.,* faeces), tears, sweat, sebum, nipple aspirate, ductal lavage, tumor exudates, synovial fluid, ascitic fluid, peritoneal fluid, amniotic fluid, cerebrospinal fluid, lymph, fine needle aspirate, amniotic fluid, any other bodily fluid, cell lysates, cellular secretion products, inflammation fluid, semen and vaginal secretions. Samples may include tissue samples and biopsies, tissue homogenates and the like. Advantageous samples may include ones comprising any one or more biomarkers as taught herein in detectable quantities. Suitably, the sample is readily obtainable by minimally invasive methods, allowing the removal or isolation of the sample from the subject. In certain embodiments, the sample contains blood, especially peripheral blood, or a fraction or extract thereof. Typically, the sample comprises blood cells such as mature, immature or developing leukocytes, including lymphocytes, polymorphonuclear leukocytes, neutrophils, monocytes, reticulocytes, basophils, coelomocytes, hemocytes, eosinophils, megakaryocytes, macrophages, dendritic cells natural killer cells, or fraction of such cells (*e.g.,* a nucleic acid or protein fraction). In specific embodiments, the sample comprises leukocytes including peripheral blood mononuclear cells (PBMC).

The term "solid support" as used herein refers to a solid inert surface or body to which a molecular species, such as a nucleic acid and polypeptides can be immobilized. Non-limiting examples of solid supports include glass surfaces, plastic surfaces, latex, dextran, polystyrene surfaces, polypropylene surfaces, polyacrylamide gels, gold surfaces, and silicon wafers. The solid supports may be in the form of membranes, chips or particles. For example, the solid support may be a glass surface (*e.g.,* a planar surface of a flow cell channel). The solid support may comprise an inert substrate or matrix which has been "functionalized", such as by applying a layer or coating of an intermediate material comprising reactive groups which permit covalent attachment to molecules such as polynucleotides. By way of non-limiting example, such supports can include polyacrylamide hydrogels supported on an inert substrate such as glass. The molecules (*e.g.,* polynucleotides) can be directly covalently attached to the intermediate material (*e.g.,* a hydrogel) but the intermediate material can itself be non-covalently attached to the substrate or matrix (*e.g.,* a glass substrate). The support can include a plurality of particles or beads each having a different attached molecular species.

As used herein, the term SIRS ("systemic inflammatory response syndrome") refers to a clinical response arising from a non-specific insult with two or more of the following measureable clinical characteristics; a body temperature greater than 38° C or less than 36° C, a heart rate greater than 90 beats per minute, a respiratory rate greater than 20 per minute, a white blood cell count (total leukocytes) greater than 12,000 per $mm^3$ or less than 4,000 per $mm^3$, or a band neutrophil percentage greater than 10%. From an immunological perspective, it may be seen as representing a systemic response to insult (*e.g.,* major surgery) or systemic inflammation. As used herein, "HVaSIRS" includes any one or more (*e.g.,* 1, 2, 3, 4, 5) of the clinical responses noted above but with underlying herpesvirus infection etiology. Confirmation of infection can be determined using any suitable procedure known in the art, illustrative examples of which include nucleic acid detection (*e.g.,* polymerase chain reaction (PCR), immunological detection (*e.g.,* ELISA), isolation of virus from infected cells, cell lysis and imaging techniques such as electron microscopy. From an immunological perspective, HVaSIRS may be seen as a systemic response to herpesvirus infection, whether it is a local, peripheral or systemic infection.

The terms "subject", "individual" and "patient" are used interchangeably herein to refer to an animal subject, particularly a vertebrate subject, and even more particularly a mammalian subject. Suitable vertebrate animals that fall within the scope of the invention include, but are not restricted to, any member of the phylum Chordata, subphylum vertebrata including primates, rodents (*e.g.,* mice rats, guinea pigs), lagomorphs (*e.g.,* rabbits, hares), bovines (*e.g.,* cattle), ovines (*e.g.,* sheep), caprines (*e.g.,* goats), porcines (*e.g.,* pigs), equines (*e.g.,* horses), canines (*e.g.,* dogs), felines (*e.g.,* cats), avians (*e.g.,* chickens, turkeys, ducks, geese, companion birds such as canaries, budgerigars *etc.*), marine mammals (*e.g.,* dolphins, whales), reptiles (snakes, frogs, lizards, *etc.*), and fish. A preferred subject is a primate (*e.g.,* a human, ape, monkey, chimpanzee). The subject suitably has at least one (*e.g.,* 1, 2, 3, 4, 5 or more) clinical sign of SIRS.

As used herein, the term "treatment regimen" refers to prophylactic and/or therapeutic (*i.e.,* after onset of a specified condition) treatments, unless the context specifically indicates otherwise. The term "treatment regimen" encompasses natural substances and pharmaceutical agents (*i.e.,* "drugs") as well as any other treatment regimen including but not limited to dietary treatments, physical therapy or exercise regimens, surgical interventions, and combinations thereof.

It will be appreciated that the terms used herein and associated definitions are used for the purpose of explanation only and are not intended to be limiting.

## 2. Herpesvirus systemic inflammation biomarkers and their use for identifying subjects with HVaSIRS

Disclosed in the context of the present invention are methods, apparatus, compositions and kits for identifying subjects with HVaSIRS or for providing a prognosis for subjects with HVaSIRS. In particular, HVaSIRS biomarkers are disclosed for use in these modalities to assess the likelihood of the presence, absence or degree of HVaSIRS in subjects, or for providing a prognosis for subjects with HVaSIRS. The methods, apparatus, compositions and kits of the invention are useful for early detection of HVaSIRS, thus allowing better treatment interventions for subjects with symptoms of SIRS that stem at least in part from a viral infection.

The present inventors have determined that certain expression products are commonly, specifically and differentially expressed during systemic inflammations with a range of herpesvirus etiologies (*e.g.*, HHV 1, 2, 4, 5, 6), underscoring the conserved nature of the host response to a HVaSIRS. The results presented herein provide clear evidence that a unique biologically-relevant biomarker profile predicts HVaSIRS with a remarkable degree of accuracy. This herpesvirus systemic inflammation biomarker profile was validated in an independently derived external dataset (MARS) (*see,* Table 3 for details) and used to distinguish HVaSIRS from other SIRS conditions including bacterium associated SIRS (BaSIRS), autoimmune disease associated SIRS (ADaSIRS), cancer associated SIRS (CaSIRS) and non-herpesvirus (influenza virus, Lassa virus, respiratory syncytial virus) virus associated SIRS (nHVVaSIRS) Overall, these findings provide compelling evidence that the expression products disclosed herein can function as biomarkers for HVaSIRS and may potentially serve as a useful diagnostic for triaging treatment decisions for SIRS-affected subjects. In this regard, it is proposed that the methods, apparatus, compositions and kits disclosed herein that are based on these biomarkers may serve in the point-of-care diagnostics that allow for rapid and inexpensive screening for HVaSIRS, which may result in significant cost savings to the medical system as HVaSIRS-affected subjects can be exposed to therapeutic agents that are suitable for treating HVaSIRS as opposed to therapeutic agents for other SIRS conditions.

Thus, specific expression products are disclosed herein as HVaSIRS biomarkers that provide a means for identifying HVaSIRS and/or for distinguishing HVaSIRS from other SIRS conditions including BaSIRS, ADaSIRS, CaSIRS, TaSIRS and nHVVaSIRS, and/or for providing a prognosis for a subject with HVaSIRS. Evaluation of these HVaSIRS biomarkers through analysis of their levels in a subject or in a sample taken from a subject provides a measured or derived biomarker value for determinating an indicator that can be used for assessing the presence, absence or degree of HVaSIRS in a subject or for providing a prognosis for HVaSIRS in a subject.

Accordingly, biomarker values can be measured derived biomarker values, which are values that have been derived from one or more measured biomarker values, for example by applying a function to the one or more measured biomarker values. As used herein, biomarkers to which a function has been applied are referred to as "derived markers".

The biomarker values may be determined in any one of a number of ways. An exemplary method of determining biomarker values is described by the present inventors in WO 2015/117204. In one example, the process of determining biomarker values can include measuring the biomarker values, for example by performing tests on the subject or on sample(s) taken from the subject. More typically however, the step of determining the biomarker values includes having an electronic processing device receive or otherwise obtain biomarker values that have been previously measured or derived. This could include for example, retrieving the biomarker values from a data store such as a remote database, obtaining biomarker values that have been manually input, using an input device, or the like. The indicator is determined using a combination of the plurality of biomarker values, the indicator being at least partially indicative of the presence, absence, degree or prognosis of HVaSIRS. Assuming the method is performed using an electronic processing device, an indication of the indicator is optionally displayed or otherwise provided to the user. In this regard, the indication could be a graphical or alphanumeric representation of an indicator value. Alternatively, however, the indication could be the result of a comparison of the indicator value to predefined thresholds or ranges, or alternatively could be an indication of the presence, absence, degree of a HVaSIRS or prognosis for a HVaSIRS, derived using the indicator.

In some embodiments, biomarker values are combined, for example by adding, multiplying, subtracting, or dividing biomarker values to determine an indicator value. This step is performed so that multiple biomarker values can be combined into a single indicator value, providing a more useful and straightforward mechanism for allowing the indicator to be interpreted and hence used in diagnosing the presence, absence or degree of HVaSIRS in the subject, or providing a prognosis for a HVaSIRS in the subject.

In some embodiments in which a plurality of biomarkers and biomarker values are used, in order to ensure that an effective diagnosis or prognosis can be determined, at least two of the biomarkers have a mutual correlation in respect of HVaSIRS that lies within a mutual correlation range, the mutual correlation range being between ±0.9. This requirement means that the two biomarkers are not entirely correlated in respect of each other when considered in the context of the HVaSIRS being diagnosed or prognosed. In other words, at least two of the biomarkers in the combination respond differently as the condition changes, which adds significantly to their ability when combined to discriminate between at least two conditions, to diagnose the presence, absence or degree of HVaSIRS, and/or to provide a prognosis for the HVaSIRS in or of the subject.

Typically, the requirement that biomarkers have a low mutual correlation means that the biomarkers may relate to different biological attributes or domains such as, but not limited, to different molecular functions, different biological processes and different cellular components. Illustrative examples of molecular function include addition of, or removal of, one of more of the following moieties to, or from, a protein, polypeptide, peptide, nucleic acid (e.g., DNA, RNA): linear, branched, saturated or unsaturated alkyl (e.g., $C_1$-$C_{24}$ alkyl); phosphate; ubiquitin; acyl; fatty acid, lipid, phospholipid; nucleotide base; hydroxyl and the like. Molecular functions also include signaling pathways, including without limitation, receptor signaling pathways and nuclear signaling pathways. Non-limiting examples of molecular functions also include cleavage of a nucleic acid, peptide, polypeptide or protein at one or more sites; polymerization of a nucleic acid, peptide, polypeptide or protein; translocation through a cell membrane (e.g., outer cell membrane; nuclear membrane); translocation into or out of a cell organelle (e.g., Golgi apparatus, lysosome, endoplasmic reticulum, nucleus, mitochondria); receptor binding, receptor signaling, membrane channel binding, membrane channel influx or efflux; and the like.

Illustrative examples of biological processes include: stages of the cell cycle such as meiosis, mitosis, cell division, prophase, metaphase, anaphase, telophase and interphase, stages of cell differentiation; apoptosis; necrosis; chemotaxis; immune responses including adaptive and innate immune responses, pro-inflammatory immune responses, autoimmune responses, tolerogenic responses and the like. Other illustrative examples of biological processes include generating or breaking down adenosine triphosphate (ATP), saccharides, polysaccharides, fatty acids, lipids, phospholipids, sphingolipids, glycolipids, cholesterol, nucleotides, nucleic acids, membranes (e.g., cell plasma membrane, nuclear membrane), amino acids, peptides, polypeptides, proteins and the like. Representative examples of cellular components include organelles, membranes, as for example noted above, and others.

It will be understood that the use of biomarkers that have different biological attributes or domains provides further information than if the biomarkers were related to the same or common biological attributes or domains. In this regard, it will be appreciated if the at least two biomarkers are highly correlated to each other, the use of both biomarkers would add little diagnostic/prognostic improvement compared to the use of a single one of the biomarkers. Accordingly, an indicator-determining method of the present invention in which a plurality of biomarkers and biomarker values are used preferably employ biomarkers that are not well correlated with each other, thereby ensuring that the inclusion of each biomarker in the method adds significantly to the discriminative ability of the indicator.

Despite this, in order to ensure that the indicator can accurately be used in performing the discrimination between at least two conditions (e.g., HVaSIRS and healthy condition) or the diagnosis of the presence, absence or degree of HVaSIRS or the provision of a prognosis for the HVaSIRS, the indicator has a performance value that is greater than or equal to a performance threshold. The performance threshold may be of any suitable form but is to be typically indicative of an explained variance of at least 0.3, or an equivalent value of another performance measure.

Suitably, a combination of biomarkers is employed, which biomarkers have a mutual correlation between ±0.9 and which combination provides an explained variance of at least 0.3. This typically allows an indicator to be defined that is suitable for ensuring that an accurate discrimination, diagnosis or prognosis can be obtained whilst minimizing the number of biomarkers that are required. Typically, the mutual correlation range is one of ±0.8; ±0.7; ±0.6; ±0.5; ±0.4; ±0.3; ±0.2; and, ±0.1. Typically, each HVaSIRS biomarker has a condition correlation with the presence, absence or degree of HVaSIRS, or with a prognosis for a HVaSIRS that lies outside a condition correlation range, the condition correlation range being between ±0.3 and more typically ± 0.9; ±0.8; ±0.7; ±0.6; ±0.5; and, ±0.4. Typically, the performance threshold is indicative of an explained variance of at least one of 0.4; 0.5; 0.6; 0.7; 0.8; and 0.9.

It will be understood that in this context, the biomarkers used within the above-described method can define a biomarker profile for a HVaSIRS, which includes a minimal number of biomarkers, whilst maintaining sufficient performance to allow the biomarker profile to be used in making a clinically relevant diagnosis, prognosis, or differentiation. Minimizing the number of biomarkers used minimizes the costs associated with performing diagnostic or prognostic tests and in the case of nucleic acid expression products, allows the test to be performed utilizing relatively straightforward techniques such as nucleic acid array, and polymerase chain reaction (PCR) processes, or the like, allowing the test to be performed rapidly in a clinical environment.

Furthermore, producing a single indicator value allows the results of the test to be easily interpreted by a clinician or other medical practitioner, so that test can be used for reliable diagnosis in a clinical environment.

Processes for generating suitable biomarker profiles are described for example in WO 2015/117204, which uses the term "biomarker signature" in place of "biomarker profile" as defined herein. It will be understood, therefore, that terms "biomarker profile" and "biomarker signature" are equivalent in scope. The biomarker profile-generating processes disclosed in WO 2015/117204 provide mechanisms for selecting a combination of biomarkers, and more typically derived biomarkers, that can be used to form a biomarker profile, which in turn can be used in diagnosing the presence, absence or degree of HVaSIRS or in providing a prognosis for a HVaSIRS. In this regard, the biomarker profile defines the biomarkers that should be measured (i.e., the profile biomarkers), how derived biomarker values should be determined for measured biomarker values, and then how biomarker values should be subsequently combined to generate an indicator value. The biomarker profile can also specify defined indicator value ranges that indicate a particular presence, absence or degree of HVaSIRS or that provide a prognosis for a HVaSIRS.

Using the above-described methods a number of biomarkers have been identified that are particularly useful for assessing a likelihood that a subject has a presence, absence or degree of HVaSIRS or for providing a prognosis for a HVaSIRS in a subject. These biomarkers are referred to herein as "HVaSIRS biomarkers". As used herein, the term "HVaSIRS biomarker" refers to a biomarker of the host, generally a biomarker of the host's immune system, which is altered, or whose level of expression is altered, as part of an inflammatory response to damage or insult resulting from a herpesvirus infection. The HVaSIRS biomarkers are suitably expression products of genes (also referred to interchangeably herein as "HVaSIRS biomarker genes"), including polynucleotide and polypeptide expression products. As used herein, polynucleotide expression products of HVaSIRS biomarker genes are referred to herein as "HVaSIRS biomarker polynucleotides." Polypeptide expression products of the HVaSIRS biomarker genes are referred to herein as "HVaSIRS biomarker polypeptides."

HVaSIRS biomarker are suitably selected from expression products of any one or more of the following HVaSIRS genes: *ACAT1, ACSL1, ADCY3, ADCY7, ADRB2, ADSL, AKAP11, ALDOC, ARHGEF18, ARNTL, ARSB, ATP2B4, BACH2, BCL11A, BNIP3, BTG1, BTG3, C1QB, C6orf48, CAMK1D, CASP4, CASZ1, CCL5, CCR7, CD300A, CD37, CD79A, CD79B, CD8A, CD93, CHST12, COL17A1, COX4I1, CRY2, CST7, CYB561, CYLD, DGKA, DHRS3, DNPEP, DPEP2, EEF1G, EHD1, EIF2AK2, EIF2S3, EIF4B, EPHX2, ETS1, FAM129A, FDFT1, FLNB, FLT3LG, GALNT10, GDPD5, GFI1, GNG7, GPR162, GPR56, GRWD1, GZMA, HERC5, HERC6, HIC2, HSD17B8, ICAM2, IFI16, IFI44, IFITM1, IKBKG, IL4R, ITM2C, ITPKB, KIF13B, KPNB1, LAMA5, LBH, LDLR, LDLRAP1, LEF1, LRMP, LSM7, LTBP3, MAL, MED25, MLLT11, MRPS18B, MZF1, NAALADL1, NACA, NAP1L1, NECAP2, NET1, NGFRAP1, NKG7, NOSIP, OASL, PABPC4, PARP3, PEX11B, PFKL, PFKP, PHB2, PIK3IP1, PLEKHB1, POP5, PPP2R2B, PRDM1, PRKD2, PTOV1, PXN, RAB3GAP1, RABGAP1L, RALA, RARRES3, RBM17, RBM4B, REC8, RPAIN, RPL10A, RPL11, RPL12, RPL15, RPL22, RPL27, RPL30, RPL36, RPL38, RPL8, RPS11, RPS5, RPS9, RUNX3, SAC3D1, SATB1, SERBP1, SERPINE2, SERTAD2, SLAMF7, SLC5A6, SPINT2, SQRDL, SREBF1, SRM, SVIL, SYNE2, SYPL1, SYT11, TANK, TAP1, TBC1D4, TBK1, TCF4, TCL1A, TDRD7, TGFBR3, TMC6, TMEM134, TMEM39B, TMUB2, TOP2B, TPK1, TPST2, TRAF3IP3, TXN, UBE2D2, UBE2L6, UBR2, VASH1, VPREB3, VPS35, WDR61, XPC, ZBP1 and ZW10.* Non-limiting examples of nucleotide sequences for these HVaSIRS biomarkers are listed in SEQ ID NOs: 1-174. Non-limiting examples of amino acid sequences for these HVaSIRS biomarkers are listed in SEQ ID NOs: 175-348.

The present inventors have determined that HVaSIRS biomarkers have strong diagnostic performance when combined with one or more other HVaSIRS biomarkers. In advantageous embodiments, pairs of HVaSIRS biomarkers have been identified that can be used to determine the indicator. Accordingly, in representative examples of this type, an indicator is determined that correlates to a ratio of HVaSIRS biomarkers, which can be used in assessing a likelihood of a subject having a presence, absence or degree of HVaSIRS, or in providing a prognosis for a subject with HVaSIRS.

In these examples, the indicator-determining methods suitably include determining a pair of biomarker values, wherein each biomarker value is a value measured or derived for at least one corresponding HVaSIRS biomarker of the subject and is at least partially indicative of a concentration of the HVaSIRS biomarker in a sample taken from the subject. The biomarker values are typically used to determine a derived biomarker value using the pair of biomarker values, wherein the derived biomarker value is indicative of a ratio of concentrations of the pair of HVaSIRS biomarkers. Thus, if the biomarker values denote the concentrations of the HVaSIRS biomarkers, then the derived biomarker value will be based on a ratio of the biomarker values. However, if the biomarker values are related to the concentrations of the biomarkers, for example if they are logarithmically related by virtue of the biomarker values being based on PCR cycle times, or the like, then the biomarker values may be combined in some other manner, such as by subtracting the cycle times to determine a derived biomarker value indicative of a ratio of the concentrations of the HVaSIRS biomarkers.

The derived biomarker value is then used to determine the indicator, either by using the derived biomarker value as an indicator value, or by performing additional processing, such as comparing the derived biomarker value to a reference or the like, as will be described in more detail below.

In some embodiments in which pairs of HVaSIRS biomarkers are used to determine a derived biomarker value, one biomarker of a biomarker pair is selected from Group A HVaSIRS biomarkers and the other is selected from Group B HVaSIRS biomarkers, wherein an individual Group A HVaSIRS biomarker is an expression product of a gene selected from the group consisting of: *GZMA, TGFBR3, CD8A, PABPC4, CHST12, NKG7, SYT11, RARRES3, RPL12, KIF13B, ICAM2, ADRB2, MRPS18B, RPL8, RPS11, SLAMF7, PRKD2, FDFT1, ATP2B4, RPL38, BTG1, DNPEP, NAP1L1, RABGAP1L and ACAT1,* and wherein an individual Group B HVaSIRS biomarker is an expression product of a gene selected from the group consisting of: *EPHX2, CCR7, MAL, LEF1, TRAF3IP3, PIK3IP1, ITM2C, LDLRAP1, NOSIP,*

*ARHGEF18, SATB1, ITPKB, TBC1D4, FLT3LG, DHRS3, RBM4B, HSD17B8, MLLT11, TMC6, PLEKHB1, ALDOC, RPL22, EEF1G, TOP2B, BACH2, BNI P3, ADSL, GRWD1, RPL10A, TMEM134, ETS1, CRY2, SERBP1, RPS5, ZW10, EIF4B, PEX11B, PHB2, SERPINE2, SRM, LSM7, RPL15, RBM17, DGKA, PTOV1, HIC2, NET1, XPC, SLC5A6, SERTAD2, TMEM39B, RPL27, RPL11, C6orf48, WDR61, PFKP, EIF2S3, NECAP2, RPL30, NACA, NGFRAP1, RPS9, RALA, COX411, UBE2D2 and VPS35.*

Furthermore, when pairs of HVaSIRS biomarkers are used to determine a derived biomarker value, one biomarker of a biomarker pair may be selected from Group C HVaSIRS biomarkers and the other may be selected from Group D HVaSIRS biomarkers, wherein an individual Group C HVaSIRS biomarker is an expression product of a gene selected from the group consisting of: *RUNX3, GPR56, LBH, POPS, SYNE2, TPST2, RPL36, GALNT10, GFI1, MZF1, SREBF1, AKAP11, CAMK1D, CYB561, BTG3, CST7, SVIL, EIF2AK2, ARNTL, ZBP1, ADCY7, SYPL1 and HERC6,* and wherein an individual Group D HVaSIRS biomarker is an expression product of a gene selected from the group consisting of: *CD79B, CD79A, VPREB3, TCL1A, LAMAS, BCL11A, TCF4, DPEP2, RPAIN, RAB3GAP1, MED25 and LRMP.*

Furthermore, when pairs of HVaSIRS biomarkers are used to determine a derived biomarker value, one biomarker of a biomarker pair may be selected from Group E HVaSIRS biomarkers and the other may be selected from Group F HVaSIRS biomarkers, wherein an individual Group E HVaSIRS biomarker is an expression product of a gene selected from the group consisting of: *TMUB2, ARSB, C1QB, PFKL, EHD1, PRDM1, IKBKG, TAP1, TANK, PARP3, OASL, ADCY3, FAM129A, SQRDL, UBR2, IFITM1, CYLD, ACSL1, PXN, UBE2L6, IL4R, COL17A1, TXN, CD300A, LDLR, TBK1, HERC5, TDRD7, KPNB1, IFI44, CASP4 and IFI16,* and wherein an individual Group F HVaSIRS biomarker is an expression product of a gene selected from the group consisting of: *GPR162, NAALADL1, LTBP3, REC8, CD37, TPK1, GDPD5, SAC3D1, SPINT2 and CD93.*

In specific embodiments, the indicator-determining methods involve determining a first derived biomarker value using a first pair of biomarker values, the first derived biomarker value being indicative of a ratio of concentrations of first and second HVaSIRS biomarkers, determining a second derived biomarker value using a second pair of biomarker values, the second derived biomarker value being indicative of a ratio of concentrations of third and fourth HVaSIRS biomarkers, determining a third derived biomarker value using a third pair of biomarker values, the third derived biomarker value being indicative of a ratio of concentrations of fifth and sixth HVaSIRS biomarkers and determining the indicator by combining the first, second and third derived biomarker values. Thus, in these embodiments, three pairs of derived biomarker values can be used, which can assist in increasing the ability of the indicator to reliably determine the likelihood of a subject having or not having HVaSIRS.

The derived biomarker values could be combined using a combining function such as an additive model; a linear model; a support vector machine; a neural network model; a tree-learning method (*e.g.*, random forest model); a regression model; a genetic algorithm; an annealing algorithm; a weighted sum; a nearest neighbor model; an ensemble method (*e.g.*, bagging, boosting weighted averaging); and a probabilistic model. Biomarker values may be measured or derived for a Group A HVaSIRS biomarker and for a Group B HVaSIRS biomarker, and the indicator may be determined by combining the biomarker values. Biomarker values may be also measured or derived for a Group C HVaSIRS biomarker and for a Group D HVaSIRS biomarker, and the indicator may be determined by combining the biomarker values. Furthermore, biomarker values may be measured or derived for a Group E HVaSIRS biomarker and for a Group F HVaSIRS biomarker, and the indicator may be determined by combining the biomarker values. Furthermore, biomarker values may be measured or derived for a Group A HVaSIRS biomarker, for a Group B HVaSIRS biomarker, for a Group C HVaSIRS biomarker and for a Group D HVaSIRS biomarker, and the indicator may be determined by combining the biomarker values. Furthermore, biomarker values may be measured or derived for a Group A HVaSIRS biomarker, for a Group B HVaSIRS biomarker, for a Group C HVaSIRS biomarker, for a Group D HVaSIRS biomarker, for a Group E HVaSIRS biomarker and for a Group F HVaSIRS biomarker and the indicator may be determined by combining the biomarker values.

The indicator may be compared to an indicator reference, with a likelihood being determined in accordance with results of the comparison. The indicator reference may be derived from indicators determined for a number of individuals in a reference population. The reference population typically includes individuals having different characteristics, such as a plurality of individuals of different sexes; and/or ethnicities, with different groups being defined based on different characteristics, with the subject's indicator being compared to indicator references derived from individuals with similar characteristics. The reference population can also include a plurality of healthy individuals, a plurality of individuals suffering from HVaSIRS, a plurality of individuals suffering from a SIRS other than HVaSIRS (*e.g.*, ADaSIRS, CaSIRS, TaSIRS and nHVVaSIRS), a plurality of individuals showing clinical signs of HVaSIRS, a plurality of individuals showing clinical signs of a SIRS other than HVaSIRS (*e.g.*, *e.g.*, ADaSIRS, CaSIRS, TaSIRS and nHVVaSIRS), and/or first and second groups of individuals, each group of individuals suffering from a respective diagnosed SIRS.

The indicator can also be used for determining a likelihood of the subject having a first or second condition, wherein the first condition is HVaSIRS and the second condition is a healthy condition or another SIRS (*e.g.*, ADaSIRS, CaSIRS, TaSIRS and nHVVaSIRS); in other words, to distinguish between these conditions. In this case, this would typically be achieved by comparing the indicator to first and second indicator references, the first and second indicator references being indicative of first and second conditions and determining the likelihood in accordance with the results of the comparison. In particular, this can include determining first and second indicator probabilities using the results of the comparisons and combining the first and second indicator probabilities, for example using a Bayes method, to determine a condition probability corresponding to the likelihood of the subject having one of the conditions. In this situation the first and second conditions could include HVaSIRS and another SIRS conditions, or HVaSIRS and a healthy condition. In this case, the first and second indicator references are distributions of indicators determined for first and second groups of a reference population, the first and second group consisting of individuals diagnosed with the first or second condition respectively.

In specific embodiments, the indicator-determining methods of the present invention are performed using at least one electronic processing device, such as a suitably programmed computer system or the like. In this case, the electronic processing device typically obtains at least three pairs of measured biomarker values, either by receiving these from a measuring or other quantifying device, or by retrieving these from a database or the like. The processing device then determines a first derived biomarker value indicative of a ratio of concentrations of first and second immune system biomarkers, a second derived biomarker value indicative of a ratio of third and fourth immune system biomarkers, and a third derived biomarker value indicative of a ratio of fifth and sixth immune system biomarkers . The processing device then determines the indicator by combining the first, second and third derived biomarker values.

The processing device can then generate a representation of the indicator, for example by generating an alphanumeric indication of the indicator, a graphical indication of a comparison of the indicator to one or more indicator references or an alphanumeric indication of a likelihood of the subject having at least one medical condition.

It is disclosed, that the indicator-determining methods of the present invention typically include obtaining a sample from a subject, who typically has at least one clinical sign of SIRS, wherein the sample includes one or more HVaSIRS biomarkers (*e.g.*, polynucleotide or polypeptide expression products of HVaSIRS genes) and quantifying at least two (*e.g.,* 2, 3, 4, 5, 6, 7, 8, 9, 10 or more) of the HVaSIRS biomarkers within the sample to determine biomarker values. This can be achieved using any suitable technique, and will depend on the nature of the HVaSIRS biomarkers. Suitably, an individual measured or derived HVaSIRS biomarker value corresponds to the level, abundance or amount of a respective HVaSIRS biomarker or to a function that is applied to that level or amount. As used herein the terms "level", "abundance" and "amount" are used interchangeably herein to refer to a quantitative amount (*e.g.*, weight or moles), a semi-quantitative amount, a relative amount (*e.g.*, weight % or mole % within class), a concentration, and the like. Thus, these terms encompass absolute or relative amounts or concentrations of HVaSIRS biomarkers in a sample. For example, if the indicator in some embodiments of the indicator-determining method of the present invention, which uses a plurality of HVaSIRS biomarkers, is based on a ratio of concentrations of the polynucleotide expression products, this process would typically include quantifying polynucleotide expression products by amplifying at least some polynucleotide expression products in the sample, determining an amplification amount representing a degree of amplification required to obtain a defined level of each of a pair of polynucleotide expression products and determining the indicator by determining a difference between the amplification amounts. In this regard, the amplification amount is generally a cycle time, a number of cycles, a cycle threshold and an amplification time. In this case, the method includes determining a first derived biomarker value by determining a difference between the amplification amounts of a first pair of polynucleotide expression products, determining a second derived biomarker value by determining a difference between the amplification amounts of a second pair of polynucleotide expression products, determining a third derived biomarker value by determining a difference between the amplification amounts of a third pair of polynucleotide expression products and determining the indicator by adding the first, second and third derived biomarker values.

The presence, absence or degree of HVaSIRS or prognosis for a HVaSIRS in a subject may be established by determining two or more HVaSIRS biomarker values, wherein a HVaSIRS biomarker value is indicative of a value derived for HVaSIRS biomarkers in a subject or in a sample taken from the subject. These biomarkers are referred to herein as "sample HVaSIRS biomarkers". In accordance with the present invention, a sample HVaSIRS biomarker corresponds to a reference HVaSIRS biomarker (also referred to herein as a "corresponding HVaSIRS biomarker"). By "corresponding HVaSIRS biomarker" is meant a HVaSIRS biomarker that is structurally and/or functionally similar to a reference HVaSIRS biomarker as set forth for example in SEQ ID NOs: 1-348. Representative corresponding HVaSIRS biomarkers include expression products of allelic variants (same locus), homologues (different locus), and orthologues (different organism) of reference HVaSIRS biomarker genes. Nucleic acid variants of reference HVaSIRS biomarker genes and encoded HVaSIRS biomarker polynucleotide expression products can contain nucleotide substitutions, deletions, inversions and/or insertions. Variation can occur in either or both the coding and non-coding regions. The variations can produce both conservative and non-conservative amino acid substitutions (as compared in the encoded product). For nucleotide sequences, conservative variants include those sequences that, because of the degeneracy of the genetic code, encode the amino acid sequence of a reference HVaSIRS polypeptide.

Generally, variants of a particular HVaSIRS biomarker gene or polynucleotide will have at least about 40%, 45%, 50%, 51%, 52%, 53%, 54%, 55%, 56%, 57%, 58%, 59% 60%, 61%, 62%, 63%, 64%, 65%, 66%, 67%, 68%, 69%

70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or more sequence identity to that particular nucleotide sequence as determined by sequence alignment programs known in the art using default parameters. The HVaSIRS biomarker gene or polynucleotide may display at least about 40%, 45%, 50%, 51%, 52%, 53%, 54%, 55%, 56%, 57%, 58%, 59% 60%, 61%, 62%, 63%, 64%, 65%, 66%, 67%, 68%, 69% 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or more sequence identity to a nucleotide sequence selected from any one of SEQ ID NO: 1-174, as summarized in Table 11.

Corresponding HVaSIRS biomarkers also include amino acid sequences that display substantial sequence similarity or identity to the amino acid sequence of a reference HVaSIRS biomarker polypeptide. In general, an amino acid sequence that corresponds to a reference amino acid sequence will display at least about 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 97, 88, 89, 90, 91, 92, 93, 94, 95, 96, 97, 98, 99% or even up to 100% sequence similarity or identity to a reference amino acid sequence selected from any one of SEQ ID NO: 175 - 348, as summarized in Table 12.

Calculations of sequence similarity or sequence identity between sequences may be performed as follows:

To determine the percentage identity of two amino acid sequences, or of two nucleic acid sequences, the sequences are aligned for optimal comparison purposes (e.g., gaps can be introduced in one or both of a first and a second amino acid or nucleic acid sequence for optimal alignment and non-homologous sequences can be disregarded for comparison purposes). The length of a reference sequence aligned for comparison purposes may be at least 30%, usually at least 40%, more usually at least 50%, 60%, and even more usually at least 70%, 80%, 90%, 100% of the length of the reference sequence. The amino acid residues or nucleotides at corresponding amino acid positions or nucleotide positions are then compared. When a position in the first sequence is occupied by the same amino acid residue or nucleotide at the corresponding position in the second sequence, then the molecules are identical at that position. For amino acid sequence comparison, when a position in the first sequence is occupied by the same or similar amino acid residue (i.e., conservative substitution) at the corresponding position in the second sequence, then the molecules are similar at that position.

The percentage identity between the two sequences is a function of the number of identical amino acid residues shared by the sequences at individual positions, taking into account the number of gaps, and the length of each gap, which need to be introduced for optimal alignment of the two sequences. By contrast, the percentage similarity between the two sequences is a function of the number of identical and similar amino acid residues shared by the sequences at individual positions, taking into account the number of gaps, and the length of each gap, which need to be introduced for optimal alignment of the two sequences.

The comparison of sequences and determination of percentage identity or percentage similarity between sequences can be accomplished using a mathematical algorithm. The percentage identity or similarity between amino acid sequences may be determined using the Needleman and Wünsch, (1970, J. Mol. Biol. 48: 444-453) algorithm which has been incorporated into the GAP program in the GCG software package (available at http://www.gcg.com), using either a Blossum 62 matrix or a PAM250 matrix, and a gap weight of 16, 14, 12, 10, 8, 6, or 4 and a length weight of 1, 2, 3, 4, 5, or 6. Specifically, the percent identity between nucleotide sequences may be determined using the GAP program in the GCG software package (available at http://www.gcg.com), using a NWSgapdna.CMP matrix and a gap weight of 40, 50, 60, 70, or 80 and a length weight of 1, 2, 3, 4, 5, or 6. An non-limiting set of parameters (and the one that should be used unless otherwise specified) includes a Blossum 62 scoring matrix with a gap penalty of 12, a gap extend penalty of 4, and a frameshift gap penalty of 5.

Furthermore, the percentage identity or similarity between amino acid or nucleotide sequences can be determined using the algorithm of E. Meyers and W. Miller (1989, Cabios, 4: 11-17) which has been incorporated into the ALIGN program (version 2.0), using a PAM120 weight residue table, a gap length penalty of 12 and a gap penalty of 4.

The nucleic acid and protein sequences described herein can be used as a "query sequence" to perform a search against public databases to, for example, identify other family members or related sequences. Such searches can be performed using the NBLAST and XBLAST programs (version 2.0) of Altschul, et al., (1990, J Mol Biol., 215: 403-10). BLAST nucleotide searches can be performed with the NBLAST program, score = 100, wordlength = 12 to obtain nucleotide sequences homologous to 53010 nucleic acid molecules of the invention. BLAST protein searches can be performed with the XBLAST program, score = 50, wordlength = 3 to obtain amino acid sequences homologous to protein molecules of the invention. To obtain gapped alignments for comparison purposes, Gapped BLAST can be utilized as described in Altschul et al., (1997, Nucleic Acids Res, 25: 3389-3402). When utilizing BLAST and Gapped BLAST programs, the default parameters of the respective programs (e.g., XBLAST and NBLAST) can be used.

Corresponding HVaSIRS biomarker polynucleotides also include nucleic acid sequences that hybridize to reference HVaSIRS biomarker polynucleotides, or to their complements, under stringency conditions described below. As used herein, the term "hybridizes under low stringency, medium stringency, high stringency, or very high stringency conditions" describes conditions for hybridization and washing. "Hybridization" is used herein to denote the pairing of complementary nucleotide sequences to produce a DNA-DNA hybrid or a DNA-RNA hybrid. Complementary base sequences are those sequences that are related by the base-pairing rules. In DNA, A pairs with T and C pairs with G. In RNA, U pairs with A and C pairs with G. In this regard, the terms "match" and "mismatch" as used herein refer to the hybridization potential of paired nucleotides in complementary nucleic acid strands. Matched nucleotides hybridize efficiently, such as the classical A-T and G-C base pair mentioned above. Mismatches are other combinations of nucleotides that do not hybridize efficiently.

Guidance for performing hybridization reactions can be found in Ausubel *et al.,* (1998, *supra),* Sections 6.3.1-6.3.6. Aqueous and non-aqueous methods are described in that reference and either can be used. Reference herein to low stringency conditions include and encompass from at least about 1% v/v to at least about 15% v/v formamide and from at least about 1 M to at least about 2 M salt for hybridization at 42° C, and at least about 1 M to at least about 2 M salt for washing at 42° C. Low stringency conditions also may include 1% Bovine Serum Albumin (BSA), 1 mM EDTA, 0.5 M NaHPO$_4$ (pH 7.2), 7% SDS for hybridization at 65° C, and (i) 2 × SSC, 0.1% SDS; or (ii) 0.5% BSA, 1 mM EDTA, 40 mM NaHPO$_4$ (pH 7.2), 5% SDS for washing at room temperature. Low stringency conditions may further include hybridization in 6 × sodium chloride/sodium citrate (SSC) at about 45° C, followed by two washes in 0.2 × SSC, 0.1% SDS at least at 50° C (the temperature of the washes can be increased to 55° C for low stringency conditions). Medium stringency conditions include and encompass from at least about 16% v/v to at least about 30% v/v formamide and from at least about 0.5 M to at least about 0.9 M salt for hybridization at 42° C, and at least about 0.1 M to at least about 0.2 M salt for washing at 55° C. Medium stringency conditions also may include 1% Bovine Serum Albumin (BSA), 1 mM EDTA, 0.5 M NaHPO$_4$ (pH 7.2), 7% SDS for hybridization at 65° C, and (i) 2 × SSC, 0.1% SDS; or (ii) 0.5% BSA, 1 mM EDTA, 40 mM NaHPO$_4$ (pH 7.2), 5% SDS for washing at 60-65° C. Medium stringency conditions may further include hybridizing in 6 × SSC at about 45° C, followed by one or more washes in 0.2 × SSC, 0.1% SDS at 60° C. High stringency conditions include and encompass from at least about 31% v/v to at least about 50% v/v formamide and from about 0.01 M to about 0.15 M salt for hybridization at 42° C, and about 0.01 M to about 0.02 M salt for washing at 55° C. High stringency conditions also may include 1% BSA, 1 mM EDTA, 0.5 M NaHPO$_4$ (pH 7.2), 7% SDS for hybridization at 65° C, and (i) 0.2 × SSC, 0.1% SDS; or (ii) 0.5% BSA, 1 mM EDTA, 40 mM NaHPO$_4$ (pH 7.2), 1% SDS for washing at a temperature in excess of 65° C. High stringency conditions may further include hybridizing in 6 × SSC at about 45° C, followed by one or more washes in 0.2 × SSC, 0.1% SDS at 65° C.

A corresponding HVaSIRS biomarker polynucleotide may be one that hybridizes to a disclosed nucleotide sequence under very high stringency conditions. Very high stringency conditions may include hybridizing 0.5 M sodium phosphate, 7% SDS at 65° C, followed by one or more washes at 0.2 × SSC, 1% SDS at 65° C.

Other stringency conditions are well known in the art and a skilled addressee will recognize that various factors can be manipulated to optimize the specificity of the hybridization. Optimization of the stringency of the final washes can serve to ensure a high degree of hybridization. For detailed examples, see Ausubel *et al., supra* at pages 2.10.1 to 2.10.16 and Sambrook *et al.* (1989, *supra*) at sections 1.101 to 1.104.

Generally, a sample is processed prior to HVaSIRS biomarker detection or quantification. For example, nucleic acid and/or proteins may be extracted, isolated, and/or purified from a sample prior to analysis. Various DNA, mRNA, and/or protein extraction techniques are well known to those skilled in the art. Processing may include centrifugation, ultracentrifugation, ethanol precipitation, filtration, fractionation, resuspension, dilution, concentration, *etc.* Methods and systems may provide analysis (e.g., quantification of RNA or protein biomarkers) from raw sample (*e.g.,* biological fluid such as blood, serum, *etc.)* without or with limited processing.

Methods may comprise steps of homogenizing a sample in a suitable buffer, removal of contaminants and/or assay inhibitors, adding a HVaSIRS biomarker capture reagent (*e.g.*, a magnetic bead to which is linked an oligonucleotide complementary to a target HVaSIRS nucleic acid biomarker), incubated under conditions that promote the association (*e.g.*, by hybridization) of the target biomarker with the capture reagent to produce a target biomarker:capture reagent complex, incubating the target biomarker:capture complex under target biomarker-release conditions. Multiple HVaSIRS biomarkers may be isolated in each round of isolation by adding multiple HVaSIRS biomarkers capture reagents (*e.g.*, specific to the desired biomarkers) to the solution. For example, multiple HVaSIRS biomarker capture reagents, each comprising an oligonucleotide specific for a different target HVaSIRS biomarker can be added to the sample for isolation of multiple HVaSIRS biomarker. It is contemplated that the methods encompass multiple experimental designs that vary both in the number of capture steps and in the number of target HVaSIRS biomarker captured in each capture step. Capture reagents include molecules, moieties, substances, or compositions that preferentially (*e.g.*, specifically and selectively) interact with a particular biomarker sought to be isolated, purified, detected, and/or quantified. Any capture reagent having desired binding affinity and/or specificity to the particular HVaSIRS biomarker can be used in the present technology. For example, the capture reagent can be a macromolecule such as a peptide, a protein *(e.g.,* an antibody or receptor), an oligonucleotide, a nucleic acid, *(e.g.,* nucleic acids capable of hybridizing with the HVaSIRS biomarkers), vitamins, oligosaccharides, carbohydrates, lipids, or small molecules, or a complex thereof. As illustrative and non-limiting examples, an avidin target capture reagent may be used to isolate and purify targets comprising a biotin moiety, an antibody may be used to isolate and purify targets comprising the appropriate antigen or epitope, and an oligonucleotide may be used to isolate and purify a complementary oligonucleotide.

Any nucleic acids, including single-stranded and double-stranded nucleic acids, that are capable of binding, or specifically binding, to a target HVaSIRS biomarker can be used as the capture reagent. Examples of such nucleic acids include DNA, RNA, aptamers, peptide nucleic acids, and other modifications to the sugar, phosphate, or nucleoside base. Thus, there are many strategies for capturing a target and accordingly many types of capture reagents are known to those in the art.

In addition, HVaSIRS biomarker capture reagents may comprise a functionality to localize, concentrate, aggregate, *etc.* the capture reagent and thus provide a way to isolate and purify the target HVaSIRS biomarker when captured (*e.g.*, bound, hybridized, *etc.*) to the capture reagent (*e.g.,* when a target:capture reagent complex is formed). For example, the portion of the capture reagent that interacts with the HVaSIRS biomarker (*e.g.,* an oligonucleotide) may be linked to a solid support (*e.g.,* a bead, surface, resin, column, and the like) that allows manipulation by the user on a macroscopic scale. Often, the solid support allows the use of a mechanical means to isolate and purify the target:capture reagent complex from a heterogeneous solution. For example, when linked to a bead, separation is achieved by removing the bead from the heterogeneous solution, *e.g.*, by physical movement. When the bead is magnetic or paramagnetic, a magnetic field may be used to achieve physical separation of the capture reagent (and thus the target HVaSIRS biomarker) from the heterogeneous solution.

The HVaSIRS biomarkers may be quantified or detected using any suitable technique. Specifically, the HVaSIRS biomarkers may be quantified using reagents that determine the level, abundance or amount of individual HVaSIRS biomarkers. Non-limiting reagents of this type include reagents for use in nucleic acid- and protein-based assays.

In illustrative nucleic acid-based assays, nucleic acid is isolated from cells contained in the biological sample according to standard methodologies (Sambrook, *et al.,* 1989, *supra*; and Ausubel *et al.,* 1994, *supra).* The nucleic acid is typically fractionated (*e.g.,* poly $A^+$ RNA) or whole cell RNA. Where RNA is used as the subject of detection, it may be desired to convert the RNA to a complementary DNA. The nucleic acid may be amplified by a template-dependent nucleic acid amplification technique. A number of template dependent processes are available to amplify the HVaSIRS biomarker sequences present in a given template sample. An exemplary nucleic acid amplification technique is the polymerase chain reaction (referred to as PCR), which is described in detail in U.S. Pat. Nos. 4,683,195, 4,683,202 and 4,800,159, Ausubel *et al.* (*supra),* and in Innis et al., ("PCR Protocols", Academic Press, Inc., San Diego Calif., 1990). Briefly, in PCR, two primer sequences are prepared that are complementary to regions on opposite complementary strands of the biomarker sequence. An excess of deoxynucleotide triphosphates are added to a reaction mixture along with a DNA polymerase, *e.g., Taq* polymerase. If a cognate HVaSIRS biomarker sequence is present in a sample, the primers will bind to the biomarker and the polymerase will cause the primers to be extended along the biomarker sequence by adding on nucleotides. By raising and lowering the temperature of the reaction mixture, the extended primers will dissociate from the biomarker to form reaction products, excess primers will bind to the biomarker and to the reaction products and the process is repeated. A reverse transcriptase PCR amplification procedure may be performed in order to quantify the amount of mRNA amplified. Methods of reverse transcribing RNA into cDNA are well known and described in Sambrook *et al.,* 1989, *supra.* Alternative methods for reverse transcription utilize thermostable, RNA-dependent DNA polymerases. These methods are described in WO 90/07641. Polymerase chain reaction methodologies are well known in the art. In specific embodiments in which whole cell RNA is used, cDNA synthesis using whole cell RNA as a sample produces whole cell cDNA.

Advantageously, the template-dependent amplification involves quantification of transcripts in real-time. For example, RNA or DNA may be quantified using the Real-Time PCR technique (Higuchi, 1992, et al., Biotechnology 10: 413-417). By determining the concentration of the amplified products of the target DNA in PCR reactions that have completed the same number of cycles and are in their linear ranges, it is possible to determine the relative concentrations of the specific target sequence in the original DNA mixture. If the DNA mixtures are cDNAs synthesized from RNAs isolated from different tissues or cells, the relative abundance of the specific mRNA from which the target sequence was derived can be determined for the respective tissues or cells. This direct proportionality between the concentration of the PCR products and the relative mRNA abundance is only true in the linear range of the PCR reaction. The final concentration of the target DNA in the plateau portion of the curve is determined by the availability of reagents in the reaction mix and is independent of the original concentration of target DNA. Specifically, multiplexed, tandem PCR (MT-PCR) may be employed, which uses a two-step process for gene expression profiling from small quantities of RNA or DNA, as described for example in US Pat. Appl. Pub. No. 20070190540. In the first step, RNA is converted into cDNA and amplified using multiplexed gene specific primers. In the second step each individual gene is quantitated by real time PCR.

Target nucleic acids may be quantified using blotting techniques, which are well known to those of skill in the art. Southern blotting involves the use of DNA as a target, whereas Northern blotting involves the use of RNA as a target. Each provides different types of information, although cDNA blotting is analogous, in many aspects, to blotting or RNA species. Briefly, a probe is used to target a DNA or RNA species that has been immobilized on a suitable matrix, often a filter of nitrocellulose. The different species should be spatially separated to facilitate analysis. This often is accomplished by gel electrophoresis of nucleic acid species followed by "blotting" on to the filter. Subsequently, the blotted target is incubated with a probe (usually labeled) under conditions that promote denaturation and rehybridization. Because the probe is designed to base pair with the target, the probe will bind a portion of the target sequence under renaturing conditions. Unbound probe is then removed, and detection is accomplished as described above. Following detection/quantification, one may compare the results seen in a given subject with a control reaction or a statistically significant reference group or population of control subjects as defined herein. In this way, it is possible to correlate the amount of HVaSIRS biomarker nucleic acid detected with the progression or severity of the disease.

Also contemplated are biochip-based technologies such as those described by Hacia et al. (1996, Nature Genetics 14: 441-447) and Shoemaker et al. (1996, Nature Genetics 14: 450-456). Briefly, these techniques involve quantitative methods for analyzing large numbers of genes rapidly and accurately. By tagging genes with oligonucleotides or using fixed nucleic acid probe arrays, one can employ biochip technology to segregate target molecules as high-density arrays and screen these molecules on the basis of hybridization. See also Pease et al. (1994, Proc. Natl. Acad. Sci. U.S.A. 91: 5022-5026); Fodor et al. (1991, Science 251: 767-773). Briefly, nucleic acid probes to HVaSIRS biomarker polynucleotides are made and attached to biochips to be used in screening and diagnostic methods, as outlined herein. The nucleic acid probes attached to the biochip are designed to be substantially complementary to specific expressed HVaSIRS biomarker nucleic acids, *i.e.,* the target sequence (either the target sequence of the sample or to other probe sequences, for example in sandwich assays), such that hybridization of the target sequence and the probes of the present invention occur. This complementarity need not be perfect; there may be any number of base pair mismatches, which will interfere with hybridization between the target sequence and the nucleic acid probes of the present invention. However, if the number of mismatches is so great that no hybridization can occur under even the least stringent of hybridization conditions, the sequence is not a complementary target sequence. More than one probe per sequence may be used, with either overlapping probes or probes to different sections of the target being used. That is, two, three, four or more probes, with three being desirable, are used to build in a redundancy for a particular target. The probes can be overlapping (*i.e.* have some sequence in common), or separate.

In an illustrative biochip analysis, oligonucleotide probes on the biochip are exposed to or contacted with a nucleic acid sample suspected of containing one or more HVaSIRS biomarker polynucleotides under conditions favoring specific hybridization. Sample extracts of DNA or RNA, either single or double-stranded, may be prepared from fluid suspensions of biological materials, or by grinding biological materials, or following a cell lysis step which includes, but is not limited to, lysis effected by treatment with SDS (or other detergents), osmotic shock, guanidinium isothiocyanate and lysozyme. Suitable DNA, which may be used in the method of the invention, includes cDNA. Such DNA may be prepared by any one of a number of commonly used protocols as for example described in Ausubel, *et al.,* 1994, *supra,* and Sambrook, *et al.,* 1989, *supra.*

Suitable RNA, which may be used in the method of the invention, includes messenger RNA, complementary RNA transcribed from DNA (cRNA) or genomic or subgenomic RNA. Such RNA may be prepared using standard protocols as for example described in the relevant sections of Ausubel, *et al.* 1994, *supra* and Sambrook, *et al.* 1989, *supra).*

cDNA may be fragmented, for example, by sonication or by treatment with restriction endonucleases. Suitably, cDNA is fragmented such that resultant DNA fragments are of a length greater than the length of the immobilized oligonucleotide probe(s) but small enough to allow rapid access thereto under suitable hybridization conditions. Alternatively, fragments of cDNA may be selected and amplified using a suitable nucleotide amplification technique, as described for example above, involving appropriate random or specific primers.

Usually the target HVaSIRS biomarker polynucleotides are detectably labeled so that their hybridization to individual probes can be determined. The target polynucleotides are typically detectably labeled with a reporter molecule illustrative examples of which include chromogens, catalysts, enzymes, fluorochromes, chemiluminescent molecules, bioluminescent molecules, lanthanide ions (*e.g.,* $Eu^{34}$), a radioisotope and a direct visual label. In the case of a direct visual label, use may be made of a colloidal metallic or non-metallic particle, a dye particle, an enzyme or a substrate, an organic polymer, a latex particle, a liposome, or other vesicle containing a signal producing substance and the like. Illustrative labels of this type include large colloids, for example, metal colloids such as those from gold, selenium, silver, tin and titanium oxide. When an enzyme is used as a direct visual label, biotinylated bases may be incorporated into a target polynucleotide.

The hybrid-forming step can be performed under suitable conditions for hybridizing oligonucleotide probes to test nucleic acid including DNA or RNA. In this regard, reference may be made, for example, to NUCLEIC ACID HYBRIDIZATION, A PRACTICAL APPROACH (Homes and Higgins, eds.) (IRL press, Washington D.C., 1985). In general, whether hybridization takes place is influenced by the length of the oligonucleotide probe and the polynucleotide sequence under test, the pH, the temperature, the concentration of mono- and divalent cations, the proportion of G and C nucleotides in the hybrid-forming region, the viscosity of the medium and the possible presence of denaturants. Such variables also influence the time required for hybridization. The preferred conditions will therefore depend upon the particular application. Such empirical conditions, however, can be routinely determined without undue experimentation.

After the hybrid-forming step, the probes are washed to remove any unbound nucleic acid with a hybridization buffer. This washing step leaves only bound target polynucleotides. The probes are then examined to identify which probes have hybridized to a target polynucleotide.

The hybridization reactions are then detected to determine which of the probes has hybridized to a corresponding target sequence. Depending on the nature of the reporter molecule associated with a target polynucleotide, a signal may be instrumentally detected by irradiating a fluorescent label with light and detecting fluorescence in a fluorimeter; by providing for an enzyme system to produce a dye which could be detected using a spectrophotometer; or detection of a dye particle or a colored colloidal metallic or non-metallic particle using a reflectometer; in the case of using a radioactive label or chemiluminescent molecule employing a radiation counter or autoradiography. Accordingly, a detection means may be adapted to detect or scan light associated with the label which light may include fluorescent, luminescent, focused beam or laser light. In such a case, a charge couple device (CCD) or a photocell can be used to scan for emission of light from a probe:target polynucleotide hybrid from each location in the micro-array and record the data directly in a digital computer. In some cases, electronic detection of the signal may not be necessary. For example, with enzymatically generated color spots associated with nucleic acid array format, visual examination of the array will allow interpretation of the pattern on the array. In the case of a nucleic acid array, the detection means is suitably interfaced with pattern recognition software to convert the pattern of signals from the array into a plain language genetic profile. Oligonucleotide probes specific for different HVaSIRS biomarker polynucleotides may be in the form of a nucleic acid array and detection of a signal generated from a reporter molecule on the array is performed using a 'chip reader'. A detection system that can be used by a 'chip reader' is described for example by Pirrung et al. (U.S. Patent No. 5,143,854). The chip reader will typically also incorporate some signal processing to determine whether the signal at a particular array position or feature is a true positive or maybe a spurious signal. Exemplary chip readers are described for example by Fodor et al. (U.S. Patent No., 5,925,525). Alternatively, when the array is made using a mixture of individually addressable kinds of labeled microbeads, the reaction may be detected using flow cytometry.

In certain embodiments, the HVaSIRS biomarker is a target RNA *(e.g.,* mRNA) or a DNA copy of the target RNA whose level or abundance is measured using at least one nucleic acid probe that hybridizes under at least low, medium, or high stringency conditions to the target RNA or to the DNA copy, wherein the nucleic acid probe comprises at least 15 *(e.g.,* 15, 16, 17, 18, 19, 20, 21, 22, 23, 24, 25, 26, 27, 28, 29, 30, or more) contiguous nucleotides of HVaSIRS biomarker polynucleotide. In some embodiments, the measured level or abundance of the target RNA or its DNA copy is normalized to the level or abundance of a reference RNA or a DNA copy of the reference RNA. Suitably, the nucleic acid probe is immobilized on a solid or semi-solid support. In illustrative examples of this type, the nucleic acid probe forms part of a spatial array of nucleic acid probes. In some embodiments, the level of nucleic acid probe that is bound to the target RNA or to the DNA copy is measured by hybridization (*e.g.,* using a nucleic acid array). In other embodiments, the level of nucleic acid probe that is bound to the target RNA or to the DNA copy is measured by nucleic acid amplification (*e.g.,* using a polymerase chain reaction (PCR)). In still other embodiments, the level of nucleic acid probe that is bound to the target RNA or to the DNA copy is measured by nuclease protection assay.

Sequencing technologies such as Sanger sequencing, pyrosequencing, sequencing by ligation, massively parallel sequencing, also called "Next-generation sequencing" (NGS), and other high-throughput sequencing approaches with or without sequence amplification of the target can also be used to detect or quantify the presence of HVaSIRS nucleic acid biomarker in a sample. Sequence-based methods can provide further information regarding alternative splicing and sequence variation in previously identified genes. Sequencing technologies include a number of steps that are grouped broadly as template preparation, sequencing, detection and data analysis. Current methods for template preparation involve randomly breaking genomic DNA into smaller sizes from which each fragment is immobilized to a support. The immobilization of spatially separated fragment allows thousands to billions of sequencing reaction to be performed simultaneously. A sequencing step may use any of a variety of methods that are commonly known in the art. One specific example of a sequencing step uses the addition of nucleotides to the complementary strand to provide the DNA sequence. The detection steps range from measuring bioluminescent signal of a synthesized fragment to four-color imaging of single molecule. When NGS is used to detect or quantify the presence of HVaSIRS nucleic acid biomarker in a sample, the methods may be suitably selected from semiconductor sequencing (Ion Torrent; Personal Genome Machine); Helicos True Single Molecule Sequencing (tSMS) (Harris et al. 2008, Science 320:106-109); 454 sequencing (Roche) (Margulies et al. 2005, Nature, 437, 376-380); SOLiD technology (Applied Biosystems); SOLEXA sequencing (Illumina); single molecule, real-time (SMRT™) technology of Pacific Biosciences; nanopore sequencing (Soni and Meller, 2007. Clin Chem 53: 1996-2001); DNA nanoball sequencing; sequencing using technology from Dover Systems (Polonator), and technologies that do not require amplification or otherwise transform native DNA prior to sequencing (*e.g.,* Pacific Biosciences and Helicos), such as nanopore-based strategies (*e.g.,* Oxford Nanopore, Genia Technologies, and Nabsys).

In other embodiments, HVaSIRS biomarker protein levels are assayed using protein-based assays known in the art. For example, when HVaSIRS biomarker protein is an enzyme, the protein can be quantified based upon its catalytic activity or based upon the number of molecules of the protein contained in a sample. Antibody-based techniques may be employed including, for example, immunoassays, such as the enzyme-linked immunosorbent assay (ELISA) and the radioimmunoassay (RIA).

Specifically, protein-capture arrays that permit simultaneous detection and/or quantification of a large number of proteins may be employed. For example, low-density protein arrays on filter membranes, such as the universal protein array system (Ge, 2000 Nucleic Acids Res. 28(2):e3) allow imaging of arrayed antigens using standard ELISA techniques and a scanning charge-coupled device (CCD) detector. Immuno-sensor arrays have also been developed that enable the simultaneous detection of clinical analytes. It is now possible using protein arrays, to profile protein expression in bodily fluids, such as in sera of healthy or diseased subjects, as well as in subjects pre- and post-drug treatment.

Exemplary protein capture arrays include arrays comprising spatially addressed antigen-binding molecules, commonly referred to as antibody arrays, which can facilitate extensive parallel analysis of numerous proteins defining a proteome or subproteome. Antibody arrays have been shown to have the required properties of specificity and acceptable background, and some are available commercially (*e.g.*, BD Biosciences, Clontech, Bio-Rad and Sigma). Various methods for the preparation of antibody arrays have been reported (see, *e.g.,* Lopez et al., 2003 J. Chromatogram. B 787:19-27; Cahill, 2000 Trends in Biotechnology 7:47-51; U.S. Pat. App. Pub. 2002/0055186; U.S. Pat. App. Pub. 2003/0003599; PCT publication WO 03/062444; PCT publication WO 03/077851; PCT publication WO 02/59601; PCT publication WO 02/39120; PCT publication WO 01/79849; PCT publication WO 99/39210). The antigen-binding molecules of such arrays may recognize at least a subset of proteins expressed by a cell or population of cells, illustrative examples of which include growth factor receptors, hormone receptors, neurotransmitter receptors, catecholamine receptors, amino acid derivative receptors, cytokine receptors, extracellular matrix receptors, antibodies, lectins, cytokines, serpins, proteases, kinases, phosphatases, ras-like GTPases, hydrolases, steroid hormone receptors, transcription factors, heat-shock transcription factors, DNA-binding proteins, zinc-finger proteins, leucine-zipper proteins, homeodomain proteins, intracellular signal transduction modulators and effectors, apoptosis-related factors, DNA synthesis factors, DNA repair factors, DNA recombination factors and cell-surface antigens.

Individual spatially distinct protein-capture agents are typically attached to a support surface, which is generally planar or contoured. Common physical supports include glass slides, silicon, microwells, nitrocellulose or PVDF membranes, and magnetic and other microbeads.

Particles in suspension can also be used as the basis of arrays, providing they are coded for identification; systems include color coding for microbeads (*e.g.*, available from Luminex, Bio-Rad and Nanomics Biosystems) and semiconductor nanocrystals (*e.g.*, QDots™, available from Quantum Dots), and barcoding for beads (UltraPlex™, available from Smartbeads) and multimetal microrods (Nanobarcodes™ particles, available from Surromed). Beads can also be assembled into planar arrays on semiconductor chips (*e.g.*, available from LEAPS technology and BioArray Solutions). Where particles are used, individual protein-capture agents are typically attached to an individual particle to provide the spatial definition or separation of the array. The particles may then be assayed separately, but in parallel, in a compartmentalized way, for example in the wells of a microtiter plate or in separate test tubes.

In operation, a protein sample, which is optionally fragmented to form peptide fragments (see, *e.g.,* U.S. Pat. App. Pub. 2002/0055186), is delivered to a protein-capture array under conditions suitable for protein or peptide binding, and the array is washed to remove unbound or non-specifically bound components of the sample from the array. Next, the presence or amount of protein or peptide bound to each feature of the array is detected using a suitable detection system. The amount of protein bound to a feature of the array may be determined relative to the amount of a second protein bound to a second feature of the array. The amount of the second protein in the sample may be already known or known to be invariant.

In specific embodiments, the HVaSIRS biomarker is a target polypeptide whose level is measured using at least one antigen-binding molecule that is immuno-interactive with the target polypeptide. In these embodiments, the measured level of the target polypeptide is normalized to the level of a reference polypeptide. Suitably, the antigen-binding molecule is immobilized on a solid or semi-solid support. In illustrative examples of this type, the antigen-binding molecule forms part of a spatial array of antigen-binding molecule. The level of antigen-binding molecule that is bound to the target polypeptide may be measured by immunoassay (*e.g.*, using an ELISA).

All the essential reagents required for detecting and quantifying the HVaSIRS biomarkers of the invention may be assembled together in a kit. In some embodiments, the kit comprises a reagent that permits quantification of at least one HVaSIRS biomarker. In some embodiments the kit comprises: (i) a reagent that allows quantification (*e.g.*, determining the level or abundance) of a first HVaSIRS biomarker; and (ii) a reagent that allows quantification (*e.g.*, determining the level or abundance) of a second HVaSIRS biomarker, wherein the first and second biomarkers have a mutual correlation in respect of HVaSIRS that lies within a mutual correlation range of between $\pm 0.9$, and wherein a combination of respective biomarker values for the first and second HVaSIRS biomarkers that are measured for or derived from a subject has a performance value greater than or equal to a performance threshold representing the ability of the combination of the first and second HVaSIRS biomarkers to diagnose the presence, absence or degree of the at least one condition, or to provide a prognosis for the at least one condition, the performance threshold being a variance explained of at least 0.3. In some embodiments, the kit further comprises (iii) a reagent that allows quantification (*e.g.*, determining the level or abundance) of a third HVaSIRS biomarker; and (iv) a reagent that allows quantification (*e.g.*, determining the level or abundance) of a fourth HVaSIRS biomarker, wherein the third and fourth HVaSIRS biomarkers have a mutual correlation in respect of HVaSIRS that lies within a mutual correlation range of between $\pm 0.9$, and wherein a combination of respective biomarker values for the third and fourth HVaSIRS biomarkers that are measured for or derived from a subject has a performance value greater than or equal to a performance threshold representing the ability of the combination of the third and fourth HVaSIRS biomarkers to diagnose the presence, absence or degree of HVaSIRS, or to provide a prognosis for a HVaSIRS, the performance threshold being a variance explained of at least 0.3. In some embodiments, the kit further comprises (v) a reagent that allows quantification (*e.g.*, determining the level or abundance) of a fifth HVaSIRS biomarker; and (vi) a reagent that allows quantification (*e.g.*, determining the level or abundance) of a sixth HVaSIRS biomarker, wherein the fifth and sixth HVaSIRS biomarkers have a mutual correlation in respect of HVaSIRS that lies within a mutual correlation range of between $\pm 0.9$, and wherein a combination of respective biomarker values for the fifth and sixth HVaSIRS biomarkers that are measured for or derived from a subject has a performance value greater than or equal to a performance threshold representing the ability of the combination of the fifth and sixth HVaSIRS biomarkers to diagnose the presence, absence or degree of HVaSIRS, or to provide a prognosis for a HVaSIRS, the performance threshold being a variance explained of at least 0.3.

In the context of the present invention, "kit" is understood to mean a product containing the different reagents necessary for carrying out the methods of the invention packed so as to allow their transport and storage. Materials suitable for packing the components of the kit include crystal, plastic (polyethylene, polypropylene, polycarbonate and the like), bottles, vials, paper, envelopes and the like. Additionally, the kits of the invention can contain instructions for the simultaneous, sequential or separate use of the different components contained in the kit. The instructions can be in the form of printed material or in the form of an electronic support capable of storing instructions such that they can be read by a subject, such as electronic storage media (magnetic disks, tapes and the like), optical media (CD-ROM, DVD) and the like. Alternatively, or in addition, the media can contain Internet addresses that provide the instructions.

Reagents that allow quantification of a HVaSIRS biomarker include compounds or materials, or sets of compounds or materials, which allow quantification of the HVaSIRS biomarker. Specifically, the compounds, materials or sets of compounds or materials may permit determining the expression level of a gene (*e.g.*, HVaSIRS biomarker gene), including without limitation the extraction of RNA material, the determination of the level of a corresponding RNA, *etc.,* primers for the synthesis of a corresponding cDNA, primers for amplification of DNA, and/or probes capable of specifically hybridizing with the RNAs (or the corresponding cDNAs) encoded by the genes, TaqMan probes, *etc.*

The kits may also optionally include appropriate reagents for detection of labels, positive and negative controls, washing solutions, blotting membranes, microtiter plates, dilution buffers and the like. For example, a nucleic acid-based detection kit may include (i) a HVaSIRS biomarker polynucleotide (which may be used as a positive control), (ii) a primer or probe that specifically hybridizes to a HVaSIRS biomarker polynucleotide. Also included may be enzymes suitable for amplifying nucleic acids including various polymerases (reverse transcriptase, *Taq,* Sequenase™, DNA ligase *etc.* depending on the nucleic acid amplification technique employed), deoxynucleotides and buffers to provide the necessary reaction mixture for amplification. Such kits also generally will comprise, in suitable means, distinct containers for each individual reagent and enzyme as well as for each primer or probe. Alternatively, a protein-based detection kit may include (i) a HVaSIRS biomarker polypeptide (which may be used as a positive control), (ii) an antibody that binds specifically to a HVaSIRS biomarker polypeptide. The kit can also feature various devices (*e.g.*, one or more) and reagents (*e.g.*, one or more) for performing one of the assays described herein; and/or printed instructions for using the kit to quantify the expression of a HVaSIRS biomarker gene.

The reagents described herein, which may be optionally associated with detectable labels, can be presented in the format of a microfluidics card, a chip or chamber, a microarray or a kit adapted for use with the assays described in the examples or below, *e.g.,* RT-PCR or Q PCR techniques described herein.

The reagents also have utility in compositions for detecting and quantifying the biomarkers of the invention. For example, a reverse transcriptase may be used to reverse transcribe RNA transcripts, including mRNA, in a nucleic acid sample, to produce reverse transcribed transcripts, including reverse transcribed mRNA (also referred to as "cDNA"). Specifically, the reverse transcribed mRNA may be whole cell reverse transcribed mRNA (also referred to herein as "whole cell cDNA"). The nucleic acid sample is suitably derived from components of the immune system, representative examples of which include components of the innate and adaptive immune systems as broadly discussed for example above. Specifically, the reverse transcribed RNA may be derived blood cells (*e.g.*, peripheral blood cells). Suitably, the reverse transcribed RNA is derived leukocytes.

The reagents are suitably used to quantify the reverse transcribed transcripts. For example, oligonucleotide primers that hybridize to the reverse transcribed transcript can be used to amplify at least a portion of the reverse transcribed transcript *via* a suitable nucleic acid amplification technique, *e.g.*, RT-PCR or qPCR techniques described herein. Alternatively, oligonucleotide probes may be used to hybridize to the reverse transcribed transcript for the quantification, using a nucleic acid hybridization analysis technique (*e.g.*, microarray analysis), as described for example above. Thus, a respective oligonucleotide primer or probe may be hybridized to a complementary nucleic acid sequence of a reverse transcribed transcript in the compositions of the invention. The compositions typically comprise labeled reagents for detecting and/or quantifying the reverse transcribed transcripts. Representative reagents of this type include labeled oligonucleotide primers or probes that hybridize to RNA transcripts or reverse transcribed RNA, labeled RNA, labeled reverse transcribed RNA as well as labeled oligonucleotide linkers or tags (e.g., a labeled RNA or DNA linker or tag) for labeling (e.g., end labeling such as 3' end labeling) RNA or reverse transcribed RNA. The primers, probes, RNA or reverse transcribed RNA (i.e., cDNA) (whether labeled or non-labeled) may be immobilized or free in solution. Representative reagents of this type include labeled oligonucleotide primers or probes that hybridize to reverse transcribed and transcripts as well as labeled reverse transcribed transcripts. The label can be any reporter molecule as known in the art, illustrative examples of which are described above and elsewhere herein.

Disclosed in the context of the present invention are also non-reverse transcribed RNA in which cDNA is not made and the RNA transcripts are directly the subject of the analysis. Thus, reagents may be suitably used to quantify RNA transcripts directly. For example, oligonucleotide probes can be used to hybridize to transcripts for quantification of immune system biomarkers of the invention, using a nucleic acid hybridization analysis technique (e.g., microarray analysis), as described for example above. Thus, a respective oligonucleotide probe may be hybridized to a complementary nucleic acid sequence of an immune system biomarker transcript in the compositions of the invention. In illustrative examples of this type, the compositions may comprise labeled reagents that hybridize to transcripts for detecting and/or quantifying the transcripts. Representative reagents of this type include labeled oligonucleotide probes that hybridize to transcripts as well as labeled transcripts. The primers or probes may be immobilized or free in solution.

Disclosed in the context of the present invention is also the management of HVaSIRS, or prevention of further progression of HVaSIRS, or assessment of the efficacy of therapies in subjects following positive diagnosis for the presence of HVaSIRS, in a subject. Once a subject is positively identified as having HVaSIRS, the subject may be administered a therapeutic agent for treating the HVaSIRS such as an anti-herpes viral agent, illustrative examples of which include; Aciclovir, Brivudine, Cidofovir, Famciclovir, Fomivirsen, Foscarnet, Ganciclovir, HDP-CDV, Idoxuridine, Letermovir, Maribavir, Penciclovir, Resiquimod, Sorivudine, Trifluridine, Tromantadine, Valaciclovir, Valganciclovir, Vidarabine or salts and combinations thereof.

Typically, the therapeutic agents will be administered in pharmaceutical (or veterinary) compositions together with a pharmaceutically acceptable carrier and in an effective amount to achieve their intended purpose. The dose of active compounds administered to a subject should be sufficient to achieve a beneficial response in the subject over time such as a reduction in, or relief from, the symptoms of HVaSIRS. The quantity of the pharmaceutically active compounds(s) to be administered may depend on the subject to be treated inclusive of the age, sex, weight and general health condition thereof. In this regard, precise amounts of the active compound(s) for administration will depend on the judgment of the practitioner. In determining the effective amount of the active compound(s) to be administered in the treatment or prevention of HVaSIRS, the medical practitioner or veterinarian may evaluate severity of any symptom or clinical sign associated with the presence of HVaSIRS or degree of HVaSIRS including, inflammation, blood pressure anomaly, tachycardia, tachypnea fever, chills, vomiting, diarrhea, skin rash, headaches, confusion, muscle aches, seizures. In any event, those of skill in the art may readily determine suitable dosages of the therapeutic agents and suitable treatment regimens without undue experimentation.

The therapeutic agents may be administered in concert with adjunctive (palliative) therapies to increase oxygen supply to major organs, increase blood flow to major organs and/or to reduce the inflammatory response. Illustrative examples of such adjunctive therapies include non-steroidal-anti-inflammatory drugs (NSAIDs), intravenous saline and oxygen.

Also contemplated in the context of the present invention is the use of the indicator-determining methods, apparatus, compositions and kits disclosed herein in methods of treating, preventing or inhibiting the development of HVaSIRS in a subject. These methods (also referred to herein as "treatment methods") generally comprise: exposing the subject to a treatment regimen for treating HVaSIRS, or avoiding exposing the subject to a treatment regimen for treating a SIRS other than HVaSIRS based on an indicator obtained from an indicator-determining method as disclosed herein. Specifically, the treatment methods may compris: (a) determining a plurality of biomarker values for at least two (e.g., 2, 3, 4, 5, 6, 7, 8, 9, 10, or more) HVaSIRS biomarkers of the subject, each biomarker value being indicative of a value measured or derived for a respective HVaSIRS biomarker; (b) determining an indicator using a combination of the plurality of biomarker values, the indicator being at least partially indicative of the presence, absence or degree of HVaSIRS, wherein: (i) at least two (e.g., 2, 3, 4, 5, 6, 7, 8, 9, 10, or more) HVaSIRS biomarkers have a mutual correlation in respect of the at least one condition that lies within a mutual correlation range, the mutual correlation range being between ±0.9; and (ii) the indicator has a performance value greater than or equal to a performance threshold representing the ability of the indicator to diagnose the presence, absence or degree of HVaSIRS, the performance threshold being indicative of an explained variance of at least 0.3; and (c) administering to the subject, on the basis that the indicator indicates the presence of HVaSIRS, an effective amount of an agent that treats or ameliorates the symptoms or reverses or inhibits the development of HVaSIRS.

Advantageously, the treatment methods comprise: (1) determining a plurality of measured biomarker values, each measured biomarker value being a measured value of an individual HVaSIRS biomarker of the subject; and (2) applying a function to at least two of the measured biomarker values to determine at least one derived biomarker value, the at least one derived biomarker value being indicative of a value of a corresponding derived HVaSIRS biomarker. The function suitably includes at least one of: (a) multiplying two biomarker values; (b) dividing two biomarker values; (c) adding two biomarker values; (d) subtracting two biomarker values; (e) a weighted sum of at least two biomarker values; (f) a log sum of at least two biomarker values; (g) a geometric mean of at least two biomarker values; and (h) a sigmoidal function of at least two biomarker values.

The present invention can be practiced in the field of predictive medicine for the purpose of diagnosis or monitoring the presence or development of HVaSIRS in a subject, and/or monitoring response to therapy efficacy. The biomarker profiles and corresponding indicators of the present invention further enable determination of endpoints in pharmacotranslational studies. For example, clinical trials can take many months or even years to establish the pharmacological parameters for a medicament to be used in treating or preventing HVaSIRS. However, these parameters may be associated with a biomarker profile and corresponding indicator of a health state (*e.g.*, a healthy condition). Hence, the clinical trial can be expedited by selecting a treatment regimen (*e.g.*, medicament and pharmaceutical parameters), which results in a biomarker profile associated with a desired health state (*e.g.*, healthy condition). This may be determined for example by: (1) determining biomarker values that are measured or derived for at least two (*e.g.*, 2, 3, 4, 5, 6, 7, 8, 9, 10, or more) corresponding HVaSIRS biomarker of a subject after treatment with a treatment regimen; (2) determining the indicator using the biomarker values; and (3) determining that the treatment regimen is effective for changing the health status of the subject to the desired health state (*e.g.*, healthy condition) on the basis that the indicator indicates the presence of a healthy condition or the presence of a condition of a lower degree relative to the degree of the condition in the subject before treatment with the treatment regimen. As used herein, the term "degree" refers to the extent or stage of a condition. Alternatively, selection of the treatment regimen may be determined by: (a) determining a plurality of biomarker values for at least two (*e.g.*, 2, 3, 4, 5, 6, 7, 8, 9, 10, or more) HVaSIRS biomarkers of a subject after treatment with a treatment regimen, each biomarker value being indicative of a value measured or derived for a respective HVaSIRS biomarker; (b) determining an indicator using a combination of the plurality of HVaSIRS biomarker values, the indicator being at least partially indicative of the presence, absence or degree of at least one condition selected from a healthy condition or HVaSIRS, wherein: (i) at least two (*e.g.*, 2, 3, 4, 5, 6, 7, 8, 9, 10, or more) HVaSIRS biomarkers have a mutual correlation in respect of the at least one condition that lies within a mutual correlation range, the mutual correlation range being between ±0.9; and (ii) the indicator has a performance value greater than or equal to a performance threshold representing the ability of the indicator to diagnose the presence, absence or degree of the at least one condition, or to provide a prognosis for the at least one condition, the performance threshold being indicative of an explained variance of at least 0.3, and (c) determining that the treatment regimen is effective for changing the health status of the subject to the desired health state (*e.g.*, healthy condition) on the basis that the indicator indicates the presence of a healthy condition or the presence of HVaSIRS of a lower degree relative to the degree of HVaSIRS in the subject before treatment with the treatment regimen. Accordingly, this advantageously provides methods of monitoring the efficacy of a particular treatment regimen in a subject (for example, in the context of a clinical trial) already diagnosed with HVaSIRS. These methods take advantage of measured or derived biomarker values that correlate with treatment efficacy to determine, for example, whether measured or derived biomarker values of a subject undergoing treatment partially or completely normalize during the course of or following therapy or otherwise shows changes associated with responsiveness to the therapy.

Thus, further disclosed in the context of the invention are methods of correlating a biomarker profile with an effective treatment regimen for HVaSIRS. These methods may comprise: (1) determining a biomarker profile defining biomarker values that are measured or derived for at least two (*e.g.*, 2, 3, 4, 5, 6, 7, 8, 9, 10, or more) corresponding HVaSIRS biomarkers of a subject with HVaSIRS and for whom an effective treatment has been identified; and (2) correlating the biomarker profile so determined with an effective treatment regimen for HVaSIRS. These methods may also comprise: (a) determining a biomarker profile defining a combination of at least two (*e.g.*, 2, 3, 4, 5, 6, 7, 8, 9, 10, or more) biomarker values corresponding to values of at least two HVaSIRS biomarkers that can be measured or derived for a subject with HVaSIRS and for whom an effective treatment has been identified, wherein: (i) the at least two HVaSIRS biomarkers have a mutual correlation in respect of the condition that lies within a mutual correlation range, the mutual correlation range being between ± 0.9; and (ii) the combination of at least two biomarker values has a performance value greater than or equal to a performance threshold representing the ability of the combination of at least two biomarker values to diagnose the presence, absence or degree of HVaSIRS, or to provide a prognosis for the HVaSIRS, the performance threshold being indicative of an explained variance of at least 0.3; and (b) correlating the biomarker profile so determined with an effective treatment regimen for HVaSIRS. Specifically, an indicator or biomarker profile may be correlated to a global probability or a particular outcome, using ROC curves.

Further disclosed in the context of the invention are methods of determining whether a treatment regimen is effective for treating a subject with HVaSIRS. These methods may comprise: (1) determining post-treatment biomarker values that are measured or derived for at least two (*e.g.*, 2, 3, 4, 5, 6, 7, 8, 9, 10, or more) corresponding HVaSIRS biomarkers of a subject after treatment with a treatment regimen; (2) determining a post-treatment indicator using the post-treatment biomarker values, wherein the post-treatment indicator is at least partially indicative of the presence, absence or degree of HVaSIRS, wherein the post-treatment indicator indicates whether the treatment regimen is effective for treating HVaSIRS in the subject on the basis that post-treatment indicator indicates the presence of a healthy condition or the presence of HVaSIRS of a lower degree relative to the degree of HVaSIRS in the subject before treatment with the treatment regimen. Alternatively, these methods may comprise: (a) determining a plurality of post-treatment biomarker values, each post-treatment HVaSIRS biomarker value being indicative of a value measured or derived for at least one (e.g., 1, 2, 3, 4, 5, 6, 7, 8, 9, 10, or more) HVaSIRS biomarker of a subject after treatment with the treatment regimen; (b) determining a post-treatment indicator using a combination of the plurality of post-treatment biomarker values, the post-treatment indicator being at least partially indicative of the presence, absence or degree of HVaSIRS, wherein: (i) at the least two (e.g., 2, 3, 4, 5, 6, 7, 8, 9, 10, or more) HVaSIRS biomarkers have a mutual correlation in respect of the at least one condition that lies within a mutual correlation range, the mutual correlation range being between ±0.9; and (ii) the post-treatment indicator has a performance value greater than or equal to a performance threshold representing the ability of the post-treatment indicator to diagnose the presence, absence or degree of HVaSIRS, the performance threshold being indicative of an explained variance of at least 0.3, wherein the post-treatment indicator indicates whether the treatment regimen is effective for treating the HVaSIRS in the subject on the basis that post-treatment indicator indicates the presence of a healthy condition or the presence of HVaSIRS of a lower degree relative to the degree of HVaSIRS in the subject before treatment with the treatment regimen.

The invention can also be practiced to evaluate whether a subject is responding (i.e., a positive response) or not responding (i.e., a negative response) to a treatment regimen. Thus, further disclosed in the context of the invention are methods of correlating a biomarker profile with a positive or negative response to a treatment regimen. These methods may comprise: (1) determining a biomarker profile defining biomarker values that are measured or derived for at least two (e.g., 2, 3, 4, 5, 6, 7, 8, 9, 10, or more) corresponding HVaSIRS biomarkers of a subject following commencement of the treatment regimen; and (2) correlating the biomarker profile so determined with a positive or negative response to the treatment regimen. Alternatively, these methods may comprise: (a) determining a biomarker profile defining a combination of at least two (e.g., 2, 3, 4, 5, 6, 7, 8, 9, 10, or more) biomarker values corresponding to values of at least two VaSIRS biomarkers that can be measured or derived for a subject following commencement of the treatment regimen, wherein: (i) the at least two HVaSIRS biomarkers have a mutual correlation in respect of HVaSIRS, which lies within a mutual correlation range, the mutual correlation range being between ± 0.9; and (ii) the combination of at least two biomarker values has a performance value greater than or equal to a performance threshold representing the ability of the combination of at least two biomarker values to diagnose the presence, absence or degree of HVaSIRS, or to provide a prognosis for the HVaSIRS, the performance threshold being indicative of an explained variance of at least 0.3; and (b) correlating the biomarker profile so determined with a positive or negative response to the treatment regimen.

Also disclosed in the context of the invention are methods of determining a positive or negative response to a treatment regimen by a subject with HVaSIRS. These methods may comprise: (1) correlating a reference biomarker profile with a positive or negative response to the treatment regimen, wherein the biomarker profile defines biomarker values that are measured or derived for at least two (e.g., 2, 3, 4, 5, 6, 7, 8, 9, 10, or more) corresponding HVaSIRS biomarkers of a control subject or control group; (2) determining a sample biomarker profile defining biomarker values that are measured or derived for the at least two (e.g., 2, 3, 4, 5, 6, 7, 8, 9, 10, or more) corresponding HVaSIRS biomarker of the subject following commencement of the treatment regimen, wherein the sample biomarker profile indicates whether the subject is responding positively or negatively to the treatment regimen, based on the correlation of the reference biomarker signature with the positive or negative response to the treatment regimen. Alternatively, the methods may comprise: (a) correlating a reference biomarker profile with a positive or negative response to the treatment regimen, wherein the biomarker profile defines a combination of at least two (e.g., 2, 3, 4, 5, 6, 7, 8, 9, 10, or more) biomarker values corresponding to values of at least two HVaSIRS biomarkers that are measured for or derived from a control subject or control group, wherein: (i) the at least two HVaSIRS biomarkers have a mutual correlation in respect of HVaSIRS, which lies within a mutual correlation range, the mutual correlation range being between ± 0.9; and (ii) the combination of at least two biomarker values has a performance value greater than or equal to a performance threshold representing the ability of the combination of at least two biomarker values to diagnose the presence, absence or degree of HVaSIRS, or to provide a prognosis for the HVaSIRS, the performance threshold being indicative of an explained variance of at least 0.3; (b) determining a sample biomarker profile defining a combination of at least two (e.g., 2, 3, 4, 5, 6, 7, 8, 9, 10, or more) biomarker values corresponding to values of the at least two VaSIRS biomarkers that are measured or derived from the subject following commencement of the treatment regimen, wherein the sample biomarker profile indicates whether the subject is responding positively or negatively to the treatment regimen, based on the correlation of the reference biomarker profile with the positive or negative response to the treatment regimen.

In the context of the invention, further contemplated are methods of determining a positive or negative response to a treatment regimen by a biological subject. These methods may comprise: (1) determining a sample biomarker profile defining biomarker values that are measured or derived for at least two (e.g., 2, 3, 4, 5, 6, 7, 8, 9, 10, or more) corresponding HVaSIRS biomarker of a subject following commencement of the treatment regimen, wherein the sample biomarker profile is correlated with a positive or negative response to the treatment regimen; and (2) determining whether the subject is responding positively or negatively to the treatment regimen based on the sample biomarker profile. In other embodiments, these methods comprise: (a) determining a sample biomarker profile defining a combination of at least two (e.g., 2, 3, 4, 5, 6, 7, 8, 9, 10, or more) biomarker values corresponding to values of at least two HVaSIRS biomarkers that are measured for or derived from a subject following commencement of the treatment regimen, wherein: (i) the at least two HVaSIRS biomarkers have a mutual correlation in respect of HVaSIRS, which lies within a mutual correlation range, the mutual correlation range being between ±0.9; and (ii) the combination of at least two biomarker values has a performance value greater than or equal to a performance threshold representing the ability of the combination of at least two biomarker values to diagnose the presence, absence or degree of HVaSIRS, or to provide a prognosis for the HVaSIRS, the performance threshold being indicative of an explained variance of at least 0.3, wherein the sample biomarker profile is correlated with a positive or negative response to the treatment regimen; and (b) determining whether the subject is responding positively or negatively to the treatment regimen based on the sample biomarker profile.

The above methods can be practiced to identify responders or non-responders relatively early in the treatment process, i.e., before clinical manifestations of efficacy. In this way, the treatment regimen can optionally be discontinued, a different treatment protocol can be implemented and/or supplemental therapy can be administered. Thus, a sample HVaSIRS biomarker profile may be obtained within about 2 hours, 4 hours, 6 hours, 12 hours, 1 day, 2 days, 3 days, 4 days, 5 days, 1 week, 2 weeks, 3 weeks, 4 weeks, 6 weeks, 8 weeks, 10 weeks, 12 weeks, 4 months, six months or longer of commencing therapy.

Also contemplated in the context of the present invention are methods in which the indicator-determining method of the invention is implemented using one or more processing devices. These methods may comprise: (1) determining a pair of biomarker values, the pair of biomarker values being selected from the group consisting of: (a) a first pair of biomarker values indicative of a concentration of polynucleotide expression products of a Group A HVaSIRS biomarker gene (e.g., CCL5) and a Group B HVaSIRS biomarker gene (e.g., FLNB); and (b) a second pair of biomarker values indicative of a concentration of polynucleotide expression products of a Group C HVaSIRS biomarker gene (e.g., PPP2R2B) gene and a Group D HVaSIRS biomarker gene (e.g., GNG7); and (c) a third pair of biomarker values indicative of a concentration of polynucleotide expression products of a Group E HVaSIRS biomarker gene (e.g., CASZ' 1) gene and a Group F HVaSIRS biomarker gene (e.g., VASH1); (2) determining an indicator indicative of a ratio of the concentrations of the polynucleotide expression products using all three biomarker values; (3) retrieving previously determined first, second and third indicator references from a database, the first, second and third indicator references being determined based on indicators determined from first, second and third groups of a reference population, one of the groups consisting of individuals diagnosed with HVaSIRS; (4) comparing the indicator to the first, second and third indicator references; (5) using the results of the comparison to determine a probability indicative of the subject having or not having HVaSIRS; and (6) generating a representation of the probability, the representation being displayed to a user to allow the user to assess the likelihood of a biological subject having HVaSIRS.

Similarly apparatus can be provided for determining the likelihood of a subject having HVaSIRS, the apparatus including: (A) a sampling device that obtains a sample taken from a subject, the sample including polynucleotide expression products; (B) a measuring device that quantifies polynucleotide expression products within the sample to determine three biomarker values, the three biomarker values being selected from the group consisting of: (a) a first pair of biomarker values indicative of a concentration of polynucleotide expression products of a Group A HVaSIRS biomarker gene (e.g., CCL5) and a Group B HVaSIRS biomarker gene (e.g., FLNB); and (b) a second pair of biomarker values indicative of a concentration of polynucleotide expression products of a Group C HVaSIRS biomarker gene (e.g., PPP2R2B) gene and a Group D HVaSIRS biomarker gene (e.g., GNG7); and (c) a third pair of biomarker values indicative of a concentration of polynucleotide expression products of a Group E HVaSIRS biomarker gene (e.g., CASZ1) gene and a Group F HVaSIRS biomarker gene (e.g., VASH1); (C) at least one processing device that: (i) receives an indication of the pair of biomarker values from the measuring device; (ii) determines an indicator using a ratio of the concentration of the first, second and third polynucleotide expression products using the biomarker values; (iii) compares the indicator to at least one indicator reference; (iv) determines a likelihood of the subject having or not having HVaSIRS condition using the results of the comparison; and (v) generates a representation of the indicator and the likelihood for display to a user.

Also disclosed in the context of the present invention are methods for differentiating between HVaSIRS and another SIRS other than HVaSIRS in a subject, including SIRS generated by other non-herpesvirus viral infections (nHVVaSIRS). These methods suitably comprise: (a) obtaining a sample taken from a subject showing a clinical sign of SIRS, the sample including polynucleotide expression products; (b) in a measuring device: (i) amplifying at least some polynucleotide expression products in the sample; (ii) determining an amplification amount representing a degree of amplification required to obtain a defined level of polynucleotide expression products including: amplification amounts for a first pair of polynucleotide expression products of a Group A HVaSIRS biomarker gene (e.g., CCL5) and a Group B HVaSIRS biomarker gene (e.g., FLNB); and amplification amounts for a second pair of polynucleotide expression products of a Group C HVaSIRS biomarker gene (*e.g., PPP2R2B*) gene and a Group D HVaSIRS biomarker gene (*e.g., GNG7*); and amplification amounts for a third pair of polynucleotide expression products of a Group E HVaSIRS biomarker gene (*e.g., CASZ1*) gene and a Group F HVaSIRS biomarker gene (*e.g., VASH1*); (c) in a processing system: (i) retrieving the amplification amounts; (ii) determining an indicator by: determining a first derived biomarker value indicative of a ratio of concentrations of the first pair of polynucleotide expression products by determining a difference between the amplification amounts for the first pair; determining a second derived biomarker value indicative of a ratio of concentrations of the second pair of polynucleotide expression products by determining a difference between the amplification amounts for the second pair; determining a third derived biomarker value indicative of a ratio of concentrations of the third pair of polynucleotide expression products by determining a difference between the amplification amounts for the third pair; (d) determining the indicator by adding the first, second and third derived biomarker values; (e) retrieving previously determined first, second and third indicator references from a database, wherein the first, second and third indicator references are distributions of indicators determined for first and second groups of a reference population, the first and second groups consisting of individuals diagnosed with HVaSIRS and the other SIRS, respectively; (f) comparing the indicator to the first and second indicator references; (g) using the results of the comparison to determine a probability of the subject being classified within the first or second group; (h) generating a representation at least partially indicative of the indicator and the probability; and (i) providing the representation to a user to allow the user to assess the likelihood of a subject having or not having HVaSIRS or the other SIRS.

Additionally, methods can be provided for determining an indicator used in assessing a likelihood of a subject having a presence, absence or degree of HVaSIRS, or in providing a prognosis for a HVaSIRS. These methods suitably include: (1) determining a plurality of biomarker values, each biomarker value being indicative of a value measured or derived for at least one corresponding HVaSIRS biomarker of the subject and being at least partially indicative of a concentration of the HVaSIRS biomarker in a sample taken from the subject; (2) determining the indicator using a combination of the plurality of biomarker values, wherein: at least two biomarkers have a mutual correlation in respect of HVaSIRS that lies within a mutual correlation range, the mutual correlation range being between $\pm 0.9$; and the indicator has a performance value greater than or equal to a performance threshold representing the ability of the indicator to diagnose the presence, absence or degree of HVaSIRS, or to provide a prognosis for the HVaSIRS, the performance threshold being indicative of an explained variance of at least 0.3.

The present invention is also illustrated by the following non-limiting examples.

## EXAMPLES

### EXAMPLE 1

### GROUPS A, B, C, D, E AND F BIOMARKERS. BIOMARKERS ARE GROUPED BASED ON THEIR CORRELATION TO CCL5, FLNB, PPP2R2B, GNG7, CASZ1 AND VASH1

Three pairs of derived biomarkers (CCL5 / FLNB; PPP2R2B / GNG7; CASZ1 / VASH1) were discovered that provided the highest AUC across all of the herpesvirus datasets studied and minimal AUC across all of the non-herpesvirus datasets studied. Biomarkers as ratios that provided mean AUC >0.85 (see Table 5 for full list of derived biomarkers) with the greatest mean difference to non-herpesvirus datasets were then allocated to one of six Groups, as individual biomarkers, based on their correlation to either CCL5 (Group A), FLNB (Group B), PPP2R2B (Group C), GNG7 (Group D), CASZ1 (Group E) or VASH1 (Group F), as presented in Table 10.

### EXAMPLE 2

### HVASI RS BIOMARKER DERIVATION (DERIVED BIOMARKERS)

An illustrative process for the identification of HVaSIRS biomarkers for use in diagnostic algorithms will now be described.

Gene expression data (derived from clinical trials performed by the inventors or from Gene Expression Omnibus) were analysed using a variety of statistical approaches to identify individual and derived biomarkers (ratios) but largely follows the method described in WO 2015/117204. Individual and derived markers were graded based on performance (AUC).

Datasets used for discovery were derived from GEO (which are all MIAME-compliant) with the following restrictions; peripheral blood samples were used, appropriate controls were used, an appropriate number of samples were used to provide significance following False-Discovery Rate (FDR) adjustment, all data passed standard quality control metrics, principle component analysis did not reveal any artifacts or potential biases. A clinical trial (MARS) was also conducted. In this trial, peripheral blood samples were obtained from 624 patients retrospectively diagnosed with either SIRS or sepsis. Some patients, but not all, suspected of having a viral infection, were also tested for the presence of specific viral nucleic acid using PCR in body fluid samples, or the presence of specific anti-viral antibodies in plasma. The datasets were divided into two groups - "discovery" and "validation" listed in Table 1 and Table 3.

The main dataset used for signature "discovery" was GSE40366 (see Table 1 for a description). A subset of subjects in this dataset were used including a nonagenarians' cohort where patients were split into binary categories based on their CMV titer. The "Controls" group contained those patients with a CVM-titer of zero and "Cases" were those patients with a CMV-titer >=20,000. This dataset was the core dataset. Biomarkers discovered in this dataset were then validated using additional data.

All datasets were initially log2 transformed. Prior to analysis each dataset was filtered to include only the top 7145 genes as measured by the mean gene expression level across all samples in the dataset (max value of 8 in log space). This ensured that only those genes with relatively strong expression were analysed and that a limited number of candidates were taken forward to the next compute-time intensive step. ROC and AUC were then calculated across all biomarkers using the difference in the log 2 of the expression values.

Derived biomarkers were calculated for the core dataset (GSE40366) by subtracting the $log_2^{Numerator}$ from the $log_2^{Denominator}$. The ratio direction was set to "<" which means the denominator should be high in "Cases" and low in "Controls with an AUC > 0.5 and low in "Cases" and high in "Controls" with an AUC <0.5. Ratios with an AUC of >=80 (N=1,156,726) in GSE40366 were filtered through to the next phase of analysis. This represented 4.6% of the global ratio-space for this dataset. Any ratios below this threshold were discarded. Further, and as part of "subtracting" away non-herpes and non-viral inflammation biomarkers, various datasets were merged (see below). As part of this merging process only those biomarkers common across all datasets were able to be used. As such, the total number of ratios assessed was 104,790 rather than the total number available (1,156,726).

To ensure that the discovered derived biomarkers from the core dataset were specific to herpesvirus inflammation (were not indicative of inflammation associated with other viral infections, bacterial infection, autoimmune disease, and other non-herpesvirus systemic inflammatory conditions) a number of additional datasets (n=8, listed in Table 2) were used to identify derived biomarkers of generalised, non-herpes and non-viral inflammation. These datasets were used to "subtract" away non-herpes and non-viral biomarkers. These datasets were subject to the same restrictions as the "discovery" and "validation" datasets including; peripheral blood samples were used, appropriate controls were used, an appropriate number of samples were used to provide significance following False-Discovery Rate (FDR) adjustment, all data passed standard quality control metrics, principle component analysis did not reveal any artifacts or potential biases.

All the datasets were merged (discovery (Table 1), non-herpesvirus / non-viral inflammation (Table 2), validation (Table 3)). "Discovery" and "validation" datasets were termed "positive validation" and non-herpesvirus / non-viral inflammation datasets were termed "negative validation". Each individual dataset was normalized by mean centering to zero and forcing gene variance to one as follows: The mean of a gene in a dataset was calculated in three steps: (a) calculate the mean of the cases, (b) calculate the mean of the controls, and (c) calculate the mean of those two values. Once the mean was calculated, the expression values for that gene in each sample were adjusted by subtracting the mean value. The expression matrix was then standardized to unit variance by dividing by the genes variance. All datasets were combined into a single expression matrix after normalizing each dataset individually. The final dimensions of the merged dataset were 2085 genes $\times$ 1117 samples.

Biomarker ratios were computed for every combination in the positive validation set and then a threshold of AUC > 0.85 (vs. negative validation set) was applied to the results to filter higher confidence ratios through to a greedy search algorithm. This resulted in 452 ratios containing 174 unique genes. A lower cut-off filter of an AUC of 0.85 was chosen for the following three reasons: 1) differentiating latent and active herpesvirus infections can be difficult and in one report in only 60% of those patients with clinical signs could a herpesvirus be found using PCR (Troendle Atkins, J., Demmler, G. J., Williamson, W. D., McDonald, J. M., Istas, A. S., & Buffone, G. J. (1994). Polymerase chain reaction to detect cytomegalovirus DNA in the cerebrospinal fluid of neonates with congenital infection. The Journal of Infectious Diseases, 169(6), 1334-1337; 2) The utility of CMV viremia, antigenemia, DNAemia and IgM antibody assays in fetal blood for identifying fetuses infected with CMV has poor sensitivity (41.1%-84.8%) (Revello, M. G., Furione, M., Rognoni, V., Arossa, A., & Gerna, G. (2014). Cytomegalovirus DNAemia in pregnant women. Journal of Clinical Virology : The Official Publication of the Pan American Society for Clinical Virology, 61(4), 590-592); 3) fewer than 50% of pregnant women have detectable CMV in their blood as assessed by either PCR or pp65 antigenemia at the time of serological diagnosis (Ross SA, Novak Z, Pati S, Boppana SB. Diagnosis of Cytomegalovirus Infections. Infectious disorders drug targets. 2011;11(5):466-474). Thus, current existing diagnostic procedures and tests for determining active herpesvirus infections do not have good diagnostic performance, and in many instances no pathogen or antibody response is detected in samples taken at the time the patient presents with clinical signs. A herpesvirus signature with an AUC of at least 0.85 will therefore have greater clinical utility than most existing herpesvirus diagnostic assays in determining an active infection, and at the critical time when the patient presents with clinical signs.

EXAMPLE 3

**HVASI RS BIOMARKER DERIVATION (COMBI NATION OF DERIVED BIOMARKERS)**

A greedy search algorithm was then applied. The application of this search algorithm was designed to maximize the difference in AUC between the negative validation and positive validation sets with each addition of a biomarker ratio. The algorithm actively searches the ratio-space for a decision boundary that takes into account the negative dataset and forces its signal down. This is an iterative process that happens every time a new biomarker ratio is added to the signature.

Following the application of the greedy search algorithm the top three biomarker ratio combinations identified were CCL5 / FLNB, PPP2R2B / GNG7 and CASZ1 / VASH1. The combination of these three biomarker ratios gave an AUC of 0.947 for separating the positive validation data from the negative validation data which was considered to be the minimal set of derived biomarkers with optimal commercial utility. Optimal commercial utility in this instance means consideration of the following non-limiting factors; diagnostic performance, clinical utility, diagnostic noise (introduced by using too many derived biomarkers), transferability to available molecular chemistries (e.g. PCR, microarray, DNA sequencing), transferability to available point-of-care platforms (e.g. Biocartis Idylla, Cepheid GeneXpert, Becton Dickinson BD Max, Curetis Unyvero, Oxford Nanopore Technologies MinION), cost of assay manufacture (the more reagents and biomarkers the larger the cost), ability to multiplex biomarkers, availability of suitable reporter dyes, complexity of results interpretation.

EXAMPLE 4

**HVASI RS BIOMARKER PERFORMANCE (DERIVED BIOMARKERS AND COMBINED DERIVED BIOMARKERS)**

As discussed, following the final filtering step based on a mean AUC of 0.85 in positive validation datasets, 452 derived biomarkers remained. The performance of each of these 452 derived biomarkers, based on the decreasing mean difference in AUC between positive and negative validation datasets, is shown in Table 5.

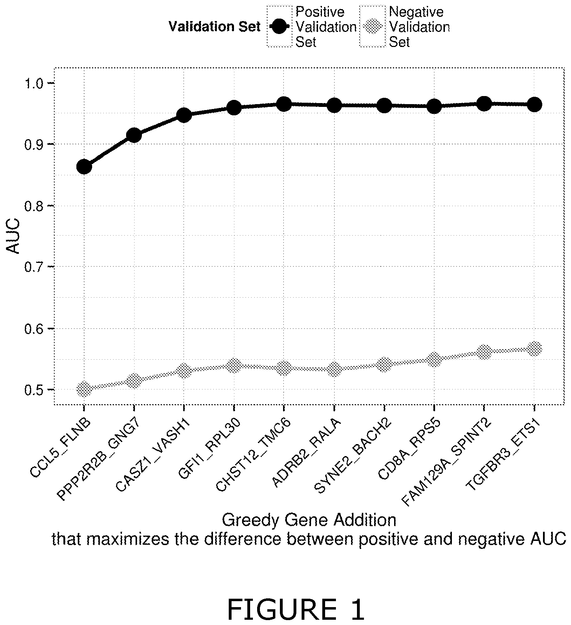

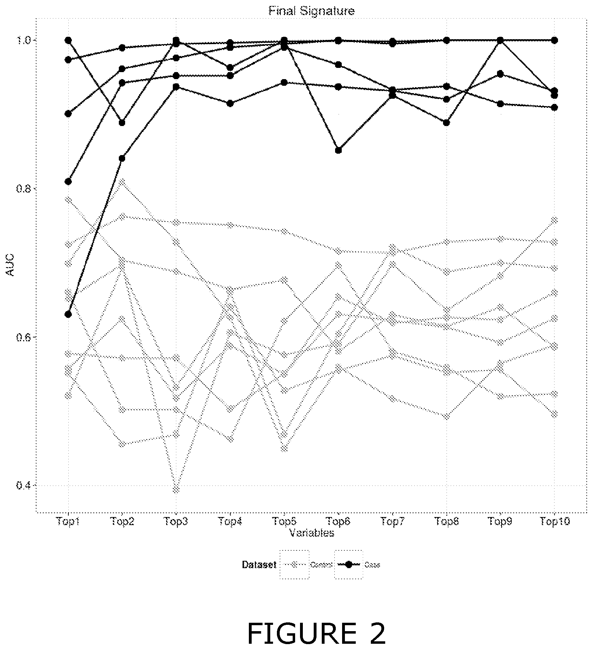

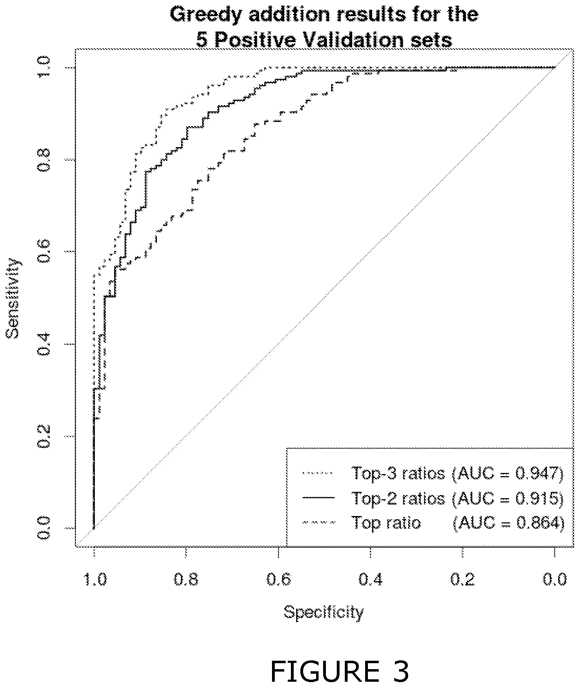

Following normalization of all datasets and a greedy search the best performing individual derived biomarker was CCL5 / FLNB with an AUC of 0.864. Note: the best performing derived biomarker for the combined, normalized dataset is different to the best performing derived biomarker listed in Table 5 because of the way in which AUC was calculated (mean of individual datasets versus mean of combined datasets). The best second unique derived biomarker to add to the first derived biomarker was PPP2R2B / GNG7. The AUC obtained across the normalized dataset using these two derived biomarkers was 0.914, an 0.05 improvement over the use of a single derived biomarker (see Figure 1 and Figure 2). The addition of a third derived biomarker (CASZ1 / VASH1) improved the AUC by 0.033 to 0.947. It is possible that a fourth derived biomarker created overfitting and noise (see Figure 2). Thus, it was considered that an advantageous HVaSIRS signature comprises the following three derived biomarkers: CCL5 / FLNB; PPP2R2B / GNG7; CASZ1 / VASH1. Figure 1, Figure 2 and Figure 3 show the effect on the overall AUC of sequentially adding derived biomarkers to CCL5 / FLNB.

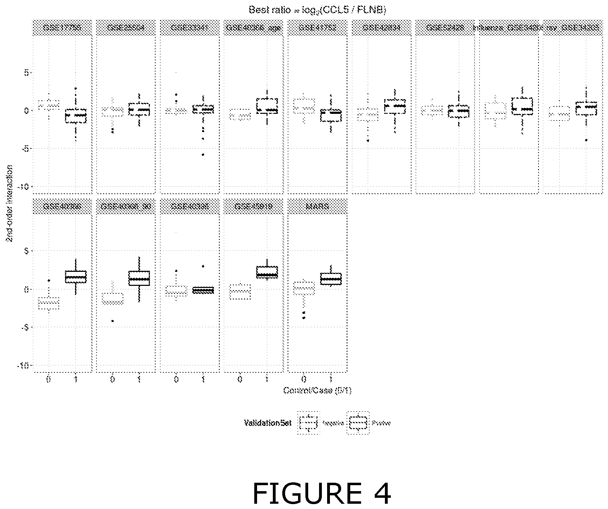

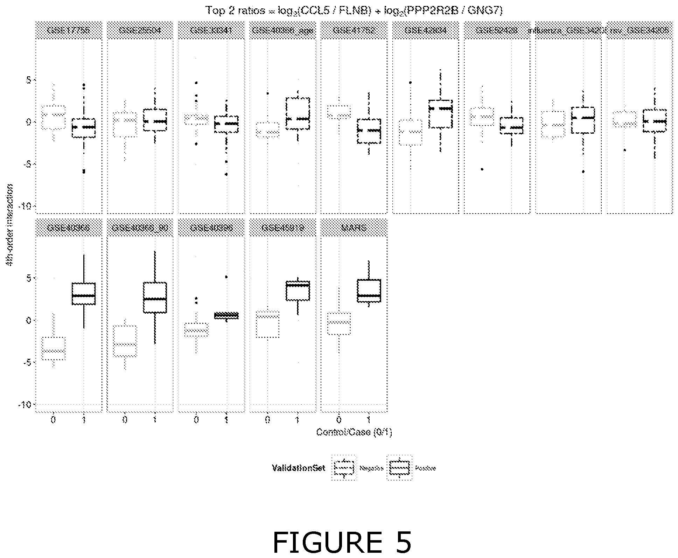

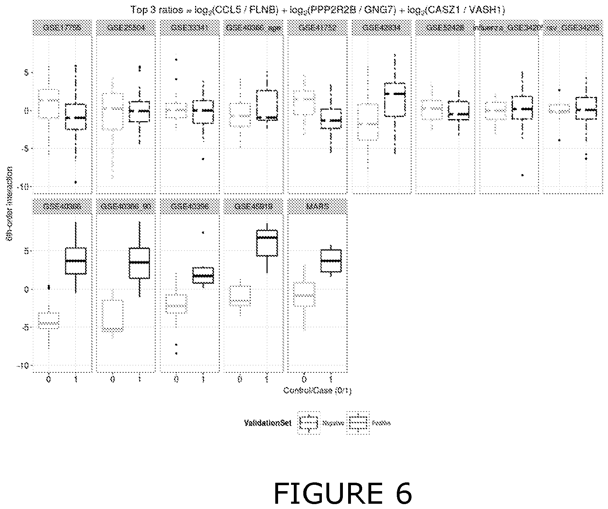

Figure 4, Figure 5 and Figure 6 show box and whisker plots demonstrating the performance of each of the top three derived biomarkers in both the negative validation datasets (9, top row) and positive validation datasets (5, bottom row).

EXAMPLE 5

**HVASIRS BIOMARKER PROFILES (GROUPING)**

The HVaSIRS biomarker profiles can be grouped: individual biomarkers, derived biomarkers, combinations of derived biomarkers.

There are six biomarkers in the best performing three derived biomarker signature: CCL5 / FLNB; PPP2R2B / GNG7; CASZ1 / VASH1, 452 derived biomarkers with an AUC > 0.85 and 174 unique biomarkers. For each unique biomarker, a correlation coefficient was calculated. Table 10 lists 174 unique biomarkers and their correlation to each of the six biomarkers in the top performing three-derived biomarker signature. Each set of biomarkers make up Groups A, B, C, D, E and F respectively.

The best combination of derived biomarkers was determined to be: CCL5 / FLNB; PPP2R2B / GNG7; CASZ1 / VASH1 (Group G).

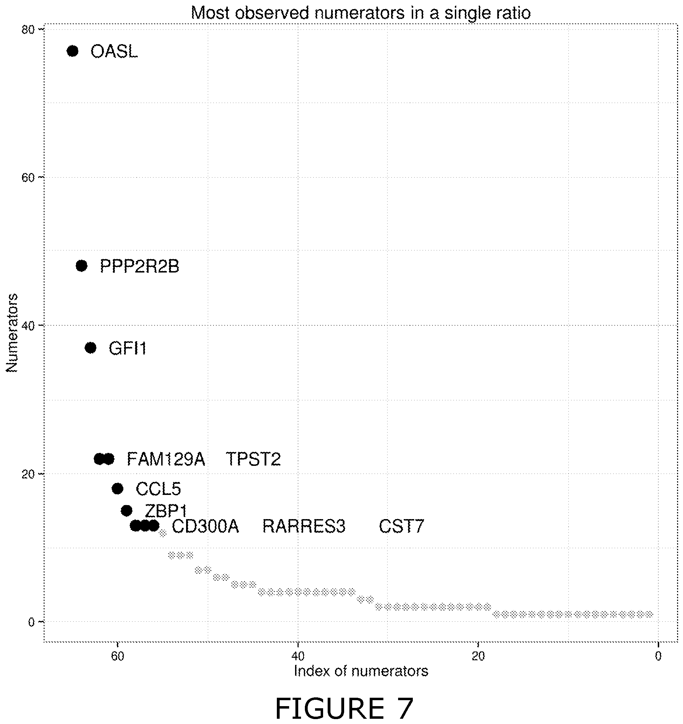

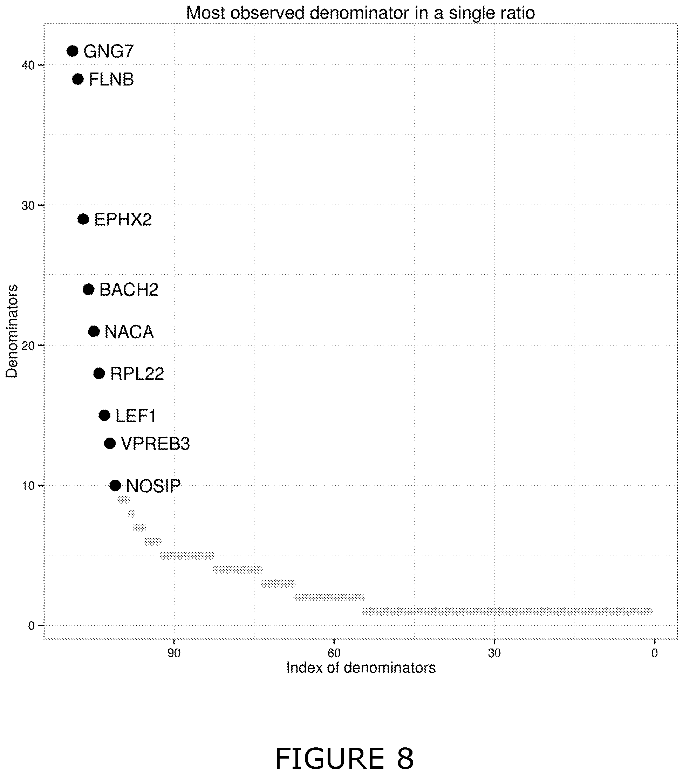

Plots of common numerators and denominators in the total 452 derived biomarkers are shown in Figure 7 and Figure 8.

EXAMPLE 6

**ANALYSIS OF DERIVED BIOMARKERS**

Performance (AUC) of the 452 derived biomarkers across all datasets (as individual datasets rather than a single combined dataset) is shown in Table 5 and in Figure 4, Figure 5 and Figure 6. Some derived biomarkers work better in some datasets than others. The combination of CCL5 / FLNB; PPP2R2B / GNG7; CASZ1 / VASH1 ensures strong diagnostic performance across all positive validation datasets with minimal diagnostic performance in all negative validation datasets.

Some individual biomarkers are part of high performing derived biomarkers more frequently than others. Figure 7 and Figure 8 show plots of biomarkers that occur frequently as numerators or denominators respectively. OASL and PPP2R2B are the most frequent numerators, and GNG7 and FLNB are the most frequent denominators.

EXAMPLE 7

**EXAMPLE APPLICATIONS OF HVASIRS BIOMARKER PROFILES**

Use of the above described biomarkers and resulting HVaSIRS biomarker profiles in patient populations and benefits in respect of differentiating various conditions, will now be described.

An assay capable of differentiating patients with a herpesvirus infection can be used in multiple patient populations, and in conjunction with presenting clinical signs, including those patients located in: Intensive Care Units (medical and surgical ICU), medical wards, Emergency Departments (ED), medical clinics. Such an assay can also be used as part of efforts to ensure judicious use of antibiotic and anti-viral compounds, detection of reactivation of latent herpesviruses, determination of the severity of a HVaSIRS, and determination of the etiology of systemic inflammation when due to an active herpesvirus infection.

*Differentiating an immune response to kev herpesvirus pathogens in patients with non-specific clinical signs*

Many patients present to medical facilities with non-specific clinical signs. Table 6 lists some key human viral pathogens (and virus type) based on the non-specific clinical signs of fever, rash and aches. Those herpesvirus types able to be detected through specific host response biomarkers outlined herein are underlined. The biomarkers described herein can therefore be used to determine the presence and extent of a HVaSIRS and enable clinicians to choose appropriate downstream diagnostic tests, treatments and management regimens.

*Detecting an immune response to kev herpesvirus pathogens early in the course of infection, and when patients present, and when it is difficult to detect herpesvirus antigen*

There are a limited number of human viruses that cause viremia and of those that do a viremia is usually only present in blood for a short period as part of the pathogenesis, making direct detection of the pathogen difficult using blood as a sample. Further, it takes 10-14 days following a primary infection for specific immunoglobulin G antibodies to appear in blood. Active infection with a herpesvirus (a primary infection or reactivation of a latent infection) causes a detectable systemic immune response (HVaSIRS) prior to, and during, the development of peak clinical signs. As such, HVaSIRS biomarkers are useful for early diagnosis, diagnosis, differentiation from other viral causes of systemic inflammation and monitoring in the key periods of viral incubation, when patients present with clinical signs and when virus antigen may or may not be detectable. The specific HVaSIRS described herein will enable clinicians to diagnose an active herpesvirus infection early in the course of infection and when clinical signs are present so that they can make appropriate therapy and management decisions. Table 7 lists common human viruses that are known to cause SIRS and a viremia along with a supporting scientific reference.

*Detecting an immune response to kev herpesvirus pathogens for which there are tailored anti-viral therapies*

Those viruses that cause a viremia and can be treated with an anti-viral agent are listed in Table 8. It is important that viruses in Table 8 be detected, differentiated and identified because 1) they can be treated with anti-viral medication, and 2) most other viral infections cause transient clinical signs and are not life-threatening. In such viral infections it is also important to know if there is a co-infection with bacteria so that antibiotics can be prescribed. The biomarkers described herein can determine the extent of systemic inflammation due to a herpesvirus infection and, as such, judgment can be made as to whether antibiotic prescription is appropriate. Further, once it has been determined that systemic inflammation is specifically due to a herpesvirus an appropriate choice of anti-viral therapy can be made. Anti-herpesvirus therapies are more effective early in the course of disease. The biomarkers outlined in this patent also allow for early and specific diagnosis so that anti- herpesvirus therapy can be used efficaciously.

### *Detecting an immune response to herpesvirus pathogens that can cause respiratory disease*

A list of common and less common viral pathogens that cause community acquired pneumonia in adults and children can be found in Table 9 (based on Pavia AT (2013) What is the Role of Respiratory Viruses in Community-Acquired Pneumonia? Infectious Disease Clinics of North America 27: 157-175). Herpesviruses can cause respiratory clinical signs, especially in immunocompromised patients, and in some instances can lead to complications, including exacerbation of existing pathologies, or concurrent or subsequent viral or bacterial infections. Patients presenting to medical facilities with respiratory herpesvirus infection(s) need to be differentiated from those with other viral infections, and from those with bacterial infections, so that appropriate anti-herpesvirus therapy or antibiotics can be administered. The biomarkers described in this patent can determine the presence and extent of systemic inflammation due to a respiratory herpesvirus infection and, as such, judgment can be made regarding appropriate management procedures, specific anti-viral treatments and/or antibiotic treatments.

### *Differentiating patients with a herpesvirus condition in ICU*

It has been shown that greater than 50% of patients in medical ICUs have inSIRS and greater than 80% in surgical ICUs have inSIRS (Brun-Buisson C (2000) The epidemiology of the systemic inflammatory response. Intensive Care Med 26 Suppl 1: S64-S74). From a clinician's perspective these patients present with non-specific clinical signs and the source and type of infection, if there is one, must be determined quickly so that appropriate therapies can be administered. Patients with inSIRS have a higher likelihood of being infected with bacteria or fungi (compared to patients without inSIRS), and have a much higher 28-day mortality (Constet P, Storgaard M, Lassen AT (2009) The Systemic Inflammatory Response Syndrome (SIRS) in acutely hospitalised medical patients: a cohort study. Scand J Trauma Resusc Emerg Med 17: 67. doi:10.1186/1757-7241-17-67). Further, patients with prolonged systemic inflammation due to bacterial infection have a higher frequency of viral infections, possibly due to reactivation of latent viruses as a result of immunosuppression (Walton, A. H., Muenzer, J. T., Rasche, D., Boomer, J. S., & Sato, B. (2014). Reactivation of multiple viruses in patients with sepsis. PLoS ONE). The high prevalence of inSIRS in ICU, the high risk of infection and death in inSIRS patients, the reactivation of viruses in ICU patients with bacterial systemic inflammation, and the benefits of early intervention in patients with bacterial systemic inflammation (Rivers EP (2010) Point: Adherence to Early Goal-Directed Therapy: Does It Really Matter? Yes. After a Decade, the Scientific Proof Speaks for Itself. Chest 138: 476-480) creates a need for triaging patients with clinical signs of inSIRS to determine whether they have a viral or bacterial infection, or both. Monitoring intensive care patients on a regular basis with biomarkers of the present invention will allow medical practitioners to determine the presence, or absence, of a herpesvirus infection so that appropriate anti-herpes-virus therapies could be administered.

In pediatric ICUs the incidence of viral infections is reportedly low (1%), consisting mostly of enterovirus, parechovirus and respiratory syncytial virus infections (Verboon-Maciolek, M. A., Krediet, T. G., Gerards, L. J., Fleer, A., & van Loon, T. M. (2005). Clinical and epidemiologic characteristics of viral infections in a neonatal intensive care unit during a 12-year period. The Pediatric Infectious Disease Journal, 24(10), 901-904). However, one of the most common congenital viral infections in humans is CMV, affecting between 20,000 and 40,000 infants per year in the USA (Ross SA, Novak Z, Pati S, Boppana SB. Diagnosis of Cytomegalovirus Infections. Infectious disorders drug targets. 2011;11(5):466-474). Further, because the mortality rate of virus-infected patients is high, and they present with similar clinical signs to those with bacterial or fungal infections, it is important to rule out the possibility of a viral infection in pediatric patients so that other appropriate therapies can be administered, or to rule in a herpesvirus infection so that appropriate therapies can be administered and management procedures performed.

Determining which patients have herpesvirus infections in the ICU will allow for early intervention, appropriate choice of therapies, when to start and stop therapies, whether a patient needs to be isolated, when to start and stop appropriate patient management procedures, and in determining how a patient is responding to therapy. Information provided by these biomarkers will therefore allow medical intensivists to tailor and modify therapies and management procedures to ensure that patients with active herpesvirus infections survive and spend less time in intensive care. Less time in intensive care leads to considerable savings in medical expenses including through less occupancy time and through appropriate use and timing of medications.

### *Differentiating patients with a viral condition in hospital wards*

In a study in a US hospital of over 4000 inpatients over an 11-week period at least one episode of fever occurred in 1,194 patients (29%) (McGowan JEJ, Rose RC, Jacobs NF, Schaberg DR, Haley RW (1987) Fever in hospitalized patients. With special reference to the medical service. Am J Med 82: 580-586). The rate of fever was highest on medical and surgical services and the authors found that both infectious and non-infectious processes played important roles in the cause. However, determining the cause of fever was complicated by the fact that over 390 different factors were identified. In this study, a review of 341 episodes of fever in 302 patients on the medical service identified a single potential cause in 56%, multiple factors were present in 26%, and no potential causes were found in 18%. Of all factors identified, 44% were community-acquired infections, 9% were nosocomial infections, 20% possibly involved infection, and 26% were non-infectious processes. Thus, fever is common in hospital surgical and medical wards, there are many causes including infectious and non-infectious, diagnosis is difficult and in many instances a cause is not found. The biomarkers outlined in this patent can differentiate herpesvirus infections from other causes of SIRS which will assist medical practitioners in determining the cause of fever, ensuring that resources are not wasted on unnecessary diagnostic procedures and that patients are managed and treated appropriately.

### Differentiating patients with a herpesvirus condition in emergency departments

In 2010, approximately 130 million people presented to emergency departments in the USA and the third most common primary reason for the visit was fever (5.6 million people had a fever (>38°C) and for 5 million people it was the primary reason for the visit) (Niska R, Bhuiya F, Xu J (2010) National hospital ambulatory medical care survey: 2007 emergency department summary. Natl Health Stat Report 26: 1-31). Of those patients with a fever, 664,000 had a fever of unknown origin - that is, the cause of the fever was not obvious at presentation. As part of diagnosing the reason for the emergency department visit 48,614,000 complete blood counts (CBC) were performed and 5.3 million blood cultures were taken. In 3.65 million patients presenting the primary diagnosis was "infectious" and in approximately 25% of cases (32.4 million) antibiotics were administered. 13.5% of all people presenting to emergency were admitted to hospital. Clinicians in emergency need to determine the answer to a number of questions quickly, including: what is the reason for the visit, is the reason for the visit an infection, does the patient need to be admitted? The diagnosis, treatment and management of patients with a fever, inSIRS, HVaSIRS or bacterial systemic inflammation are different. By way of example, a patient with a fever without other inSIRS clinical signs and no obvious source of viral, or microbial infection may be sent home, or provided with other non-hospital services, without further hospital treatment. However, a patient with a fever may have early BaSIRS, and not admitting such a patient and aggressively treating with antibiotics may put their life at risk. Such a patient may also have HVaSIRS and quickly deteriorate, or progress to BaSIRS without appropriate hospital care and/or the use of anti-viral agents. The difference in the number of patients presenting to emergency that are ultimately diagnosed with an "infection" (3.65 million) and the number treated with antibiotics (32.4 million) suggests the following; 1) diagnostic tools that determine the presence of an infection are not available, or are not being used, or are not accurate enough, or do not provide strong enough negative predictive value, or are not providing accurate information that can be acted on within a reasonable timeframe 2) when it comes to suspected infection, and because of the acute nature of infections, clinicians err on the side of caution by administering antibiotics. Further, in a study performed in the Netherlands on patients presenting to emergency with fever, 36.6% of patients admitted to hospital had a suspected bacterial infection (that is, it was not confirmed) (Limper M, Eeftinck Schattenkerk D, de Kruif MD, van Wissen M, Brandjes DPM, et al. (2011) One-year epidemiology of fever at the Emergency Department. Neth J Med 69: 124-128). This suggests that a large proportion of patients presenting to emergency are admitted to hospital without a diagnosis. The biomarkers outlined in this patent can identify those patients with an active herpesvirus infection from those without an active herpesvirus infection, assisting medical practitioners in triaging patients with fever or SIRS. Such effective triage tools make best use of scarce hospital resources, including staff, equipment and therapies. Accurate triage decision-making also ensures that patients requiring hospital treatment are given it, and those that don't are provided with other appropriate services.

In a study performed in Argentina in patients presenting to emergency with influenza-like symptoms, only 37% of samples taken and analysed for the presence of viruses (using immunofluorescence, RT-PCR and virus culture) were positive (Santamaria, C., Uruena, A., Videla, C., Suarez, A., Ganduglia, C., Carballal, G., et al. (2008). Epidemiological study of influenza virus infections in young adult outpatients from Buenos Aires, Argentina. Influenza and Other Respiratory Viruses, 2(4), 131-134). In a study based in Boston , USA, acute respiratory infections were a common reason children presented to emergency departments in Winter (Bourgeois, F. T., Valim, C., Wei, J. C., McAdam, A. J., & Mandl, K. D. (2006). Influenza and other respiratory virus-related emergency department visits among young children. Pediatrics, 118(1), e1-8). Using a respiratory classifier (based on clinical signs) these authors found that in children less than, or equal to, 7 years of age an acute respiratory infection was suspected in 39.8% of all emergency department visits (less at a whole city or state level). In this latter study only 55.5% of these patients had a virus isolated. Thus, a large percentage of patients with influenza-like symptoms presenting to emergency are likely not being diagnosed as having a viral infection using laboratory-based tests. The biomarkers outlined in this patent can identify those patients with an active herpesvirus infection from those without an active herpesvirus infection, assisting medical practitioners in making an accurate diagnosis of an active herpesvirus infection in patients with influenza-like symptoms. Such patients can then be appropriately treated with anti-viral therapies. Accurate diagnosis of an active viral infection also assists in ensuring that only those patients that need either anti-viral treatment or antibiotics receive them which may lead to fewer side effects and fewer days on antibiotics (Adcock, P. M., Stout, G. G., Hauck, M. A., & Marshall, G. S. (1997). Effect of rapid viral diagnosis on the management of children hospitalized with lower respiratory tract infection. The Pediatric Infectious Disease Journal, 16(9), 842-846).

Patients presenting to medical clinics as outpatients often have clinical signs of SIRS including abnormal temperature, heart rate or respiratory rate and there are many causes of these clinical signs. Such patients need to be assessed thoroughly to determine the cause of the clinical signs because in some instances it could be a medical emergency. By way of example, a patient with colic might present with clinical signs of increased heart rate. Differential diagnoses could be (but not limited to) appendicitis, urolithiasis, cholecystitis, pancreatitis, enterocolitis. In each of these conditions it would be important to determine if there was a non-infectious systemic inflammatory response (inSIRS) or whether an infection was contributing to the systemic response. The treatment and management of patients with non-infectious systemic inflammation and/or SIRS due to infectious causes are different. The biomarkers detailed in this patent can differentiate an active herpesvirus infection from other causes of SIRS so that a medical practitioner can either rule in or rule out an active herpesvirus etiology. As a result, medical practitioners can more easily determine the next medical actions and procedure(s) to perform to satisfactorily resolve the patient issue.

### Diagnosing maternal and prenatal cytomegalovirus infection

Congenital CMV infection (transfer from mother to child) is the most common congenital viral condition in humans affecting between 20,000 and 40,000 children per year in the USA (Ross SA, Novak Z, Pati S, Boppana SB. Diagnosis of Cytomegalovirus Infections. Infectious disorders drug targets. 2011;11(5):466-474). It is therefore important to determine whether a mother has suffered, or is suffering from a primary and active CMV infection during pregnancy. Current tests involve two consecutive maternal blood samples and the measurement of both IgM and IgG avidity. The presence of both IgM and IgG with low avidity indicates a more recent and primary CMV infection. Such tests have variable sensitivity with respect to determining whether CMV was transmitted to the fetus and depend upon when the blood samples were taken during gestation. The biomarkers outlined in the current application have utility in determining an active and primary CMV infection in mothers using a single blood sample and at the time of presenting clinical signs.

Perinatal testing for CMV currently involves the use of serological testing (IgM and IgG) and proof of absence of CMV shedding in the first two weeks of life using PCR. Such testing requires multiple samples over time and relies on assay sensitivity. The biomarkers outlined in the current application have utility in determining an active and primary CMV infection (or lack thereof) in neonates using a single blood sample.

### Detection of active herpesvirus infection in the immunocompromised

Primary infection with viruses and reactivation of latent viruses is common in patients that are immunocompromised, including those with prolonged sepsis and those on immunosuppressive therapy, and correct and timely diagnosis of a herpesvirus infection is important in such patients (Walton AH, Muenzer JT, Rasche D, Boomer JS, Sato B, et al. (2014) Reactivation of multiple viruses in patients with sepsis. PLoS ONE 9: e98819; Andersen, H. K., and E. S. Spencer. 1969. Cytomegalovirus infection among renal allograft recipients. Acta Med. Scand. 186:7-19; Bustamante CI, Wade JC (1991) Herpes simplex virus infection in the immunocompromised cancer patient. J Clin Oncol 9: 1903-1915; Ljungman P, Griffiths P, Paya C. Definitions of cytomegalovirus infection and disease in transplant recipients. Clin. Infect. Dis. 2002; 34(8):1094-1097).

For patients with sepsis (Walton et al., 2014), cytomegalovirus (CMV), Epstein-Barr (EBV), herpes-simplex (HSV), human herpesvirus-6 (HHV-6), and anellovirus TTV were all detectable in blood at higher rates compared to control patients, and those patients with detectable CMV had higher 90-day mortality. However, because these viruses have only been detected in sepsis patients it is not known whether reactivated latent viruses contribute to pathology, morbidity and mortality. Further, such viruses have a limited viremic period making detection of viral antigen in blood unreliable as a method of diagnosis.

Solid organ transplant recipients are commonly affected by herpesvirus infections, either as a primary infection, infection with a different herpesvirus strain, or reactivation of latent virus. Current serological tests are useful in determining that both the donor and recipient are negative for a herpesvirus infection (assuming that a herpesvirus infection is not incubating at the time the sample was taken). However, current serological tests are not useful in determining whether there is an active herpesvirus infection in the donor, and detection of virus antigen (PCR, virus antigen detection) in circulating blood or donor tissue is unreliable.

Patients with hemopoietic stem cell transplantation are also commonly affected by herpesvirus infections. Those patients most at risk are seronegative receiving a transplant from a positive donor. Currently such patients are monitored weekly for the presence of herpesvirus antigens but such tests rely on virus shedding, which is often late in the course of infection or transient. Donors are screened using serological tests or antigen detection tests but both methods can miss detecting an active infection.

HIV/AIDS patients are also commonly affected by herpesvirus infections, especially when they have low CD4-T cell counts. Again, such patients are currently tested for the presence of herpesvirus antigens but such tests rely on virus shedding, which is often late in the course of infection. Testing such patients for an active herpesvirus infection, which precedes active virus shedding into blood and tissues, would be a reliable method of determining whether a patient was suffering from a herpesvirus infection.

Thus, the biomarkers detailed in this patent can determine the presence of an active herpesvirus infection and could therefore be useful in monitoring immunocompromised patients, and screening tissue donors to; 1) detect the presence of an active herpesvirus infection early in the course of disease, and/or 2) determine the contribution to existing pathology and clinical signs of an active herpesvirus infection, and/or 3) to ensure that early and appropriate therapies can be administered.

### Determining the Extent of Systemic Inflammation in Patients with a HVaSIRS

Patients presenting to medical facilities often have any one of the four clinical signs of SIRS. However, many different conditions can present with one of the four clinical signs of SIRS and such patients need to be assessed to determine if they have inSIRS, and if so the extent of inSIRS, or HVaSIRS, and if so the extent of HVaSIRS, and to exclude other differential diagnoses.

By way of example, a patient presenting with abdominal colic could have any number of conditions, including (but not limited to): renal colic, appendicitis, peritonitis, cystitis, varicella zoster, food poisoning, hepatic colic, viral hepatitis, physical blockage. In this instance it would be important to determine if there was a systemic inflammatory response (inSIRS) or whether an infection (viral, bacterial, fungal or parasitic) was contributing to the condition. The treatment and management of patients with and without systemic inflammation and/or viral, bacterial, fungal or parasitic infections are different. Because the biomarkers described in this patent can determine the degree of systemic inflammatory involvement, the use of them will allow medical practitioners to determine the next medical procedure(s) to perform to satisfactorily resolve the patient issue. Patients with a physical blockage are unlikely to have a systemic inflammatory response and patients with renal or hepatic colic may have a large systemic inflammatory response but not likely due to an infection. The extent, or not, of HVaSIRS, as indicated by biomarkers presented in this patent, allows clinicians to definitively diagnosis a herpesvirus infection, or to rule out a herpesvirus cause, and provides clinicians with information to assist in treatment and patient management decision making. For example, a patient with abdominal colic and a fever and a strong marker response indicating HVaSIRS due to herpes zoster is likely to be hospitalised and to ensure that appropriate anti-herpesvirus therapy is administered.

### Antibiotic stewardship

In patients suspected of having a systemic infection (viral, bacterial, fungal, parasitic) a clinical diagnosis and treatment regimen is provided by the physician(s) at the time the patient presents and often in the absence of any results from diagnostic tests. This is done in the interests of rapid treatment and positive patient outcomes. However, such an approach leads to over-prescribing of antibiotics irrespective of whether the patient has a microbial infection or not. Clinician diagnosis of BaSIRS is reasonably reliable (0.88) in children but only with respect to differentiating between patients ultimately shown to be blood culture positive and those that were judged to be unlikely to have an infection at the time antibiotics were administered (Fischer, J. E., Harbarth, S., Agthe, A. G., Benn, A., Ringer, S. A., Goldmann, D. A., & Fanconi, S. (2004). Quantifying uncertainty: physicians' estimates of infection in critically ill neonates and children. Clinical Infectious Diseases : An Official Publication of the Infectious Diseases Society of America, 38(10), 1383-1390). In Fischer *et al.,* (2004), 54% of critically ill children were put on antibiotics during their hospital stay, of which only 14% and 16% had proven systemic bacterial infection or localized infection respectively. In this study, 53% of antibiotic treatment courses for critically ill children were for those that had an unlikely infection and 38% were antibiotic treatment courses for critically ill children as a rule-out treatment episode. Clearly, pediatric physicians err on the side of caution with respect to treating critically ill patients by placing all patients suspected of an infection on antibiotics - 38% of all antibiotics used in critically ill children are used on the basis of ruling out BaSIRS, that is, are used as a precaution. Antibiotics are also widely prescribed and overused in adult patients as reported in Braykov *et al.,* 2014 (Braykov, N. P., Morgan, D. J., Schweizer, M. L., Uslan, D. Z., Kelesidis, T., Weisenberg, S. A., et al. (2014). Assessment of empirical antibiotic therapy optimisation in six hospitals: an observational cohort study. The Lancet Infectious Diseases, 14(12), 1220-1227). In this study, across six US hospitals over four days in 2009 and 2010, 60% of all patients admitted received antibiotics. Of those patients prescribed antibiotics 30% were afebrile and had a normal white blood cell count and where therefore prescribed antibiotics as a precaution. As such, an assay that can accurately diagnose a herpesvirus infection in patients presenting with non-pathognomonic clinical signs of infection would be clinically useful and may lead to more appropriate use of antibiotics and anti-herpesvirus therapies.

*Differentiating patients with a viral condition in medical clinics*

Patients presenting to medical clinics as outpatients often have clinical signs of SIRS including abnormal temperature, heart rate or respiratory rate and there are many causes of these clinical signs. Such patients need to be assessed thoroughly to determine the cause of the clinical signs because in some instances it could be a medical emergency. By way of example, a patient with colic might present with clinical signs of increased heart rate. Differential diagnoses could be (but not limited to) appendicitis, urolithiasis, cholecystitis, pancreatitis, enterocolitis. In each of these conditions it would be important to determine if there was a non-infectious systemic inflammatory response (inSIRS) or whether an infection was contributing to the systemic response. The treatment and management of patients with non-infectious systemic inflammation and/or SIRS due to infectious causes are different. The HVaSIRS biomarkers detailed herein can differentiate a HVaSIRS from other causes of SIRS so that a medical practitioner can either rule in or rule out a systemic inflammation of herpesvirus etiology. As a result, medical practitioners can more easily determine the next medical actions and procedure(s) to perform to satisfactorily resolve the patient issue.

*Detection of reactivation of latent viruses*

Reactivation of latent viruses is common in patients that are immunocompromised, including those with prolonged sepsis and those on immunosuppressive therapy (Walton AH, Muenzer JT, Rasche D, Boomer JS, Sato B, et al. (2014) Reactivation of multiple viruses in patients with sepsis. PLoS ONE 9: e98819; Andersen, H. K., and E. S. Spencer. 1969. Cytomegalovirus infection among renal allograft recipients. Acta Med. Scand. 186:7-19; Bustamante CI, Wade JC (1991) Herpes simplex virus infection in the immunocompromised cancer patient. J Clin Oncol 9: 1903-1915). For patients with sepsis (Walton *et al.,* 2014), cytomegalovirus (CMV), Epstein-Barr (EBV), herpes-simplex (HSV), human herpesvirus-6 (HHV-6), and anellovirus TTV were all detectable in blood at higher rates compared to control patients, and those patients with detectable CMV had higher 90-day mortality. However, because these viruses have only been detected in sepsis patients it is not known whether reactivated latent viruses contribute to pathology, morbidity and mortality.

EXAMPLE 8

**FIRST EXAMPLE WORKFLOW FOR DETERMINING HOST RESPONSE**

A first example workflow for measuring host response to HVaSIRS will now be described. The workflow involves a number of steps depending upon availability of automated platforms. The assay uses quantitative, real-time determination of the amount of each host immune cell RNA transcript in the sample based on the detection of fluorescence on a qRT-PCR instrument (e.g. Applied Biosystems 7500 Fast Dx Real-Time PCR Instrument, Applied Biosystems, Foster City, CA, catalogue number 440685; K082562). Transcripts are each reverse-transcribed, amplified, detected, and quantified in a separate reaction well using a probe that is visualized in the FAM channel (by example). Such reactions can be run as single-plexes (one probe for one transcript per tube), multiplexed (multiple probes for multiple transcripts in one tube), one-step (reverse transcription and PCR are performed in the same tube), or two-step (reverse transcription and PCR performed as two separate reactions in two tubes). A score is calculated using interpretive software provided separately to the kit but designed to integrate with RT-PCR machines.
The workflow below describes the use of manual processing and a pre-prepared kit.

Pre-analytical

Blood collection
Total RNA isolation

Analytical

Reverse transcription (generation of cDNA)
qPCR preparation
qPCR
Software, Interpretation of Results and Quality Control
Output.

Kit Contents

Diluent
RT Buffer
RT Enzyme Mix
qPCR Buffer
Primer/Probe Mix
AmpliTaq Gold® (or similar)
High Positive Control
Low Positive Control
Negative Control

Blood Collection

The specimen used is a 2.5 mL sample of blood collected by venipuncture using the PAXgene® collection tubes within the PAXgene® Blood RNA System (Qiagen, kit catalogue # 762164; Becton Dickinson, Collection Tubes catalogue number 762165; K042613). An alternate collection tube is Tempus® (Life Technologies).

Total RNA Isolation

Blood (2.5 mL) collected into a PAXgene RNA tube is processed according to the manufacturer's instructions. Briefly, 2.5mL sample of blood collected by venipuncture using the PAXgene™ collection tubes within the PAXgene™ Blood RNA System (Qiagen, kit catalogue # 762164; Becton Dickinson, Collection Tubes catalogue number 762165; K042613). Total RNA isolation is performed using the procedures specified in the PAXgene™ Blood RNA kit (a component of the PAXgene™ Blood RNA System). The extracted RNA is then tested for purity and yield (for example by running an $A_{260/280}$ ratio using a Nanodrop® (Thermo Scientific)) for which a minimum quality must be (ratio > 1.6). RNA should be adjusted in concentration to allow for a constant input volume to the reverse transcription reaction (below). RNA should be processed immediately or stored in single-use volumes at or below -70°C for later processing.

Reverse Transcription

Determine the appropriate number of reaction equivalents to be prepared (master mix formulation) based on a plate map and the information provided directly below. Each clinical specimen is run in singleton.
Each batch run desirably includes the following specimens:

• High Control, Low Control, Negative Control, and No Template Control (Test Diluent instead of sample) in singleton each
  Program the ABI 7500 Fast Dx Instrument as detailed below.

• Launch the software.

• Click Create New Document

• In the New Document Wizard, select the following options:

  i. Assay: Standard Curve (Absolute Quantitation)

  ii. Container: 96-Well Clear

  iii. Template: Blank Document (or select a laboratory-defined template)

  iv. Run Mode: Standard 7500

  v. Operator: Enter operator's initials

  vi. Plate name: [default]

• Click Finish

- Select the Instrument tab in the upper left

- In the Thermal Cycler Protocol area, Thermal Profile tab, enter the following times:

  i. 25° C for 10 minutes

  ii. 45° C for 45 minutes

  iii. 93° C for 10 minutes

  iv. Hold at 25° C for 60 minutes

In a template-free area, remove the test Diluent and RT-qPCR Test RT Buffer to room temperature to thaw. Leave the RT-qPCR Test RT Enzyme mix in the freezer and/or on a cold block.

In a template-free area, assemble the master mix in the order listed below.

RT Master Mix - Calculation:

|  | Per well | × N |
|---|---|---|
| RT-qPCR Test RT Buffer | 3.5 $\mu$L | 3.5 × N |
| RT-qPCR Test RT Enzyme mix | 1.5 $\mu$L | 1.5 × N |
| Total Volume | 5$\mu$L | 5 × N |

Gently vortex the master mix then pulse spin. Add the appropriate volume (5 $\mu$L) of the RT Master Mix into each well at room temperature.

Remove clinical specimens and control RNAs to thaw. (If the specimens routinely take longer to thaw, this step may be moved upstream in the validated method.)

Vortex the clinical specimens and control RNAs, then pulse spin. Add 10 $\mu$L of control RNA or RT-qPCR Test Diluent to each respective control or negative well.

Add 10 $\mu$L of sample RNA to each respective sample well (150 ng total input for RT; $OD_{260}/OD_{280}$ ratio greater than 1.6). Add 10 $\mu$L of RT-qPCR Test Diluent to the respective NTC well.

Note: The final reaction volume per well is 15 $\mu$L.

|  | Samples |
|---|---|
| RT Master Mix | 5 $\mu$L |
| RNA sample | 10 $\mu$L |
| Total Volume (per well) | 15 $\mu$L |

Mix by gentle pipetting. Avoid forming bubbles in the wells.

Cover wells with a seal.

Spin the plate to remove any bubbles (1 minute at 400 x *g*).

Rapidly transfer to ABI 7500 Fast Dx Instrument pre-programmed as detailed above.

Click Start. Click Save and Continue. Before leaving the instrument, it is recommended to verify that the run started successfully by displaying a time under Estimated Time Remaining.

qPCR master mix may be prepared to coincide roughly with the end of the RT reaction. For example, start about 15 minutes before this time. See below.

When RT is complete (i.e. resting at 25 °C; stop the hold at any time before 60 minutes is complete), spin the plate to collect condensation (1 minute at 400 x *g*).

qPCR Preparation

Determine the appropriate number of reaction equivalents to be prepared (master mix formulation) based on a plate map and the information provided in RT Preparation above.

Program the ABI 7500 Fast Dx with the settings below.

a) Launch the software.

b) Click Create New Document

c) In the New Document Wizard, select the following options:

   i. Assay: Standard Curve (Absolute Quantitation)

   ii. Container: 96-Well Clear

   iii. Template: Blank Document (or select a laboratory-defined template)

   iv. Run Mode: Standard 7500

   v. Operator: Enter operator's initials

   vi. Plate name: Enter desired file name

d) Click Next

e) In the Select Detectors dialog box:

   i. Select the detector for the first biomarker, and then click Add>>.

   ii. Select the detector second biomarker, and then click Add>>, etc.

   iii. Passive Reference: ROX

f) Click Next

g) Assign detectors to appropriate wells according to plate map.

   i. Highlight wells in which the first biomarker assay will be assigned

   ii. Click use for the first biomarker detector

   iii. Repeat the previous two steps for the other biomarkers

   iv. Click Finish

h) Ensure that the Setup and Plate tabs are selected

i) Select the Instrument tab in the upper left

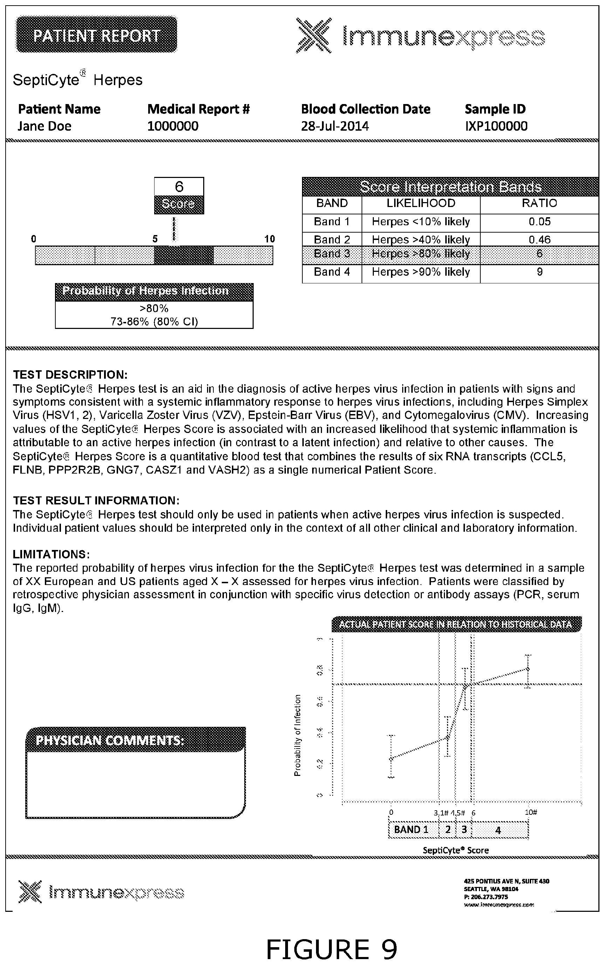

j) In the Thermal Cycler Protocol area, Thermal Profile tab, perform the following actions, with the results shown in Figure 9:

   i. Delete Stage 1 (unless this was completed in a laboratory-defined template).

   ii. Enter sample volume of 25 μL.

   iii. 95 °C 10 minutes

   iv. 40 cycles of 95 °C for 15 seconds, 63 °C for 1 minute

   v. Run Mode: Standard 7500

vi. Collect data using the "stage 2, step 2 (63.0@1:00)" setting

k) Label the wells as below using this process: Right click over the plate map, then select Well Inspector. With the Well Inspector open, select a well or wells. Click back into the Well Inspector and enter the Sample Name. Close the Well Inspector when completed.

i. CONH for High Control

ii. CONL for Low Control

iii. CONN for Negative Control

iv. NTC for No Template Control

v. [Accession ID] for clinical specimens

l) Ensure that detectors and quenchers are selected as listed below.

i. FAM for CCL5 biomarker 1; quencher=none

ii. FAM for FLNB biomarker 2; quencher=none

iii. FAM for PPP2R2B; biomarker 3; quencher=none

iv. FAM for GNG7; biomarker 4; quencher=none

v. FAM for CASZ1; biomarker 5; quencher=none

vi. FAM for VASH1; biomarker 6; quencher=none

vii. Select "ROX" for passive reference

qPCR

In a template-free area, remove the assay qPCR Buffer and assay Primer/Probe Mixes for each target to room temperature to thaw. Leave the assay AmpliTaq Gold in the freezer and/or on a cold block.
Still in a template-free area, prepare qPCR Master Mixes for each target in the listed order at room temperature.

qPCR Master Mixes - Calculation Per Sample

| | Per well | $\times$ N |
|---|---|---|
| qPCR Buffer | 11 $\mu$L | 11 $\times$ N |
| Primer/Probe Mix | 3.4 $\mu$L | 3.4 $\times$ N |
| AmpliTaq Gold® | 0.6 $\mu$L | 0.6 $\times$ N |
| Total Volume | 15 $\mu$L | 15 $\times$ N |

Example forward (F) and reverse (R) primers and probes (P) and their final reaction concentration for measuring four host response transcripts to viral biomarkers are contained in the following table (F, forward; R, reverse; P, probe).

| Reagent | 5'-3' Sequence | Reaction mM |
|---|---|---|
| CCL5-F | GGCTGAACAAGGGCAAGCT | 360 |
| CCL5-R | ACTCCCGAACCCATTTCTTCTC | 360 |
| CCL5-P | GTCACCCGAAAGAACCGC | 50 |
| FLNB-F | GCCCACCACATCGTGGGC | 360 |

(continued)

| Reagent | 5'-3' Sequence | Reaction mM |
|---------|----------------|-------------|
| FLNB-R | GGTCAGTCACTCCACCAGGATGG | 360 |
| FLNB-P | GCCCTGGCTCAGCCAACG | 50 |
| PPP2R2B-F | GTCCGCTGATGACCTGAGG | 360 |
| PPP2R2B-R | CGTGAGCTCCTCCATGTTGG | 360 |
| PPP2R2B-P | GTTTTAATATTGTGGACATTAAGCCAG | 50 |
| GNG7-F | GCAAGCTGTGATTCCTGGG | 360 |
| GNG7-R | CCACCAGCTTCCGGGCCT | 360 |
| GNG7-P | GGGGCCCAGAGCTGATG | 50 |
| CASZ1 | CTCCCAGGACCGCAGTCTA | 360 |
| CASZ1 | ACCTGACTGTGAAGGAGCCC | 360 |
| CASZ1 | ATGAATTTGCCGGGACTGC | 50 |
| VASH1 | CTCCCTCTCCCACCAAGGA | 360 |
| VASH1 | CCCGTTAAGGTCTGGCATG | 360 |
| VASH1 | GCAGGAACAGCCGCAGTG | 50 |

Gently mix the master mixes by flicking or by vortexing, and then pulse spin. Add 15 μL of qPCR Master Mix to each well at room temperature.

In a template area, add 130 μL of SeptiCyte Lab Test Diluent to each cDNA product from the RT Reaction. Reseal the plate tightly and vortex the plate to mix thoroughly.

Add 10 μL of diluted cDNA product to each well according to the plate layout.

Mix by gentle pipetting. Avoid forming bubbles in the wells.

Cover wells with an optical seal.

Spin the plate to remove any bubbles (1 minute at 400 x g).

Place on real-time thermal cycler pre-programmed with the settings above.

Click Start. Click Save and Continue. Before leaving the instrument, it is recommended to verify that the run started successfully by displaying a time under Estimated Time Remaining.

Note: Do not open the qPCR plate at any point after amplification has begun. When amplification has completed, discard the unopened plate.

Software. Interpretation of Results and Quality Control

Software is specifically designed to integrate with the output of PCR machines and to apply an algorithm based on the use of multiple biomarkers. The software takes into account appropriate controls and reports results in a desired format.

When the run has completed on the ABI 7500 Fast Dx Instrument, complete the steps below in the application 7500 Fast System with 21 CFR Part 11 Software, ABI software SDS v1.4.

Click on the Results tab in the upper left corner.

Click on the Amplification Plot tab in the upper left corner.

In the Analysis Settings area, select an auto baseline and manual threshold for all targets. Enter 0.01 as the threshold.

Click on the Analyse button on the right in the Analysis Settings area.

From the menu bar in the upper left, select File then Close.

Complete the form in the dialog box that requests a reason for the change. Click OK.

Transfer the data file (.sds) to a separate computer running the specific assay RT-qPCR Test Software.

Launch the assay RT-qPCR Test Software. Log in.

From the menu bar in the upper left, select File then Open.

Browse to the location of the transferred data file (.sds). Click OK.

The data file will then be analysed using the assay's software application for interpretation of results.

Interpretation of Results and Quality Control

*Results*

Launch the interpretation software. Software application instructions are provided separately.

Following upload of the. sds file, the Software will automatically generate classifier scores for controls and clinical specimens.

Controls

The Software compares each CON (control) specimen (CONH, CONL, CONN) to its expected result. The controls are run in singleton.

| Control specimen | | |
|---|---|---|
| **Designation** | **Name** | **Expected result** |
| CONH | High Control | Score range |
| CONL | Low Control | Score range |
| CONN | Negative Control | Score range |
| NTC | No Template Control | Fail (no Ct for all targets) |

If CONH, CONL, and/or CONN fail the batch run is invalid and no data will be reported for the clinical specimens. This determination is made automatically by the interpretive software. The batch run should be repeated starting with either a new RNA preparation or starting at the RT reaction step.

If NTC yields a result other than Fail (no Ct for all targets), the batch run is invalid and no data may be reported for the clinical specimens. This determination is made by visual inspection of the run data. The batch run should be repeated starting with either a new RNA preparation or starting at the RT reaction step.

If a second batch run fails, please contact technical services. If both the calibrations and all controls are valid, then the batch run is valid and specimen results will be reported.

Specimens

Note that a valid batch run may contain both valid and invalid specimen results.

Analytical criteria (e.g. Ct values) that qualify each specimen as passing or failing (using pre-determined data) are called automatically by the software.

Scores out of range - reported.

Quality Control

Singletons each of the Negative Control, Low Positive Control, and High Positive Control must be included in each batch run. The batch is valid if no flags appear for any of these controls.

A singleton of the No Template Control is included in each batch run and Fail (no Ct for all targets) is a valid result indicating no amplifiable material was detectable in the well.

The negative control must yield a Negative result. If the negative control is flagged as Invalid, then the entire batch run is invalid.

The low positive and high positive controls must fall within the assigned ranges. If one or both of the positive controls are flagged as Invalid, then the entire batch run is invalid.

EXAMPLE 9

**EXAMPLE OUTPUT**

A possible example output from the software for a HVaSIRS assay is presented in Figure 9. The format of such a report depends on many factors including; quality control, regulatory authorities, cut-off values, the algorithm used, laboratory and clinician requirements, likelihood of misinterpretation.

In this instance the assay is called "SeptiCyte Herpes". The result is reported as a number (6), a position on a 0-10 scale, and a probability of the patient having a HVaSIRS based on historical results and the use of a pre-determined cut-off (using results from clinical studies). Results of controls within the assay may also be reported. Other information that could be reported might include: previous results and date and time of such results, a prognosis, a scale that provides cut-off values for historical testing results that separate the conditions of healthy, inSIRS and HVaSIRS such that those patients with higher scores are considered to have more severe HVaSIRS.

EXAMPLE 11

**SECOND EXAMPLE WORKFLOW**

A second example workflow will now be described. Machines have been, and are being, developed that are capable of processing a patient sample at point-of-care, or near point-of-care. Such machines require few molecular biology skills to run and are aimed at non-technical users. The idea is that the sample would be pipetted directly into a disposable cartridge(s) that is/are then inserted into the machine. For determining a specific host response, the cartridge will need to extract high quality RNA from the cells in the sample for use in reverse transcription followed by RT-PCR. The machines are designed for minimum user interaction such that the user presses "Start" and within 1-3 hours results are generated. The cartridges contain all of the required reagents to perform host cell nucleic acid extraction (RNA), reverse transcription, and qRT-PCR, and the machine has appropriate software incorporated to allow use of algorithms to interpret each result and combine results, and final interpretation and printing of results.

Fresh, whole, anti-coagulated blood can be pipetted into a specialized cartridge (e.g. cartridges designed for Enigma ML machine by Enigma Diagnostics Limited (Enigma Diagnostics Limited, Building 224, Tetricus Science Park, Dstl, Porton Down, Salisbury, Wiltshire SP4 0JQ) or similar (Unyvero, Curetis AG, Max-Eyth-Str. 42 71088 Holzgerlingen, Germany)), and on-screen instructions followed to test for differentiating a HVaSIRS from other forms of SIRS. Inside the machine RNA is first extracted from the whole blood and is then converted into cDNA. The cDNA is then used in qRT-PCR reactions. The reactions are followed in real time and Ct values calculated. On-board software generates a result output (see, Figure 9). Appropriate quality control measures for RNA quality, no template controls, high and low template controls and expected Ct ranges ensure that results are not reported erroneously.

EXAMPLE 11

**EXAMPLE ALGORITHM COMBINING DERIVED BIOMARKERS FOR ASSESSING A HVASIRS**

Derived biomarkers can be used in combination to increase the diagnostic power for separating various conditions. Determining which markers to use, and how many, for separating various conditions can be achieved by calculating Area Under Curve (AUC).

As such, and by example, a 6-HVaSIRS polynucleotide biomarker profile (AUC 0.947) offers the appropriate balance between simplicity, practicality and commercial risk for diagnosing HVaSIRS. Further, an equation using six biomarkers weighs each biomarker equally which also provides additional robustness in cases of analytical or clinical variability.

One example equation (amongst others) that provides good diagnostic power for diagnosing a HVaSIRS is:

$$\text{Diagnostic Score} = (CCL5 - FLNB) + (PPP2R2B - GNG7) + (CASZ1 - VASH1)$$

Note: each biomarker in the Diagnostic Score above is the Log 2 transformed concentration of the marker in the sample and biomarkers are oriented so that cases (HVaSIRS) are high and controls are low. Change of orientation of biomarkers in a derived biomarker does not affect diagnostic performance - rather it is the specific combination that generates diagnostic performance.

**TABLES**

TABLE 1

| DATASET USED FOR "DISCOVERY" OF HERPESVIRUS SIGNATURES | | |
|---|---|---|
| **Dataset Identifier** | **Virus name** | **Study Description, Sample Comparison** |
| GSE40366 | CMV | Human patients, single sample. Study designed to determine peripheral blood gene expression differences between old and young humans (nonagenerians vs less than 30 years of age). CMV titers were also determined as part of the study. Samples from any subject with no CMV titer (0) were compared to those with a CMV titer > 20,000. |

TABLE 2

| DATASETS USED TO IDENTIFY BIOMARKERS ASSOCIATED WITH NON-HERPES AND NON-VIRAL INFLAMMATION | | |
|---|---|---|
| **Dataset Identifier** | **Condition** | **Study Description** |
| GSE52428 | Influenza virus | 18 human subjects experimentally-infected with Influenza virus A (two strains). Samples taken pre-infection and every eight hours out to 108 hours post-infection. Symptomatic and asymptomatic responses. |
| GSE41752 | Lassa virus | 11 macaques with experimentally-induced Lassa virus infection. Samples collected pre-infection and on Days 2, 3, 6, 8, 10 and 12 post-infection. Total 46 processed samples. |
| GSE33341 | Bacteremia; Staphylocccus aurues or Escherichia coli | Human patients and subjects, single time point. Staphylococcus aureus: 34 Escherichia coli: 15 Healthy: 43 |
| GSE40366 | Age | 143 nonagenerians and 30 young subjects. Comparison of those subjects without CMV titers. |
| GSE42834 | Tuberculosis Sarcoidosis Lung cancer Pneumonia | Human subjects, single time point. Tuberculosis: 66 Sarcoidosis (active, 68; non-active, 22) Lung cancer: 16 Pneumonia: 16 Control: 147 |
| GSE25504 | Neonatal sepsis | Human subjects, single time point. Sepsis: 28 Controls: 35 |
| GSE17755 | Autoimmune disease | Human subjects, single time point. Rheumatoid arthritis: 112 |
| **Dataset Identifier** | **Condition** | **Study Description** |
| | | Systemic Lupus Erythematosis: 22 Poly juvenile idiopathic arthritis: 6 Systemic juvenile idiopathic arthritis: 51 Healthy: 53 |

(continued)

| Dataset Identifier | Condition | Study Description |
|---|---|---|
| GSE35846 | Race, age, gender, obesity | Human patients, single sample.<br>189 subjects.<br>65 men and 124 women.<br>Aged 26 and 79 (mean 51).<br>140 Caucasian, 37 African American, 11 Asian, 1 American Indian. |

TABLE 3

| DATASETS USED FOR "VALIDATION" OF HERPESVIRUS SIGNATURES | | |
|---|---|---|
| Dataset Identifier | Virus names | Study Description |
| GSE40366 | CMV_Age | Human patients, single sample.<br>Study designed to determine peripheral blood gene expression differences between old and young humans (nonagenerians vs less than 30 years of age). CMV titers were also determined as part of the study.<br>Samples from nonagenerians with no CMV titer (0) were compared to young subjects with no CMV titer. |
| GSE45919 | EBV | Human patients, three samples at three time points; before, during and convalescent.<br>Study designed to identify what gene expression changes occur over time with a primary EBV infection.<br>Samples from patients prior to EBV infection (9) were compared to those samples taken during a primary infection (3). |
| GSE40396 | HHV6 | Human patients, single sample.<br>Study designed to identify peripheral blood gene expression signatures associated with either a viral or bacterial infection.<br>Samples from patients with HHV6 (10) were compared to healthy control subjects (19). |
| MARS | CMV<br>EBV<br>HSV1 and 2 | Human patients, single time point.<br>Study designed to validate a gene expression signature for differentiating SIRS and sepsis.<br>Samples from patients where a potential herpes virus infection had been determined - either by determination of plasma IgM / IgG or direct PCR. |

TABLE 4

| GREEDY ADDITION RESULTS FOR EACH OF THE NORMALIZED AND SCALED RATIOS ACROSS ALL POSITIVE AND NEGATIVE DATASETS | | | | |
|---|---|---|---|---|
| | Greedy ratio addition | AUC positive | AUC negative | Difference |
| 1 | CCL5_FLNB | 0.864 | 0.500 | 0.364 |
| 2 | PPP2R2B_GNG7 | 0.914 | 0.514 | 0.401 |
| 3 | CASZ1_VASH1 | 0.947 | 0.530 | 0.417 |
| 4 | GFI1_RPL30 | 0.960 | 0.539 | 0.421 |
| 5 | CHST12_TMC6 | 0.966 | 0.535 | 0.431 |
| 6 | ADRB2_RALA | 0.963 | 0.533 | 0.431 |

(continued)

| GREEDY ADDITION RESULTS FOR EACH OF THE NORMALIZED AND SCALED RATIOS ACROSS ALL POSITIVE AND NEGATIVE DATASETS | | | | |
|---|---|---|---|---|
| | Greedy ratio addition | AUC positive | AUC negative | Difference |
| 7 | SYNE2_BACH2 | 0.963 | 0.541 | 0.422 |
| 8 | CD8A_RPS5 | 0.962 | 0.549 | 0.413 |
| 9 | FAM129A_SPINT2 | 0.966 | 0.561 | 0.405 |
| 10 | TGFBR3_ETS1 | 0.965 | 0.567 | 0.398 |

TABLE 5

| PERFORMANCE (AUC) OF THE 452 DERIVED BIOMARKERS ACROSS THE NEGATIVE* AND POSITIVE[1] VALIDATION DATASETS TO INCLUDE THE MEAN FOR ALL NINE NEGAT DATASETS AND FIVE POSITIVE DATASETS VE | | | | | | | | | | | | | | | | |
|---|---|---|---|---|---|---|---|---|---|---|---|---|---|---|---|---|
| | Negative Validation Datasets* | | | | | | | | | | Positive Validation Datasets (1) | | | | | | |
| Derived Biomarker | 1 | 2 | 3 | 4 | 5 | 6 | 7 | 8 | 9 | Mean | 1 | 2 | 3 | 4 | 5 | Mean | Mean Difference |
| BACH2_GDPD5 | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| CAMK1D_OASL | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| CCR7_CCL5 | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| CCR7_OASL | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| CCR7_PPP2R2B | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| CD79A_OASL | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| CRY2_CCL5 | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| DHRS3_PPP2R2B | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| DPEP2_FAM129A | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| EIF2S3_OASL | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| EPHX2_CASZ1 | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| EPHX2_CD8A | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| EPHX2_HERC6 | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| EPHX2_KPNB1 | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| EPHX2_SLAMF7 | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| FLNB_DNPEP | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| FLNB_EIF2AK2 | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| FLNB_FDFT1 | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| FLNB_GZMA | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| FLNB_IFITM1 | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| FLNB_PPP2R2B | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| FLNB_RARRES3 | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| FLNB_TBK1 | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| FLNB_UBR2 | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| FLNB_ZBP1 | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| FLT3LG_OASL | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| GNG7_BTG3 | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| GNG7_IFI44 | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| GNG7_TAP1 | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |

| PERFORMANCE (AUC) OF THE 452 DERIVED BIOMARKERS ACROSS THE NEGATIVE* AND POSITIVE[1] VALIDATION DATASETS TO INCLUDE THE MEAN FOR ALL NINE NEGAT DATASETS AND FIVE POSITIVE DATASETS VE | | | | | | | | | | | | | | | | | |
|---|---|---|---|---|---|---|---|---|---|---|---|---|---|---|---|---|---|
| | Negative Validation Datasets* | | | | | | | | | | Positive Validation Datasets (1) | | | | | | |
| Derived Biomarker | 1 | 2 | 3 | 4 | 5 | 6 | 7 | 8 | 9 | Mean | 1 | 2 | 3 | 4 | 5 | Mean | Mean Difference |
| ITM2C_GFI1 | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| ITM2C_PPP2R2B | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| LAMA5 OASL | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| LDLRAPI_OASL | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| LEF1_BTG3 | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| LEF1_CD8A | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| LEF1_CYB561 | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| LEF1_PPP2R2B | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| MAL_ZBP1 | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| NACA_ARSB | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| NACA_CD300A | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| NACA_SLAMF7 | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| NOSIP_CST7 | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| NOSIP_ZBP1 | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| PEX11B_PPP2R2B | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| PFKL_OASL | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| PIK3IP1_GFI1 | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| RALA_TPST2 | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| RPAIN_TPST2 | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| RPL22_CCL5 | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| RPL22_GALNT10 | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| RPL22_PPP2R2B | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| RPL22_SYT11 | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| RPL22_TGFBR3 | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| RPS5_CD8A | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| SERPINE2_OASL | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| SERPINE2_PPP2R2B | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| SPINT2_SQRDL | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |

| PERFORMANCE (AUC) OF THE 452 DERIVED BIOMARKERS ACROSS THE NEGATIVE* AND POSITIVE[1] VALIDATION DATASETS TO INCLUDE THE MEAN FOR ALL NINE NEGAT DATASETS AND FIVE POSITIVE DATASETS VE | | | | | | | | | | | | | | | | | |
|---|---|---|---|---|---|---|---|---|---|---|---|---|---|---|---|---|---|
| | Negative Validation Datasets* | | | | | | | | | | Positive Validation Datasets (1) | | | | | | |
| Derived Biomarker | 1 | 2 | 3 | 4 | 5 | 6 | 7 | 8 | 9 | Mean | 1 | 2 | 3 | 4 | 5 | Mean | Mean Difference |
| SREBFI_OASL | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| SYPL1_CASP4 | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| SYPL1_FAM129A | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| TCL1A_GFI1 | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| TMEM134_OASL | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| TMEM39B_OASL | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| TOP2B_PPP2R2B | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| TPK1_FAM129A | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| VASH1_PARP3 | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| VPREB3_ADRB2 | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| VPREB3_GFI1 | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| VPREB3_OASL | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| XPC_OASL | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| ZW10_PPP2R2B | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| ZW10_TAP1 | 0.54 | 0.80 | 0.51 | 0.53 | 0.79 | 0.66 | 0.71 | 0.52 | 0.65 | 0.63 | 0.85 | 0.81 | 0.93 | 0.96 | 0.87 | 0.88 | 0.25 |
| BACH2_FDFT1 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| BACH2_GFI1 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| BACH2_IFI16 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| BACH2_SYNE2 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| BACH2_TPST2 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| CAMK1D_CD300A | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| CAMK1D_CST7 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| CAMK1D_CYB561 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| CAMK1D_GALNT10 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| CAMK1D_TPST2 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| CD37_OASL | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| CD79B_CCL5 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| CD79B_SLAMF7 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |

| PERFORMANCE (AUC) OF THE 452 DERIVED BIOMARKERS ACROSS THE NEGATIVE* AND POSITIVE[1] VALIDATION DATASETS TO INCLUDE THE MEAN FOR ALL NINE NEGAT DATASETS AND FIVE POSITIVE DATASETS VE | | | | | | | | | | | | | | | | | |
|---|---|---|---|---|---|---|---|---|---|---|---|---|---|---|---|---|---|
| | Negative Validation Datasets* | | | | | | | | | | Positive Validation Datasets (1) | | | | | | |
| Derived Biomarker | 1 | 2 | 3 | 4 | 5 | 6 | 7 | 8 | 9 | Mean | 1 | 2 | 3 | 4 | 5 | Mean | Mean Difference |
| CYLD_OASL | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| DHRS3_GFI1 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| DHRS3_TPST2 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| DPEP2_UBR2 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| EEFIG_GALNT10 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| EPHX2_BTG3 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| EPHX2_CYB561 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| EPHX2_EIF2AK2 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| EPHX2_GFI1 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| ETS1_TGFBR3 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| FLNB_CST7 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| FLNB_EHD1 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| FLNB_GDPD5 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| FLNB_GPR56 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| FLNB_NKG7 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| FLNB_SLAMF7 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| GNG7_COL17A1 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| GNG7_CST7 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| GNG7_EIF2AK2 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| GNG7_HERC5 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| GNG7_KIF13B | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| GNG7_TMUB2 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| GNG7_TPST2 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| LAMA5_GFI1 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| LDLRAP1_GFI1 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| LSM7_PPP2R2B | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| MRPS18B_OASL | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| MZF1_PPP2R2B | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |

| PERFORMANCE (AUC) OF THE 452 DERIVED BIOMARKERS ACROSS THE NEGATIVE* AND POSITIVE[1] VALIDATION DATASETS TO INCLUDE THE MEAN FOR ALL NINE NEGAT DATASETS AND FIVE POSITIVE DATASETS VE | | | | | | | | | | | | | | | | | |
|---|---|---|---|---|---|---|---|---|---|---|---|---|---|---|---|---|---|
| | Negative Validation Datasets* | | | | | | | | | | Positive Validation Datasets (1) | | | | | | |
| Derived Biomarker | 1 | 2 | 3 | 4 | 5 | 6 | 7 | 8 | 9 | Mean | 1 | 2 | 3 | 4 | 5 | Mean | Mean Difference |
| NACA_ADRB2 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| NACA_OASL | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| NAP1L1_FAM129A | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| NGFRAP1_OASL | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| NOSIP_CHST12 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| NOSIP_GDPD5 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| NOSIP_TAP1 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| PABPC4_CCL5 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| PHB2_GFI1 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| PTOV1_PRDM1 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| RAB3GAP1_OASL | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| RALA_ADRB2 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| RALA_OASL | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| RALA_PPP2R2B | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| RBM17_PPP2R2B | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| RBM4B_PPP2R2B | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| RPL22_RARRES3 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| RPL22_TPST2 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| RPL36_OASL | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| SATBI_OASL | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| SLC5A6_OASL | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| TCF4_OASL | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| TCL1A_CCL5 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| TMC6_CCL5 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| TMC6_NKG7 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| TMC6_OASL | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| UBE2D2_OASL | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| UBE2D2_PPP2R2B | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |

| PERFORMANCE (AUC) OF THE 452 DERIVED BIOMARKERS ACROSS THE NEGATIVE* AND POSITIVE[1] VALIDATION DATASETS TO INCLUDE THE MEAN FOR ALL NINE NEGAT DATASETS AND FIVE POSITIVE DATASETS VE | | | | | | | | | | | | | | | | | |
|---|---|---|---|---|---|---|---|---|---|---|---|---|---|---|---|---|---|
| | Negative Validation Datasets* | | | | | | | | | | Positive Validation Datasets (1) | | | | | | |
| Derived Biomarker | 1 | 2 | 3 | 4 | 5 | 6 | 7 | 8 | 9 | Mean | 1 | 2 | 3 | 4 | 5 | Mean | Mean Difference |
| VASH1_CASZ1 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| VASH1_PRDM1 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| VPREB3_CST7 | 0.61 | 0.63 | 0.91 | 0.68 | 0.83 | 0.63 | 0.69 | 0.64 | 0.59 | 0.69 | 0.86 | 0.93 | 0.91 | 1.00 | 0.90 | 0.92 | 0.23 |
| AKAP11_OASL | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| ARHGEF18_OASL | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| BACH2_IFITM1 | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| BACH2_PPP2R2B | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| BACH2_TDRD7 | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| BNIP3_PPP2R2B | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| C6orf48_OASL | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| CAMK1D_ADCY7 | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| CD79A_CCL5 | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| COX4I1_OASL | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| DGKA_OASL | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| DHRS3_CCL5 | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| DHRS3_OASL | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| DPEP2_OASL | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| EEF1G_TPST2 | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| EIF4B_GFI1 | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| EPHX2_ADCY7 | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| EPHX2_ADRB2 | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| EPHX2_GZMA | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| EPHX2_TAP1 | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| EPHX2_TPST2 | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| ETS1_CCL5 | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| ETS1_CD8A | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| ETS1_PPP2R2B | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| FLNB_ADRB2 | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |

EP 3 371 324 B1

| PERFORMANCE (AUC) OF THE 452 DERIVED BIOMARKERS ACROSS THE NEGATIVE* AND POSITIVE[1] VALIDATION DATASETS TO INCLUDE THE MEAN FOR ALL NINE NEGAT DATASETS AND FIVE POSITIVE DATASETS VE | | | | | | | | | | | | | | | | | |
|---|---|---|---|---|---|---|---|---|---|---|---|---|---|---|---|---|---|
| | Negative Validation Datasets* | | | | | | | | | | Positive Validation Datasets (1) | | | | | | |
| Derived Biomarker | 1 | 2 | 3 | 4 | 5 | 6 | 7 | 8 | 9 | Mean | 1 | 2 | 3 | 4 | 5 | Mean | Mean Difference |
| FLNB_CASZ1 | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| FLNB_HERC5 | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| FLNB_PFKP | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| GNG7_ADRB2 | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| GNG7_CASZ1 | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| GNG7_CD300A | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| GNG7_FDFT1 | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| GNG7_GALNT10 | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| GNG7_IFITM1 | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| GNG7_NKG7 | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| GNG7_PARP3 | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| GNG7_PXN | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| GNG7_SLAMF7 | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| GNG7_SYT11 | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| GNG7_UBE2L6 | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| HIC2_CASZ1 | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| HSD17B8_CD8A | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| HSD17B8_GFI1 | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| ITM2C_ZBP1 | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| LAMA5_PPP2R2B | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| LBH_PPP2R2B | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| LEF1_GDPD5 | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| LEF1_TGFBR3 | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| MAL_OASL | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| MAL_PPP2R2B | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| NAALADL1_RARRES 3 | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| NACA_PPP2R2B | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| NACA_RABGAP1L | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |

| PERFORMANCE (AUC) OF THE 452 DERIVED BIOMARKERS ACROSS THE NEGATIVE* AND POSITIVE[1] VALIDATION DATASETS TO INCLUDE THE MEAN FOR ALL NINE NEGAT DATASETS AND FIVE POSITIVE DATASETS VE | | | | | | | | | | | | | | | | | |
|---|---|---|---|---|---|---|---|---|---|---|---|---|---|---|---|---|---|
| | Negative Validation Datasets* | | | | | | | | | | Positive Validation Datasets (1) | | | | | | |
| Derived Biomarker | 1 | 2 | 3 | 4 | 5 | 6 | 7 | 8 | 9 | Mean | 1 | 2 | 3 | 4 | 5 | Mean | Mean Difference |
| NAP1L1_PRDM1 | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| NGFRAP1_FAM129A | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| RPL10A_CD8A | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| RPL22_CST7 | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| RPL22_FAM129A | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| RPL22_OASL | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| RPL22_TAP1 | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| RPL36_PPP2R2B | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| RPL36_RARRES3 | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| RPS5_PPP2R2B | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| TBC1D4_OASL | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| TCL1A_PPP2R2B | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| TCL1A_ZBP1 | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| TOP2B_GFI1 | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| VASH1_ARSB | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| VASH1_CST7 | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| VASH1_OASL | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| VPREB3_CD8A | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| VPREB3_RARRES3 | 0.69 | 0.52 | 0.82 | 0.61 | 0.74 | 0.64 | 0.78 | 0.60 | 0.46 | 0.65 | 0.85 | 0.86 | 1.00 | 0.93 | 0.74 | 0.88 | 0.22 |
| ALDOC_CCL5 | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| BACH2_ARNTL | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| BACH2_CD300A | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| BACH2_FAM129A | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| BACH2_IFI44 | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| BCL11A_OASL | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| BCL11A_PPP2R2B | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| BTG1_OASL | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| CD79B_CD300A | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |

EP 3 371 324 B1

58

| PERFORMANCE (AUC) OF THE 452 DERIVED BIOMARKERS ACROSS THE NEGATIVE* AND POSITIVE[1] VALIDATION DATASETS TO INCLUDE THE MEAN FOR ALL NINE NEGAT DATASETS AND FIVE POSITIVE DATASETS VE | | | | | | | | | | | | | | | | | |
|---|---|---|---|---|---|---|---|---|---|---|---|---|---|---|---|---|---|
| | Negative Validation Datasets* | | | | | | | | | | Positive Validation Datasets (1) | | | | | | |
| Derived Biomarker | 1 | 2 | 3 | 4 | 5 | 6 | 7 | 8 | 9 | Mean | 1 | 2 | 3 | 4 | 5 | Mean | Mean Difference |
| CD79B_CD8A | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| CD79B_OASL | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| CD79B_ZBP1 | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| EEF1G_OASL | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| EPHX2_CD300A | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| EPHX2_IFI44 | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| EPHX2_IFITM 1 | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| EPHX2_OASL | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| EPHX2_PPP2R2B | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| EPHX2_ZBP1 | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| FLNB_ATP2B4 | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| FLNB_CCL5 | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| FLNB_FAM129A | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| FLNB_GFI1 | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| FLNB_IKBKG | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| FLNB_KIF13B | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| FLNB_OASL | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| FLNB_RUNX3 | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| GNG7_DNPEP | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| GNG7_EHD1 | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| GNG7_PPP2R2B | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| GNG7_TDRD7 | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| GNG7_ZBP1 | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| GPR162_OASL | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| GRWD1_OASL | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| HSD17B8_OASL | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| LDLRAP1_CCL5 | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| LDLRAP1_PPP2R2B | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |

EP 3 371 324 B1

| PERFORMANCE (AUC) OF THE 452 DERIVED BIOMARKERS ACROSS THE NEGATIVE* AND POSITIVE[1] VALIDATION DATASETS TO INCLUDE THE MEAN FOR ALL NINE NEGAT DATASETS AND FIVE POSITIVE DATASETS VE | | | | | | | | | | | | | | | | | |
|---|---|---|---|---|---|---|---|---|---|---|---|---|---|---|---|---|---|
| | Negative Validation Datasets* | | | | | | | | | | Positive Validation Datasets (1) | | | | | | |
| Derived Biomarker | 1 | 2 | 3 | 4 | 5 | 6 | 7 | 8 | 9 | Mean | 1 | 2 | 3 | 4 | 5 | Mean | Mean Difference |
| LTBP3_OASL | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| MAL_CCL5 | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| MAL_TGFBR3 | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| NAALADL1-OASL | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| NACA_BTG3 | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| NACA_FAM129A | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| NACA_GALNT10 | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| NACA_GZMA | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| NACA_SYT11 | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| NAP1L1_PPP2R2B | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| NOSIP_OASL | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| NOSIP_PRKD2 | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| PIK3IP1-OASL | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| POP5_TPST2 | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| PTOV1_OASL | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| RPL10A_CST7 | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| RPL10A_PPP2R2B | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| RPL22_CD8A | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| RPL22_GZMA | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| RPL22_KPNB1 | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| RPL22_TXN | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| RPL27_TPST2 | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| RPL30_PPP2R2B | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| RPL36_GFI1 | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| RPL8_GFI1 | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| SERBP1_PPP2R2B | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| SRM_OASL | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| TMC6_PPP2R2B | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |

| PERFORMANCE (AUC) OF THE 452 DERIVED BIOMARKERS ACROSS THE NEGATIVE* AND POSITIVE[1] VALIDATION DATASETS TO INCLUDE THE MEAN FOR ALL NINE NEGAT DATASETS AND FIVE POSITIVE DATASETS VE | | | | | | | | | | | | | | | | | |
|---|---|---|---|---|---|---|---|---|---|---|---|---|---|---|---|---|---|
| | Negative Validation Datasets* | | | | | | | | | | Positive Validation Datasets (1) | | | | | | |
| Derived Biomarker | 1 | 2 | 3 | 4 | 5 | 6 | 7 | 8 | 9 | Mean | 1 | 2 | 3 | 4 | 5 | Mean | Mean Difference |
| TMEM39B_FAM129A | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| TRAF3IP3_PPP2R2B | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| VASH1_CYB561 | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| VPREB3_PPP2R2B | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| VPREB3_TPST2 | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| VPS35_FAM129A | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| VPS35_IFI16 | 0.53 | 0.85 | 0.61 | 0.53 | 0.80 | 0.83 | 0.71 | 0.60 | 0.50 | 0.66 | 0.84 | 0.82 | 0.94 | 1.00 | 0.69 | 0.86 | 0.19 |
| BACH2_KPNB1 | 0.68 | 0.53 | 0.65 | 0.80 | 0.93 | 0.74 | 0.86 | 0.71 | 0.70 | 0.73 | 0.82 | 0.90 | 0.99 | 0.89 | 0.68 | 0.85 | 0.12 |
| EEFIG_CST7 | 0.68 | 0.53 | 0.65 | 0.80 | 0.93 | 0.74 | 0.86 | 0.71 | 0.70 | 0.73 | 0.82 | 0.90 | 0.99 | 0.89 | 0.68 | 0.85 | 0.12 |
| EPHX2_GDPD5 | 0.68 | 0.53 | 0.65 | 0.80 | 0.93 | 0.74 | 0.86 | 0.71 | 0.70 | 0.73 | 0.82 | 0.90 | 0.99 | 0.89 | 0.68 | 0.85 | 0.12 |
| GNG7_RARRES3 | 0.68 | 0.53 | 0.65 | 0.80 | 0.93 | 0.74 | 0.86 | 0.71 | 0.70 | 0.73 | 0.82 | 0.90 | 0.99 | 0.89 | 0.68 | 0.85 | 0.12 |
| HSD17B8_PPP2R2B | 0.68 | 0.53 | 0.65 | 0.80 | 0.93 | 0.74 | 0.86 | 0.71 | 0.70 | 0.73 | 0.82 | 0.90 | 0.99 | 0.89 | 0.68 | 0.85 | 0.12 |
| LEF1_FAM129A | 0.68 | 0.53 | 0.65 | 0.80 | 0.93 | 0.74 | 0.86 | 0.71 | 0.70 | 0.73 | 0.82 | 0.90 | 0.99 | 0.89 | 0.68 | 0.85 | 0.12 |
| LRMP_OASL | 0.68 | 0.53 | 0.65 | 0.80 | 0.93 | 0.74 | 0.86 | 0.71 | 0.70 | 0.73 | 0.82 | 0.90 | 0.99 | 0.89 | 0.68 | 0.85 | 0.12 |
| NACA_TBK1 | 0.68 | 0.53 | 0.65 | 0.80 | 0.93 | 0.74 | 0.86 | 0.71 | 0.70 | 0.73 | 0.82 | 0.90 | 0.99 | 0.89 | 0.68 | 0.85 | 0.12 |
| NAP1L1_GFI1 | 0.68 | 0.53 | 0.65 | 0.80 | 0.93 | 0.74 | 0.86 | 0.71 | 0.70 | 0.73 | 0.82 | 0.90 | 0.99 | 0.89 | 0.68 | 0.85 | 0.12 |
| NECAP2_OASL | 0.68 | 0.53 | 0.65 | 0.80 | 0.93 | 0.74 | 0.86 | 0.71 | 0.70 | 0.73 | 0.82 | 0.90 | 0.99 | 0.89 | 0.68 | 0.85 | 0.12 |
| RPL10A_GFI1 | 0.68 | 0.53 | 0.65 | 0.80 | 0.93 | 0.74 | 0.86 | 0.71 | 0.70 | 0.73 | 0.82 | 0.90 | 0.99 | 0.89 | 0.68 | 0.85 | 0.12 |
| RPL11_OASL | 0.68 | 0.53 | 0.65 | 0.80 | 0.93 | 0.74 | 0.86 | 0.71 | 0.70 | 0.73 | 0.82 | 0.90 | 0.99 | 0.89 | 0.68 | 0.85 | 0.12 |
| RPL15_GFI1 | 0.68 | 0.53 | 0.65 | 0.80 | 0.93 | 0.74 | 0.86 | 0.71 | 0.70 | 0.73 | 0.82 | 0.90 | 0.99 | 0.89 | 0.68 | 0.85 | 0.12 |
| RPL22_ADRB2 | 0.68 | 0.53 | 0.65 | 0.80 | 0.93 | 0.74 | 0.86 | 0.71 | 0.70 | 0.73 | 0.82 | 0.90 | 0.99 | 0.89 | 0.68 | 0.85 | 0.12 |
| RPS11_OASL | 0.68 | 0.53 | 0.65 | 0.80 | 0.93 | 0.74 | 0.86 | 0.71 | 0.70 | 0.73 | 0.82 | 0.90 | 0.99 | 0.89 | 0.68 | 0.85 | 0.12 |
| RPS9_OASL | 0.68 | 0.53 | 0.65 | 0.80 | 0.93 | 0.74 | 0.86 | 0.71 | 0.70 | 0.73 | 0.82 | 0.90 | 0.99 | 0.89 | 0.68 | 0.85 | 0.12 |
| SERPINE2_TPST2 | 0.68 | 0.53 | 0.65 | 0.80 | 0.93 | 0.74 | 0.86 | 0.71 | 0.70 | 0.73 | 0.82 | 0.90 | 0.99 | 0.89 | 0.68 | 0.85 | 0.12 |
| SPINT2_TAP1 | 0.68 | 0.53 | 0.65 | 0.80 | 0.93 | 0.74 | 0.86 | 0.71 | 0.70 | 0.73 | 0.82 | 0.90 | 0.99 | 0.89 | 0.68 | 0.85 | 0.12 |
| SYPL1_TPST2 | 0.68 | 0.53 | 0.65 | 0.80 | 0.93 | 0.74 | 0.86 | 0.71 | 0.70 | 0.73 | 0.82 | 0.90 | 0.99 | 0.89 | 0.68 | 0.85 | 0.12 |
| TBC1D4_GFI1 | 0.68 | 0.53 | 0.65 | 0.80 | 0.93 | 0.74 | 0.86 | 0.71 | 0.70 | 0.73 | 0.82 | 0.90 | 0.99 | 0.89 | 0.68 | 0.85 | 0.12 |
| ADSL_TPST2 | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |

| PERFORMANCE (AUC) OF THE 452 DERIVED BIOMARKERS ACROSS THE NEGATIVE* AND POSITIVE[1] VALIDATION DATASETS TO INCLUDE THE MEAN FOR ALL NINE NEGAT DATASETS AND FIVE POSITIVE DATASETS VE | | | | | | | | | | | | | | | | | |
|---|---|---|---|---|---|---|---|---|---|---|---|---|---|---|---|---|---|
| | Negative Validation Datasets* | | | | | | | | | | Positive Validation Datasets (1) | | | | | | |
| Derived Biomarker | 1 | 2 | 3 | 4 | 5 | 6 | 7 | 8 | 9 | Mean | 1 | 2 | 3 | 4 | 5 | Mean | Mean Difference |
| AKAP11_TDRD7 | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| BACH2_ADRB2 | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| BACH2_CD8A | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| BACH2_CST7 | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| BACH2_GZMA | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| BACH2_OASL | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| BACH2_PRDM1 | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| C6orf48_PPP2R2B | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| C6orf48_TPST2 | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| CAMK1D_FAM129A | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| CD93-FAM129A | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| EEF1G_CD8A | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| EEF1G_GFI1 | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| EIF2S3_PPP2R2B | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| EIF2S3_TPST2 | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| EPHX2_ADCY3 | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| EPHX2_ARNTL | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| EPHX2-FAM129A | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| EPHX2_PFKP | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| FLNB_ARNTL | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| FLNB_CD8A | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| FLNB_CYB561 | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| FLNB_PRDM1 | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| FLNB_PRKD2 | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| FLNB_TAP1 | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| FLNB_TPST2 | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| GNG7_GFI1 | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| GNG7_GPR56 | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |

| PERFORMANCE (AUC) OF THE 452 DERIVED BIOMARKERS ACROSS THE NEGATIVE* AND POSITIVE[1] VALIDATION DATASETS TO INCLUDE THE MEAN FOR ALL NINE NEGAT DATASETS AND FIVE POSITIVE DATASETS VE | | | | | | | | | | | | | | | | | |
|---|---|---|---|---|---|---|---|---|---|---|---|---|---|---|---|---|---|
| | Negative Validation Datasets* | | | | | | | | | | Positive Validation Datasets (1) | | | | | | |
| Derived Biomarker | 1 | 2 | 3 | 4 | 5 | 6 | 7 | 8 | 9 | Mean | 1 | 2 | 3 | 4 | 5 | Mean | Mean Difference |
| GNG7_KPNB1 | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| GNG7_LDLR | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| GNG7_OASL | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| GNG7_PRDM1 | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| GNG7_PRKD2 | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| HSD17B8_RARRES3 | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| ICAM2_OASL | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| ITPKB_GFI1 | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| LEF1_GFI1 | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| LEF1_PRDM1 | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| LEF1_PRKD2 | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| LEF1_RARRES3 | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| LEF1_ZBP1 | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| MLLT11_PPP2R2B | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| MZF1_OASL | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| NAALADL1_ZBP1 | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| NACA_C1QB | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| NACA_CST7 | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| NACA_CYB561 | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| NACA_PRDM1 | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| NETI_OASL | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| NOSIP_RARRES3 | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| PEX11B_GFI1 | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| PIK3IP1_CCL5 | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| PIK3IP1_PPP2R2B | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| PIK3IP1_TGFBR3 | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| PTOV1_FAM129A | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| PTOV1_PPP2R2B | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |

| PERFORMANCE (AUC) OF THE 452 DERIVED BIOMARKERS ACROSS THE NEGATIVE* AND POSITIVE[1] VALIDATION DATASETS TO INCLUDE THE MEAN FOR ALL NINE NEGAT DATASETS AND FIVE POSITIVE DATASETS VE | | | | | | | | | | | | | | | | | |
|---|---|---|---|---|---|---|---|---|---|---|---|---|---|---|---|---|---|
| | Negative Validation Datasets* | | | | | | | | | | Positive Validation Datasets (1) | | | | | | |
| Derived Biomarker | 1 | 2 | 3 | 4 | 5 | 6 | 7 | 8 | 9 | Mean | 1 | 2 | 3 | 4 | 5 | Mean | Mean Difference |
| RPL10A_ADRB2 | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| RPL10A_TPST2 | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| RPL22_GFI1 | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| RPL38_OASL | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| SERTAD2_CCL5 | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| SPINT2_OASL | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| SYPL1-OASL | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| TBC1D4_PPP2R2B | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| TMC6_CHST12 | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| TMEM39B_CD300A | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| TRAF3IP3_GFI1 | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| TRAF3IP3_OASL | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| VASH1_FAM129A | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| VPREB3_CCL5 | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| VPREB3_TAP1 | 0.49 | 0.82 | 0.98 | 0.59 | 0.96 | 0.70 | 0.79 | 0.79 | 0.66 | 0.75 | 0.84 | 0.88 | 1.00 | 0.70 | 0.81 | 0.85 | 0.09 |
| BACH2_EIF2AK2 | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| BACH2_RARRES3 | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| BACH2_TAP1 | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| BACH2_ZBP1 | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| C6orf48_CD300A | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| C6orf48_CST7 | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| C6orf48_GFI1 | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| CAMK1D_ZBP1 | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| CCR7_CHST12 | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| CD79B_GFI1 | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| CD79B_PPP2R2B | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| CD79B_TPST2 | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| CD93_ACSL1 | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |

| PERFORMANCE (AUC) OF THE 452 DERIVED BIOMARKERS ACROSS THE NEGATIVE* AND POSITIVE[1] VALIDATION DATASETS TO INCLUDE THE MEAN FOR ALL NINE NEGAT DATASETS AND FIVE POSITIVE DATASETS VE | | | | | | | | | | | | | | | | | |
|---|---|---|---|---|---|---|---|---|---|---|---|---|---|---|---|---|---|
| | Negative Validation Datasets* | | | | | | | | | | Positive Validation Datasets (1) | | | | | | |
| Derived Biomarker | 1 | 2 | 3 | 4 | 5 | 6 | 7 | 8 | 9 | Mean | 1 | 2 | 3 | 4 | 5 | Mean | Mean Difference |
| DHRS3_CHST12 | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| DPEP2_CD300A | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| EEF1G_PPP2R2B | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| EIF2S3_CD300A | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| EIF2S3_FAM 129A | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| EPHX2_CST7 | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| EPHX2_FDFT1 | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| EPHX2_PRDM1 | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| EPHX2_RARRES3 | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| ETS1_GFI1 | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| FLNB_CD300A | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| FLNB_PXN | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| FLNB_SYT11 | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| FLNB_TGFBR3 | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| GNG7_CYB561 | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| GNG7_FAM129A | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| GNG7_GDPD5 | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| GNG7_GZMA | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| GNG7_IKBKG | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| GNG7_REC8 | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| IL4R_OASL | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| ITM2C_CD300A | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| ITM2C_OASL | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| LEF1_ARNTL | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| LEF1_IFI44 | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| LEF1_OASL | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| MED25_OASL | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| NAALADL1_PPP2R2 | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |

| PERFORMANCE (AUC) OF THE 452 DERIVED BIOMARKERS ACROSS THE NEGATIVE* AND POSITIVE[1] VALIDATION DATASETS TO INCLUDE THE MEAN FOR ALL NINE NEGAT DATASETS AND FIVE POSITIVE DATASETS VE | | | | | | | | | | | | | | | | | |
|---|---|---|---|---|---|---|---|---|---|---|---|---|---|---|---|---|---|
| | Negative Validation Datasets* | | | | | | | | | | Positive Validation Datasets (1) | | | | | | |
| Derived Biomarker | 1 | 2 | 3 | 4 | 5 | 6 | 7 | 8 | 9 | Mean | 1 | 2 | 3 | 4 | 5 | Mean | Mean Difference |
| B | | | | | | | | | | | | | | | | | |
| NACA_GFI1 | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| NACA_NKG7 | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| NACA_RARRES3 | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| NACA_ZBP1 | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| NAP1L1_OASL | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| NOSIP-GFI1 | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| NOSIP_PPP2R2B | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| PEX11B_OASL | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| PLEKHB1_OASL | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| PTOV1_GDPD5 | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| RPL10A_OASL | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| RPL12_CCL5 | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| RPL12_GFI1 | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| RPL22_ACAT1 | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| RPL30_GFI1 | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| RPS5_GFI1 | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| RPS5_TPST2 | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| SAC3D1_OASL | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| SATB1_ZBP1 | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| SPINT2-FAM129A | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| SVIL_FAM129A | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| SYPL1-IFI16 | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| SYPL1_TANK | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| TCL1A_OASL | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| TMEM134_GFI1 | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| TRAF3IP3 _RARRES3 | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| UBE2D2_GFI1 | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |

(continued)

| PERFORMANCE (AUC) OF THE 452 DERIVED BIOMARKERS ACROSS THE NEGATIVE* AND POSITIVE[1] VALIDATION DATASETS TO INCLUDE THE MEAN FOR ALL NINE NEGAT DATASETS AND FIVE POSITIVE DATASETS VE | | | | | | | | | | | | | | | | | |
|---|---|---|---|---|---|---|---|---|---|---|---|---|---|---|---|---|---|
| | Negative Validation Datasets* | | | | | | | | | | Positive Validation Datasets (1) | | | | | | |
| Derived Biomarker | 1 | 2 | 3 | 4 | 5 | 6 | 7 | 8 | 9 | Mean | 1 | 2 | 3 | 4 | 5 | Mean | Mean Difference |
| VPREB3_CD300A | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| VPREB3_FAM129A | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| VPREB3_ZBP1 | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |
| WDR61_TPST2 | 0.57 | 0.77 | 0.95 | 0.65 | 0.82 | 0.88 | 0.89 | 0.68 | 0.69 | 0.77 | 0.82 | 0.84 | 0.95 | 1.00 | 0.61 | 0.85 | 0.08 |

The difference between the negative and positive mean in Table 5 is shown in the right hand column and derived markers are ordered from highest to lowest based on this difference. * Negative validation datasets 1-9 are, in order; GSE17755 (autoimmune disease), GSE25504 (neonatal sepsis), GSE33341 (bacterial infection), GSE40366 (age), GSE41752 (Lassa virus), GSE42834 (Tuberculosis, sarcoidosis, lung cancer), GSE52428 (influenza), GSE34205 (influenza), GSE34205 (respiratory syncytial virus). [1] Positive validation datasets are, in order; GSE40366 (cytomegalovirus), GSE40366 (CMV for 90 year olds), GSE40396 (HHV6), GSE45919 (Epstein-Barr Virus), MARS (CMV, EBV, HSV1 and 2). The orientation of a derived biomarker does not affect diagnostic performance (that is, CCL5_FLNB has the same diagnostic performance as FLNB_CCL5).

TABLE 6

| KEY HUMAN VIRAL PATHOGENS (BY VIRUS NAME AND TYPE) THAT ARE KNOWN TO CAUSE SYSTEMIC INFLAMMATION AND NON-SPECIFIC CLINICAL SIGNS OF FEVER, RASH, AND ACHES | | |
| --- | --- | --- |
| Clinical Signs | Virus Name | Virus Type |
| Fever / Rash / Aches / Generalised | Measles | Morbillivirus |
| | Hantavirus | Bunyavirus |
| | Cytomegalovirus | Herpesvirus |
| | Varicella Zoster Virus | Herpesvirus |
| | Herpes Simplex Virus | Herpevirus |
| | Epstein Barr Virus | Herpesvirus |
| | Parechovirus | Picornavirus |
| | Human immunodeficiency virus | Lentivirus |
| | Hepatitis B virus | Orthohepadnavirus |
| | HTLV1 and 2 | |
| | Vaccinia virus | Retrovirus |
| | West Nile Virus | Poxvirus |
| | Coxsackie virus | Flavivirus |
| | Parvovirus B19 | Picornavirus |
| | Dengue | Parvovirus |
| | | Flavivirus |

Underlined virus types in Table 6 are those demonstrably detected using the HVaSIRS biomarker signature

TABLE 7

| COMMON HUMAN VIRUSES THAT CAUSE SIRS AND A VIREMIA AS PART OF THEIR PATHOGENESIS AND AN ASSOCIATED REFERENCE | |
| --- | --- |
| Virus | Reference |
| Measles | de Vries RD, Mesman AW, Geijtenbeek TBH, Duprex WP, de Swart RL (2012) The pathogenesis of measles. Curr Opin Virol 2: 248-255. |
| Respiratory Syncytial Virus (RSV) | Rohwedder A, Keminer O, Forster J, Schneider K, Schneider E, et al. (1998) Detection of respiratory syncytial virus RNA in blood of neonates by polymerase chain reaction. J Med Virol 54: 320-327. |
| Influenza A and B | Wootton SH, Aguilera EA, Wanger A, Jewell A, Patel K, et al. (2014) Detection of NH1N1 influenza virus in nonrespiratory sites among children. Pediatr Infect Dis J 33: 95-96. |

(continued)

| Virus | Reference |
|---|---|
| Hepatitis B virus<br>Hepatitis C virus<br>Human immunodeficiency virus 1 and 2<br>HTLV1 and 2<br>Vaccinia virus<br>West Nile Virus | Pripuzova N, Wang R, Tsai S, Li B, Hung G-C, et al. (2012) Development of Real-Time PCR Array for Simultaneous Detection of Eight Human Blood-Borne Viral Pathogens. PLoS ONE 7: e43246. |
| Cytomegalovirus<br>Varicella Zoster Virus<br>Herpes Simplex Virus<br>Epstein Barr Virus | Johnson G, Nelson S, Petric M, Tellier R (2000) Comprehensive PCR-based assay for detection and species identification of human herpesviruses. Journal of Clinical Microbiology 38: 3274-3279. |
| Rhinovirus | Xatzipsalti M, Kyrana S, Tsolia M, Psarras S, Bossios A, et al. (2005) Rhinovirus viremia in children with respiratory infections. American Journal of Respiratory and Critical Care Medicine 172: 1037-1040. |
| Adenovirus<br>Bocavirus<br>Human Coronavirus types 229e, OC43, HKU1, NL-63<br>SARS coronavirus | Dunn JJ, Miller MB (2014) Emerging respiratory viruses other than influenza. Clin Lab Med 34: 409-430. |
| Hantavirus | Evander M, Eriksson I, Pettersson L, Juto P, Ahlm C, et al. (2007) Puumala Hantavirus Viremia Diagnosed by Real-Time Reverse Transcriptase PCR Using Samples from Patients with Hemorrhagic Fever and Renal Syndrome. Journal of Clinical Microbiology 45: 2491-2497. |
| Enterovirus | Cheng H-Y, Huang Y-C, Yen T-Y, Hsia S-H, Hsieh Y-C, et al. (2014) The correlation between the presence of viremia and clinical severity in patients with enterovirus 71 infection: a multi-center cohort study. BMC Infect Dis 14: 417. |
| Parechovirus | Abed Y, Boivin G (2006) Human parechovirus infections in Canada. Emerging Infectious Diseases 12: 969. |
| BK virus | Erard V, Storer B, Corey L, Nollkamper J, Huang M-L, et al. (2004) BK virus infection in hematopoietic stem cell transplant recipients: frequency, risk factors, and association with postengraftment hemorrhagic cystitis. Clin Infect Dis 39: 1861-1865. |
| Parainfluenza virus 1, 2, 3 and 4 | Gerkowicz T, Szajner-Milart I, Szczygielska J, Blaszynska M, Karska M, et al. (1979) Clinical manifestations of viremia during infections caused by adenoviruses and parainfluenza viruses. Pediatr Pol 54: 41-45 |
| SARS coronavirus | Wang WK, Fang CT, Chen HL, Yang CF, Chen YC, et al. (2005) Detection of Severe Acute Respiratory Syndrome Coronavirus RNA in Plasma during the Course of Infection. Journal of Clinical Microbiology 43: 962-965. |
| TTV (torque teno virus) | Walton AH, Muenzer JT, Rasche D, Boomer JS, Sato B, et al. (2014) Reactivation of multiple viruses in patients with sepsis. PLoS ONE 9: e98819. |
| Coxsackie virus | Clem AL, Sims J, Telang S, Eaton JW, Chesney J (2007) Virus detection and identification using random multiplex (RT)-PCR with 3'-locked random primers. Virol J 4: 65. |
| Parvovirus B19 | Juhl D, Steppat D, Görg S, Hennig H (2014) Parvovirus B19 Infections and Blood Counts in Blood Donors. Transfus Med Hemother 41: 6-6. |

(continued)

| Virus | Reference |
|---|---|
| Dengue | La Cruz Hernandez De SI, Flores-Aguilar H, Gonzalez-Mateos S, Lopez-Martinez I, Ortiz-Navarrete V, et al. (2013) Viral load in patients infected with dengue is modulated by the presence of anti-dengue IgM antibodies. J Clin Virol 58: 258-261. |

HVaSIRS biomarkers are useful in differentiating a SIRS caused by herpesviruses from a SIRS caused by other viruses listed in this table.

TABLE 8

| COMMON HUMAN VIRUSES THAT CAUSE SIRS AND A VIREMIA AS PART OF THEIR PATHOGENESIS AND FOR WHICH THERE ARE SPECIFIC ANTI-VIRAL TREATMENTS | |
|---|---|
| **Virus** | **Reference** |
| Influenza A and B | Wootton SH, Aguilera EA, Wanger A, Jewell A, Patel K, et al. (2014) Detection of NH1N1 influenza virus in nonrespiratory sites among children. Pediatr Infect Dis J 33: 95-96. |
| Hepatitis B virus Hepatitis C virus Human immunodeficiency virus 1 and 2 | Pripuzova N, Wang R, Tsai S, Li B, Hung G-C, et al. (2012) Development of Real-Time PCR Array for Simultaneous Detection of Eight Human Blood-Borne Viral Pathogens. PLoS ONE 7: e43246. |
| Cytomegalovirus Varicella Zoster Virus Herpes Simplex Virus Epstein Barr Virus | Johnson G, Nelson S, Petric M, Tellier R (2000) Comprehensive PCR-based assay for detection and species identification of human herpesviruses. Journal of Clinical Microbiology 38: 3274-3279. |
| | |

HVaSIRS biomarkers are useful in differentiating a SIRS caused by herpes viruses from a SIRS caused by other viruses listed in this table. As such, appropriate anti-viral treatment decisions can be made.

TABLE 9

| COMMON AND LESS COMMON VIRUSES ASSOCIATED WITH PNEUMONIA IN ADULTS AND CHILDREN. | |
|---|---|
| **Common** | **Less Common (or in specific hosts or settings)** |
| Respiratory Syncytial Virus (RSV) Influenza A and B Human Metapneumovirus Adenovirus Parainfluenza virus 1, 2, 3 and 4 Human Coronavirus types 229e, OC43, HKU1, NL-63 Rhinovirus Bocavirus | Measles Cytomegalovirus Varicella Zoster Virus Herpes Simplex Virus Epstein Barr Virus Hantavirus Enterovirus Parechovirus SARS Coronavirus |

HVaSIRS biomarkers are useful in differentiating a pneumonia caused by herpesviruses from a pneumonia caused by other viruses listed in this table. As such, appropriate anti-viral treatment decisions can be made. Herpesviruses are underlined.

# TABLE 10

## LISTS OF BIOMARKERS ACCORDING TO THEIR CORRELATION TO EITHER CCL5, FLNB, PPP2R2B, GNG7, CASZ1 OR VASH1 (GROUPS A – F).

| Group A | | | Group B | | | Group C | | | Group D | | | Group E | | | Group F | | |
|---|---|---|---|---|---|---|---|---|---|---|---|---|---|---|---|---|---|
| Correlation CCL5 | Symbol | DNA SEQ ID | Correlation FLNB | Symbol | DNA SEQ ID | Correlation PPP2R2B | Symbol | DNA SEQ ID | Correlation GNG7 | Symbol | DNA SEQ ID | Correlation CASZ1 | Symbol | DNA SEQ ID | Correlation VASH1 | Symbol | DNA SEQ ID |
| 0.55 | GZMA | 60 | 0.54 | EPHX2 | 47 | 0.46 | RUNX3 | 132 | 0.41 | CD79B | 28 | 0.27 | TMUB2 | 159 | 0.28 | GPR162 | 57 |
| 0.50 | TGFBR3 | 155 | 0.50 | CCR7 | 24 | 0.45 | GPR56 | 58 | 0.40 | CD79A | 27 | 0.27 | ARSB | 11 | 0.27 | NAALADL1 | 88 |
| 0.47 | CD8A | 29 | 0.47 | MAL | 83 | 0.37 | LBH | 76 | 0.38 | VPREB3 | 169 | 0.23 | C1QB | 18 | 0.23 | LTBP3 | 82 |
| 0.38 | PABPC4 | 97 | 0.46 | LEF1 | 79 | 0.30 | POP5 | 105 | 0.36 | TCL1A | 153 | 0.22 | PFKL | 100 | 0.22 | REC8 | 117 |
| 0.37 | CHST12 | 31 | 0.46 | TRAF3IP3 | 163 | 0.30 | SYNE2 | 145 | 0.34 | LAMA5 | 75 | 0.21 | EHD1 | 43 | 0.20 | CD37 | 26 |
| 0.36 | NKG7 | 94 | 0.45 | PIK3IP1 | 103 | 0.27 | TPST2 | 162 | 0.26 | BCL11A | 14 | 0.20 | PRDM1 | 107 | 0.18 | TPK1 | 161 |
| 0.35 | SYT11 | 147 | 0.45 | ITM2C | 71 | 0.26 | RPL36 | 126 | 0.25 | TCF4 | 152 | 0.19 | IKBKG | 69 | 0.17 | GDPD5 | 54 |
| 0.34 | RARRES3 | 114 | 0.45 | LDLRAP1 | 78 | 0.26 | GALNT10 | 53 | 0.20 | DPEP2 | 41 | 0.19 | TAP1 | 149 | 0.17 | SAC3D1 | 133 |
| 0.30 | RPL12 | 121 | 0.43 | NOSIP | 95 | 0.26 | GFI1 | 55 | 0.17 | RPAIN | 118 | 0.18 | TANK | 148 | 0.13 | SPINT2 | 140 |
| 0.28 | KIF13B | 73 | 0.41 | ARHGEF18 | 9 | 0.25 | MZF1 | 87 | 0.15 | RAB3GAP1 | 111 | 0.17 | PARP3 | 98 | 0.10 | CD93 | 30 |
| 0.28 | ICAM2 | 65 | 0.40 | SATB1 | 134 | 0.24 | SREBF1 | 142 | 0.14 | MED25 | 84 | 0.16 | OASL | 96 | | | |
| 0.27 | ADRB2 | 5 | 0.40 | ITPKB | 72 | 0.21 | AKAP11 | 7 | 0.07 | LRMP | 80 | 0.16 | ADCY3 | 3 | | | |
| 0.25 | MRPS18B | 86 | 0.40 | TBC1D4 | 150 | 0.21 | CAMK1D | 20 | | | | 0.16 | FAM129A | 49 | | | |
| 0.25 | RPL8 | 128 | 0.40 | FLT3LG | 52 | 0.18 | CYB561 | 36 | | | | 0.16 | SQRDL | 141 | | | |
| 0.22 | RPS11 | 129 | 0.39 | DHRS3 | 39 | 0.17 | BTG3 | 17 | | | | 0.16 | UBR2 | 167 | | | |
| 0.22 | SLAMF7 | 138 | 0.39 | RBM4B | 116 | 0.15 | CST7 | 35 | | | | 0.15 | IFITM1 | 68 | | | |
| 0.21 | PRKD2 | 108 | 0.37 | HSD17B8 | 64 | 0.15 | SVIL | 144 | | | | 0.14 | CYLD | 37 | | | |
| 0.20 | FDFT1 | 50 | 0.36 | MLLT11 | 85 | 0.13 | EIF2AK2 | 44 | | | | 0.13 | ACSL1 | 2 | | | |
| 0.20 | ATP2B4 | 12 | 0.36 | TMC6 | 156 | 0.11 | ARNTL | 10 | | | | 0.13 | PXN | 110 | | | |
| 0.19 | RPL38 | 127 | 0.35 | PLEKHB1 | 104 | 0.10 | ZBP1 | 173 | | | | 0.13 | UBE2L6 | 166 | | | |
| 0.18 | BTG1 | 16 | 0.35 | ALDOC | 8 | 0.10 | ADCY7 | 4 | | | | 0.13 | IL4R | 70 | | | |
| 0.16 | DNPEP | 40 | 0.34 | RPL22 | 123 | 0.10 | SYPL1 | 146 | | | | 0.12 | COL17A1 | 32 | | | |

| Group A | | | Group B | | | Group C | | | Group D | | | Group E | | | Group F | | |
|---|---|---|---|---|---|---|---|---|---|---|---|---|---|---|---|---|---|
| 0.16 | NAP1L1 | 90 | 0.34 | EEF1G | 42 | 0.03 | HERC6 | 62 | | | | 0.12 | TXN | 164 | | | |
| 0.12 | RABGAP1L | 112 | 0.34 | TOP2B | 160 | | | | | | | 0.10 | CD300A | 25 | | | |
| 0.11 | ACAT1 | 1 | 0.34 | BACH2 | 13 | | | | | | | 0.10 | LDLR | 77 | | | |
| | | | 0.33 | BNIP3 | 15 | | | | | | | 0.09 | TBK1 | 151 | | | |
| | | | 0.33 | ADSL | 6 | | | | | | | 0.07 | HERC5 | 61 | | | |
| | | | 0.32 | GRWD1 | 59 | | | | | | | 0.06 | TDRD7 | 154 | | | |
| | | | 0.32 | RPL10A | 119 | | | | | | | 0.05 | KPNB1 | 74 | | | |
| | | | 0.32 | TMEM134 | 157 | | | | | | | 0.03 | IFI44 | 67 | | | |
| | | | 0.32 | ETS1 | 48 | | | | | | | 0.02 | CASP4 | 21 | | | |
| | | | 0.32 | CRY2 | 34 | | | | | | | -0.01 | IFI16 | 66 | | | |
| | | | 0.31 | SERBP1 | 135 | | | | | | | | | | | | |
| | | | 0.31 | RPS5 | 130 | | | | | | | | | | | | |
| | | | 0.30 | ZW10 | 174 | | | | | | | | | | | | |
| | | | 0.30 | EIF4B | 46 | | | | | | | | | | | | |
| | | | 0.27 | PEX11B | 99 | | | | | | | | | | | | |
| | | | 0.27 | PHB2 | 102 | | | | | | | | | | | | |
| | | | 0.27 | SERPINE2 | 136 | | | | | | | | | | | | |
| | | | 0.27 | SRM | 143 | | | | | | | | | | | | |
| | | | 0.26 | LSM7 | 81 | | | | | | | | | | | | |
| | | | 0.26 | RPL15 | 122 | | | | | | | | | | | | |
| | | | 0.26 | RBM17 | 115 | | | | | | | | | | | | |
| | | | 0.26 | DGKA | 38 | | | | | | | | | | | | |
| | | | 0.26 | PTOV1 | 109 | | | | | | | | | | | | |
| | | | 0.25 | HIC2 | 63 | | | | | | | | | | | | |
| | | | 0.24 | NET1 | 92 | | | | | | | | | | | | |
| | | | 0.24 | XPC | 172 | | | | | | | | | | | | |
| | | | 0.24 | SLC5A6 | 139 | | | | | | | | | | | | |
| | | | 0.24 | SERTAD2 | 137 | | | | | | | | | | | | |

72

Group A

Group B

| | | |
|---|---|---|
| TMEM39B | 158 | 0.23 |
| RPL27 | 124 | 0.23 |
| RPL11 | 120 | 0.22 |
| C6orf48 | 19 | 0.20 |
| WDR61 | 171 | 0.19 |
| PFKP | 101 | 0.19 |
| EIF2S3 | 45 | 0.18 |
| NECAP2 | 91 | 0.18 |
| RPL30 | 125 | 0.18 |
| NACA | 89 | 0.18 |
| NGFRAP1 | 93 | 0.17 |
| RPS9 | 131 | 0.16 |
| RALA | 113 | 0.16 |
| COX4I1 | 33 | 0.15 |
| UBE2D2 | 165 | 0.14 |
| VPS35 | 170 | 0.02 |

Group C

Group D

Group E

Group F

TABLE 11

SEQUENCE ID NUMBERS, BIOMARKER GENE SYMBOLS, AND ENSEMBL TRANSCRIPT IDS

| SEQ ID DNA | Symbol | Ensembl ID | SEQ ID DNA | Symbol | Ensembl ID | SEQ ID DNA | Symbol | Ensembl ID |
|---|---|---|---|---|---|---|---|---|
| 1 | ACAT1 | ENST00000265838 | 25 | CD300A | ENST00000360141 | 49 | FAM129A | ENST00000367511 |
| 2 | ACSL1 | ENST00000281455 | 26 | CD37 | ENST00000323906 | 50 | FDFT1 | ENST00000220584 |
| 3 | ADCY3 | ENST00000260600 | 27 | CD79A | ENST00000221972 | 51 | FLNB | ENST00000295956 |
| 4 | ADCY7 | ENST00000254235 | 28 | CD79B | ENST00000006750 | 52 | FLT3LG | ENST00000597551 |
| 5 | ADRB2 | ENST00000305988 | 29 | CD8A | ENST00000283635 | 53 | GALNT10 | ENST00000297107 |
| 6 | ADSL | ENST00000623063 | 30 | CD93 | ENST00000246006 | 54 | GDPD5 | ENST00000336898 |
| 7 | AKAP11 | ENST00000025301 | 31 | CHST12 | ENST00000618655 | 55 | GFI1 | ENST00000294702 |
| 8 | ALDOC | ENST00000226253 | 32 | COL17A1 | ENST00000353479 | 56 | GNG7 | ENST00000382159 |
| 9 | ARHGEF18 | ENST00000319670 | 33 | COX4I1 | ENST00000253452 | 57 | GPR162 | ENST00000428545 |
| 10 | ARNTL | ENST00000389707 | 34 | CRY2 | ENST00000616623 | 58 | GPR56 | ENST00000567835 |
| 11 | ARSB | ENST00000264914 | 35 | CST7 | ENST00000480798 | 59 | GRWD1 | ENST00000253237 |
| 12 | ATP2B4 | ENST00000357681 | 36 | CYB561 | ENST00000360793 | 60 | GZMA | ENST00000274306 |
| 13 | BACH2 | ENST00000257749 | 37 | CYLD | ENST00000311559 | 61 | HERC5 | ENST00000264350 |
| 14 | BCL11A | ENST00000356842 | 38 | DGKA | ENST00000331886 | 62 | HERC6 | ENST00000264346 |
| 15 | BNIP3 | ENST00000368636 | 39 | DHRS3 | ENST00000616661 | 63 | HIC2 | ENST00000407464 |
| 16 | BTG1 | ENST00000256015 | 40 | DNPEP | ENST00000273075 | 64 | HSD17B8 | ENST00000454191 |
| 17 | BTG3 | ENST00000348354 | 41 | DPEP2 | ENST00000393847 | 65 | ICAM2 | ENST00000449662 |
| 18 | C1QB | ENST00000314933 | 42 | EEF1G | ENST00000329251 | 66 | IFI16 | ENST00000368131 |
| 19 | C6orf48 | ENST00000423640 | 43 | EHD1 | ENST00000320631 | 67 | IFI44 | ENST00000370747 |
| 20 | CAMK1D | ENST00000378845 | 44 | EIF2AK2 | ENST00000395127 | 68 | IFITM1 | ENST00000408968 |
| 21 | CASP4 | ENST00000444739 | 45 | EIF2S3 | ENST00000253039 | 69 | IKBKG | ENST00000611071 |
| 22 | CASZ1 | ENST00000344008 | 46 | EIF4B | ENST00000262056 | 70 | IL4R | ENST00000395762 |
| 23 | CCL5 | ENST00000603197 | 47 | EPHX2 | ENST00000521400 | 71 | ITM2C | ENST00000326427 |
| 24 | CCR7 | ENST00000246657 | 48 | ETS1 | ENST00000319397 | 72 | ITPKB | ENST00000429204 |

| SEQ ID DNA | Symbol | Ensembl ID |
|---|---|---|
| 127 | RPL38 | ENST00000311111 |
| 128 | RPL8 | ENST00000262584 |
| 129 | RPS11 | ENST00000270625 |
| 130 | RPS5 | ENST00000196551 |
| 131 | RPS9 | ENST00000616082 |
| 132 | RUNX3 | ENST00000308873 |
| 133 | SAC3D1 | ENST00000398846 |
| 134 | SATB1 | ENST00000338745 |
| 135 | SERBP1 | ENST00000370994 |
| 136 | SERPINE2 | ENST00000258405 |
| 137 | SERTAD2 | ENST00000313349 |
| 138 | SLAMF7 | ENST00000368043 |
| 139 | SLC5A6 | ENST00000310574 |
| 140 | SPINT2 | ENST00000301244 |
| 141 | SQRDL | ENST00000260324 |
| 142 | SREBF1 | ENST00000261646 |
| 143 | SRM | ENST00000376957 |
| 144 | SVIL | ENST00000375400 |
| 145 | SYNE2 | ENST00000344113 |
| 146 | SYPL1 | ENST00000011473 |
| 147 | SYT11 | ENST00000368324 |
| 148 | TANK | ENST00000259075 |
| 149 | TAP1 | ENST00000440894 |
| 150 | TBC1D4 | ENST00000377636 |
| 151 | TBK1 | ENST00000331710 |
| 152 | TCF4 | ENST00000356073 |
| 153 | TCL1A | ENST00000402399 |

| SEQ ID DNA | Symbol | Ensembl ID |
|---|---|---|
| 100 | PFKL | ENST00000349048 |
| 101 | PFKP | ENST00000381125 |
| 102 | PHB2 | ENST00000535923 |
| 103 | PIK3IP1 | ENST00000215912 |
| 104 | PLEKHB1 | ENST00000354190 |
| 105 | POP5 | ENST00000357500 |
| 106 | PPP2R2B | ENST00000394414 |
| 107 | PRDM1 | ENST00000369096 |
| 108 | PRKD2 | ENST00000433867 |
| 109 | PTOV1 | ENST00000391842 |
| 110 | PXN | ENST00000424649 |
| 111 | RAB3GAP1 | ENST00000264158 |
| 112 | RABGAP1L | ENST00000251507 |
| 113 | RALA | ENST00000005257 |
| 114 | RARRES3 | ENST00000255688 |
| 115 | RBM17 | ENST00000379888 |
| 116 | RBM4B | ENST00000310046 |
| 117 | REC8 | ENST00000611366 |
| 118 | RPAIN | ENST00000381209 |
| 119 | RPL10A | ENST00000322203 |
| 120 | RPL11 | ENST00000374550 |
| 121 | RPL12 | ENST00000361436 |
| 122 | RPL15 | ENST00000611050 |
| 123 | RPL22 | ENST00000234875 |
| 124 | RPL27 | ENST00000253788 |
| 125 | RPL30 | ENST00000287038 |
| 126 | RPL36 | ENST00000394580 |

| SEQ ID DNA | Symbol | Ensembl ID |
|---|---|---|
| 73 | KIF13B | ENST00000524189 |
| 74 | KPNB1 | ENST00000290158 |
| 75 | LAMA5 | ENST00000252999 |
| 76 | LBH | ENST00000395323 |
| 77 | LDLR | ENST00000558518 |
| 78 | LDLRAP1 | ENST00000374338 |
| 79 | LEF1 | ENST00000265165 |
| 80 | LRMP | ENST00000354454 |
| 81 | LSM7 | ENST00000252622 |
| 82 | LTBP3 | ENST00000322147 |
| 83 | MAL | ENST00000309988 |
| 84 | MED25 | ENST00000312865 |
| 85 | MLLT11 | ENST00000368921 |
| 86 | MRPS18B | ENST00000259873 |
| 87 | MZF1 | ENST00000599369 |
| 88 | NAALADL1 | ENST00000358658 |
| 89 | NACA | ENST00000356769 |
| 90 | NAP1L1 | ENST00000261182 |
| 91 | NECAP2 | ENST00000337132 |
| 92 | NET1 | ENST00000380359 |
| 93 | NGFRAP1 | ENST00000372635 |
| 94 | NKG7 | ENST00000221978 |
| 95 | NOSIP | ENST00000596358 |
| 96 | OASL | ENST00000257570 |
| 97 | PABPC4 | ENST00000372857 |
| 98 | PARP3 | ENST00000431474 |
| 99 | PEX11B | ENST00000369306 |

| SEQ ID DNA | Symbol | Ensembl ID |
|---|---|---|
| 154 | TDRD7 | ENST00000355295 |
| 155 | TGFBR3 | ENST00000212355 |
| 156 | TMC6 | ENST00000322914 |
| 157 | TMEM134 | ENST00000308022 |
| 158 | TMEM39B | ENST00000336294 |
| 159 | TMUB2 | ENST00000357984 |
| 160 | TOP2B | ENST00000435706 |

| SEQ ID DNA | Symbol | Ensembl ID |
|---|---|---|
| 161 | TPK1 | ENST00000360057 |
| 162 | TPST2 | ENST00000338754 |
| 163 | TRAF3IP3 | ENST00000367025 |
| 164 | TXN | ENST00000374517 |
| 165 | UBE2D2 | ENST00000398733 |
| 166 | UBE2L6 | ENST00000287156 |
| 167 | UBR2 | ENST00000372899 |

| SEQ ID DNA | Symbol | Ensembl ID |
|---|---|---|
| 168 | VASH1 | ENST00000167106 |
| 169 | VPREB3 | ENST00000248948 |
| 170 | VPS35 | ENST00000299138 |
| 171 | WDR61 | ENST00000267973 |
| 172 | XPC | ENST00000285021 |
| 173 | ZBP1 | ENST00000371173 |
| 174 | ZW10 | ENST00000200135 |

## TABLE 12

### SEQUENCE ID NUMBERS, BIOMARKER GENE SYMBOLS, AND DATABASE ACCESSION IDS

| SEQ ID AA | Symbol | Accession | SEQ ID AA | Symbol | Accession | SEQ ID AA | Symbol | Accession |
|---|---|---|---|---|---|---|---|---|
| 175 | ACAT1 | NP_000010 | 199 | CD300A | NP_009192 | 223 | FAM129A | NP_443198 |
| 176 | ACSL1 | NP_001986 | 200 | CD37 | NP_001765 | 224 | FDFT1 | NP_004453 |
| 177 | ADCY3 | NP_004027 | 201 | CD79A | NP_001774 | 225 | FLNB | NP_001448 |
| 178 | ADCY7 | NP_001105 | 202 | CD79B | NP_000617 | 226 | FLT3LG | NP_001450 |
| 179 | ADRB2 | NP_000015 | 203 | CD8A | NP_001759 | 227 | GALNT10 | NP_938080 |
| 180 | ADSL | NP_000017 | 204 | CD93 | NP_036204 | 228 | GDPD5 | NP_110419 |
| 181 | AKAP11 | NP_057332 | 205 | CHST12 | NP_061111 | 229 | GFI1 | NP_005254 |
| 182 | ALDOC | NP_005156 | 206 | COL17A1 | NP_000485 | 230 | GNG7 | NP_443079 |
| 183 | ARHGEF18 | NP_056133 | 207 | COX4I1 | NP_001852 | 231 | GPR162 | NP_055264 |
| 184 | ARNTL | NP_001169 | 208 | CRY2 | NP_066940 | 232 | GPR56 | NP_005673 |
| 185 | ARSB | NP_000037 | 209 | CST7 | NP_003641 | 233 | GRWD1 | NP_113673 |
| 186 | ATP2B4 | NP_001675 | 210 | CYB561 | NP_001906 | 234 | GZMA | NP_006135 |
| 187 | BACH2 | NP_068585 | 211 | CYLD | NP_056062 | 235 | HERC5 | NP_057407 |
| 188 | BCL11A | NP_060484 | 212 | DGKA | NP_001336 | 236 | HERC6 | NP_060382 |
| 189 | BNIP3 | NP_004043 | 213 | DHRS3 | NP_004744 | 237 | HIC2 | NP_055909 |
| 190 | BTG1 | NP_001722 | 214 | DNPEP | NP_036232 | 238 | HSD17B8 | NP_055049 |
| 191 | BTG3 | NP_006797 | 215 | DPEP2 | NP_071750 | 239 | ICAM2 | NP_000864 |
| 192 | C1QB | NP_000482 | 216 | EEF1G | NP_001395 | 240 | IFI16 | NP_005522 |
| 193 | C6orf48 | NP_001035527 | 217 | EHD1 | NP_006786 | 241 | IFI44 | NP_006408 |
| 194 | CAMK1D | NP_065130 | 218 | EIF2AK2 | NP_002750 | 242 | IFITM1 | NP_003632 |
| 195 | CASP4 | NP_001216 | 219 | EIF2S3 | NP_001406 | 243 | IKBKG | NP_003630 |
| 196 | CASZ1 | NP_060236 | 220 | EIF4B | NP_001408 | 244 | IL4R | NP_000409 |
| 197 | CCL5 | NP_002976 | 221 | EPHX2 | NP_001970 | 245 | ITM2C | NP_112188 |
| 198 | CCR7 | NP_001829 | 222 | ETS1 | NP_005229 | 246 | ITPKB | NP_002212 |

| SEQ ID AA | Symbol | Accession |
|---|---|---|
| 247 | KIF13B | NP_056069 |
| 248 | KPNB1 | NP_002256 |
| 249 | LAMA5 | NP_005551 |
| 250 | LBH | NP_112177 |
| 251 | LDLR | NP_000518 |
| 252 | LDLRAP1 | NP_056442 |
| 253 | LEF1 | NP_057353 |
| 254 | LRMP | NP_006143 |
| 255 | LSM7 | NP_057283 |
| 256 | LTBP3 | NP_066548 |
| 257 | MAL | NP_002362 |
| 258 | MED25 | NP_112235 |
| 259 | MLLT11 | NP_006809 |
| 260 | MRPS18B | NP_054765 |
| 261 | MZF1 | NP_003413 |
| 262 | NAALADL1 | NP_005459 |
| 263 | NACA | NP_001106673 |
| 264 | NAP1L1 | NP_004528 |
| 265 | NECAP2 | NP_060560 |
| 266 | NET1 | NP_005854 |
| 267 | NGFRAP1 | NP_055195 |
| 268 | NKG7 | NP_005592 |
| 269 | NOSIP | NP_057037 |
| 270 | OASL | NP_003724 |
| 271 | PABPC4 | NP_003810 |
| 272 | PARP3 | NP_005476 |
| 273 | PEX11B | NP_003837 |
| 274 | PFKL | NP_002617 |

| SEQ ID AA | Symbol | Accession |
|---|---|---|
| 275 | PFKP | NP_002618 |
| 276 | PHB2 | NP_001138303 |
| 277 | PIK3IP1 | NP_443112 |
| 278 | PLEKHB1 | NP_067023 |
| 279 | POP5 | NP_057002 |
| 280 | PPP2R2B | NP_858060 |
| 281 | PRDM1 | NP_001189 |
| 282 | PRKD2 | NP_057541 |
| 283 | PTOV1 | NP_059128 |
| 284 | PXN | NP_002850 |
| 285 | RAB3GAP1 | NP_036365 |
| 286 | RABGAP1L | NP_055672 |
| 287 | RALA | NP_005393 |
| 288 | RARRES3 | NP_004576 |
| 289 | RBM17 | NP_116294 |
| 290 | RBM4B | NP_113680 |
| 291 | REC8 | NP_005123 |
| 292 | RPAIN | NP_001028174 |
| 293 | RPL10A | NP_009035 |
| 294 | RPL11 | NP_000966 |
| 295 | RPL12 | NP_000967 |
| 296 | RPL15 | NP_002939 |
| 297 | RPL22 | NP_000974 |
| 298 | RPL27 | NP_000979 |
| 299 | RPL30 | NP_000980 |
| 300 | RPL36 | NP_056229 |
| 301 | RPL38 | NP_000990 |
| 302 | RPL8 | NP_000964 |

| SEQ ID AA | Symbol | Accession |
|---|---|---|
| 303 | RPS11 | NP_001006 |
| 304 | RPS5 | NP_001000 |
| 305 | RPS9 | NP_001004 |
| 306 | RUNX3 | NP_004341 |
| 307 | SAC3D1 | NP_037431 |
| 308 | SATB1 | NP_002962 |
| 309 | SERBP1 | NP_056455 |
| 310 | SERPINE2 | NP_006207 |
| 311 | SERTAD2 | NP_055570 |
| 312 | SLAMF7 | NP_067004 |
| 313 | SLC5A6 | NP_066918 |
| 314 | SPINT2 | NP_066925 |
| 315 | SQRDL | NP_067022 |
| 316 | SREBF1 | NP_004167 |
| 317 | SRM | NP_003123 |
| 318 | SVIL | NP_003165 |
| 319 | SYNE2 | NP_055995 |
| 320 | SYPL1 | NP_006745 |
| 321 | SYT11 | NP_689493 |
| 322 | TANK | NP_004171 |
| 323 | TAP1 | NP_000584 |
| 324 | TBC1D4 | NP_055647 |
| 325 | TBK1 | NP_037386 |
| 326 | TCF4 | NP_003190 |
| 327 | TCL1A | NP_068801 |
| 328 | TDRD7 | NP_055105 |
| 329 | TGFBR3 | NP_003234 |
| 330 | TMC6 | NP_009198 |

| SEQID AA | Symbol | Accession | SEQID AA | Symbol | Accession | SEQID AA | Symbol | Accession |
|---|---|---|---|---|---|---|---|---|
| 331 | TMEM134 | NP_079400 | 337 | TRAF3IP3 | NP_079504 | 343 | VPREB3 | NP_037510 |
| 332 | TMEM39B | NP_060526 | 338 | TXN | NP_003320 | 344 | VPS35 | NP_060676 |
| 333 | TMUB2 | NP_077012 | 339 | UBE2D2 | NP_003330 | 345 | WDR61 | NP_079510 |
| 334 | TOP2B | NP_001059 | 340 | UBE2L6 | NP_004214 | 346 | XPC | NP_004619 |
| 335 | TPK1 | NP_071890 | 341 | UBR2 | NP_056070 | 347 | ZBP1 | NP_110403 |
| 336 | TPST2 | NP_003586 | 342 | VASH1 | NP_055724 | 348 | ZW10 | NP_004715 |

**Claims**

1. A method for determining an indicator used in assessing a likelihood of a subject having a presence, absence or degree of herpesvirus-associated systemic inflammatory response syndrome (HVaSIRS), wherein the subject has at least one clinical sign of SIRS, the method comprising: (a) determining a pair of biomarker values, each biomarker value being a value measured or derived for at least one corresponding HVaSIRS biomarker and that is at least partially indicative of a concentration of the HVaSIRS biomarker in a sample taken from the subject; (b) determining a derived biomarker value using the pair of biomarker values, the derived biomarker value being indicative of a ratio of concentrations of the pair of HVaSIRS biomarkers; and (c) determining the indicator using the derived biomarker value, wherein one HVaSIRS biomarker of the biomarker pair is selected from Group A HVaSIRS biomarkers and the other is selected from Group B HVaSIRS biomarkers, wherein an individual Group A HVaSIRS biomarker is an expression product of a gene selected from the group consisting of: *CCL5, GZMA, TGFBR3, CD8A, PABPC4, CHST12, NKG7, SYT11, RARRES3, RPL12, KIF13B, ICAM2, ADRB2, MRPS18B, RPL8, RPS11, SLAMF7, PRKD2, FDFT1, ATP2B4, RPL38, BTG1, DNPEP, NAP1L1, RABGAP1L and ACAT1* and wherein an individual Group B HVaSIRS biomarker is an expression product of a gene selected from the group consisting of: *FLNB, EPHX2, CCR7, MAL, LEF1, TRAF3IP3, PIK3IP1, ITM2C, LDLRAP1, NOSIP, ARHGEF18, SATB1, ITPKB, TBC1D4, FLT3LG, DHRS3, RBM4B, HSD17B8, MLLT11, TMC6, PLEKHB1, ALDOC, RPL22, EEF1G, TOP2B, BACH2, BNIP3, ADSL, GRWD1, RPL10A, TMEM134, ETS1, CRY2, SERBP1, RPS5, ZW10, EIF4B, PEX11B, PHB2, SERPINE2, SRM, LSM7, RPL15, RBM17, DGKA, PTOV1, HIC2, NET1, XPC, SLC5A6, SERTAD2, TMEM39B, RPL27, RPL11, C6orf48, WDR61, PFKP, EIF2S3, NECAP2, RPL30, NACA, NGFRAP1, RPS9, RALA, COX4I1, UBE2D2 and VPS35.*

2. A method according to claim 1, wherein the sample is selected from a leukocyte sample and a blood sample, preferably a peripheral blood sample.

3. A method according to claim 1 or claim 2, comprising: (a) determining a first derived biomarker value using a first pair of biomarker values, the first derived biomarker value being indicative of a ratio of concentrations of first and second HVaSIRS biomarkers; (b) determining a second derived biomarker value using a second pair of biomarker values, the second derived biomarker value being indicative of a ratio of concentrations of third and fourth HVaSIRS biomarkers; (c) determining a third derived biomarker value using a third pair of biomarker values, the third derived biomarker value being indicative of a ratio of concentrations of fifth and sixth HVaSIRS biomarkers, wherein the first HVaSIRS biomarker is selected from Group A HVaSIRS biomarkers, the second HVaSIRS biomarker is selected from Group B HVaSIRS biomarkers, the third HVaSIRS biomarker is selected from Group C HVaSIRS biomarkers, the fourth HVaSIRS biomarker is selected from Group D HVaSIRS biomarkers, the fifth HVaSIRS biomarker is selected from Group E HVaSIRS biomarkers, and the sixth HVaSIRS biomarker is selected from Group F HVaSIRS biomarkers, wherein an individual Group C HVaSIRS biomarker is an expression product of a gene selected from the group consisting of: *PPP2R2B, RUNX3, GPR56, LBH, POP5, SYNE2, TPST2, RPL36, GALNT10, GFI1, MZF1, SREBF1, AKAP11, CAMK1D, CYB561, BTG3, CST7, SVIL, EIF2AK2, ARNTL, ZBP1, ADCY7, SYPL1 and HERC6,* wherein an individual Group D HVaSIRS biomarker is an expression product of a gene selected from the group consisting of: *GNG7, CD79B, CD79A, VPREB3, TCL1A, LAMA5, BCL11A, TCF4, DPEP2, RPAIN, RAB3GAP1, MED25 and LRMP,* wherein an individual Group E HVaSIRS biomarker is an expression product of a gene selected from the group consisting of: *CASZ1, TMUB2, ARSB, C1QB, PFKL, EHD1, PRDM1, IKBKG, TAP1, TANK, PARP3, OASL, ADCY3, FAM129A, SQRDL, UBR2, IFITM1, CYLD, ACSL1, PXN, UBE2L6, IL4R, COL17A1, TXN, CD300A, LDLR, TBK1, HERC5, TDRD7, KPNB1, IFI44, CASP4 and IFI16* and wherein an individual Group F HVaSIRS biomarker is an expression product of a gene selected from the group consisting of: *VASH1, GPR162, NAALADL1, LTBP3, REC8, CD37, TPK1, GDPD5, SAC3D1, SPINT2 and CD93;* and (d) determining the indicator by combining the first, second and third derived biomarker values.

4. A method according to claim 3, wherein the derived biomarker values are combined using a combining function, wherein the combining function is at least one of: additive model; a linear model; a support vector machine; a neural network model; a tree-learning method (*e.g.*, random forest model); a regression model; a genetic algorithm; an annealing algorithm; a weighted sum; a nearest neighbor model; an ensemble method (*e.g.*, bagging, boosting weighted averaging); and a probabilistic model.

5. A method according to claim 3 or claim 4, wherein the Group A HVaSIRS biomarker is an expression product of *CCL5,* the Group B HVaSIRS biomarker is an expression product of *FLNB,* the Group C HVaSIRS biomarker is an expression product of **PPP2R2B,** the Group D HVaSIRS biomarker is an expression product of *GNG7,* the Group E HVaSIRS biomarker is an expression product of *CASZ1,* and the Group F HVaSIRS biomarker is an expression product of *VASH1.*

6. A method according to any one of claims 1 to 5, wherein the virus associated with the HVaSIRS is selected from human herpesviruses 1-8 (HHV1-8) *(Herpes simplex virus* 1 and 2, Varicella Zoster *virus, Epstein-Barr virus, Cytomegalovirus, Roseolovirus, Herpes simplex virus 7,* and *Kaposi's sarcoma-associated virus).*

7. Use of an apparatus for determining an indicator used in assessing a likelihood of a subject having a presence, absence or degree of HVaSIRS, the apparatus comprises at least one electronic processing device that:

   • determines a pair of biomarker values, each biomarker value being a value measured or derived for at least one corresponding HVaSIRS biomarker of a sample taken from the subject and being at least partially indicative of a concentration of the HVaSIRS biomarker in the sample, wherein one HVaSIRS biomarker of the biomarker pair is selected from Group A HVaSIRS biomarkers and the other is selected from Group B HVaSIRS biomarkers;
   • determines a derived biomarker value using the pair of biomarker values, the derived biomarker value being indicative of a ratio of concentrations of the pair of HVaSIRS biomarkers; and
   • determines the indicator using the derived biomarker value;

   wherein the Group A HVaSIRS biomarkers and the Group B HVaSIRS biomarkers are as defined in claim 1.

8. Use of a kit in a method according to any one of claims 1 to 6, the kit comprising at least a first pair of reagents, and optionally a second pair of reagents and/or a third pair of reagents; wherein the first pair of reagents comprises (i) a reagent that allows quantification of a Group A HVaSIRS biomarker, and (ii) a reagent that allows quantification of a Group B HVaSIRS biomarker; wherein the second pair of reagents comprises: (iii) a reagent that allows quantification of a Group C HVaSIRS biomarker, and (iv) a reagent that allows quantification of a Group D HVaSIRS biomarker; and wherein the third pair of reagents comprises: (v) a reagent that allows quantification of a Group E HVaSIRS biomarker, and (vi) a reagent that allows quantification of a Group F HVaSIRS biomarker.

9. A use according to claim 8, wherein the reagents are for real-time PCR.

## Patentansprüche

1. Verfahren zur Bestimmung eines Indikators, der bei der Bewertung einer Wahrscheinlichkeit verwendet wird, dass ein Individuum eine Anwesenheit, Abwesenheit oder einen Grad von Herpesvirus-assoziiertem systemischem inflammatorischem Response-Syndrom (herpesvirus-associated systemic inflammatory response syndrome; HVaSIRS) aufweist, wobei das Individuum mindestens ein klinisches Anzeichen von SIRS aufweist, wobei das Verfahren umfasst: (a) Bestimmen eines Paares von Biomarkerwerten, wobei jeder Biomarkerwert ein Wert ist, der für mindestens einen entsprechenden HVaSIRS-Biomarker gemessen oder hergeleitet wird, und der zumindest teilweise auf eine Konzentration des HVaSIRS-Biomarkers in einer von dem Individuum entnommenen Probe hinweist; (b) Bestimmen eines hergeleiteten Biomarkerwertes unter Verwendung des Paars von Biomarkerwerten, wobei der hergeleitete Biomarkerwert auf ein Verhältnis von Konzentrationen des Paars von HVaSIRS-Biomarkern hinweist; und (c) Bestimmen des Indikators unter Verwendung des hergeleiteten Biomarkerwertes, wobei ein HVaSIRS-Biomarker des Biomarkerpaares ausgewählt ist aus Gruppe A-HVaSIRS-Biomarkern und der andere ausgewählt ist aus Gruppe B-HVaSIRS-Biomarkern, wobei ein einzelner Gruppe A-HVaSIRS-Biomarker ein Expressionsprodukt eines Gens ist, das ausgewählt ist aus der Gruppe bestehend aus: *CCL5, GZMA, TGFBR3, CD8A, PABPC4, CHST12, NKG7, SYT11, RARRES3, RPL12, KIF13B, ICAM2, ADRB2, MRPS18B, RPL8, RPS11, SLAMF7, PRKD2, FDFT1, ATP2B4, RPL38, BTG1, DNPEP, NAP1L1, RABGAP1L* und *ACAT1,* und wobei ein einzelner Gruppe B-HVaSIRS-Biomarker ein Expressionsprodukt eines Gens ist, das ausgewählt ist aus der Gruppe bestehend aus: *FLNB, EPHX2, CCR7, MAL, LEF1., TRAF3IP3, PIK3IP1, ITM2C, LDLRAP1, NOSIP, ARHGEF18, SATB1, ITPKB, TBC1D4, FLT3LG, DHRS3, RBM4B, HSD17B8, MLLT11, TMC6, PLEKHB1, ALDOC, RPL22, EEF1G, TOP2B, BACH2, BNIP3, ADSL GRWD1, RPL10A, TMEM134, ETS1, CRY2, SERBP1, RPS5, ZW10 EIF4B, PEX118, PHB2, SERₚlIVE2, SRM, LSM7, RPL15, RBM17, DGKA, PTOV1, HIC2, NET1, XPC, SLC5A6, SERTAD2, TMEM39B, RPL27, RPL11, C6orf48, WOR61, PFKP, EIF2S3, NECAP2, RPL30, NACA, NGFR4P1, RPS9, RALA, COX4I1, UBE2D2* und *VPS35.*

2. Verfahren nach Anspruch 1, wobei die Probe ausgewählt ist aus einer Leukocytenprobe und einer Blutprobe, bevorzugt einer peripheren Blutprobe.

3. Verfahren nach Anspruch 1 oder Anspruch 2, umfassend: (a) Bestimmen eines ersten hergeleiteten Biomarkerwertes unter Verwendung eines ersten Paars von Biomarkerwerten, wobei der erste hergeleitete Biomarkerwert auf ein Verhältnis von Konzentrationen der ersten und zweiten HVaSIRS -Biomarker hinweist; (b) Bestimmen eines zweiten hergeleiteten Biomarkerwertes unter Verwendung eines zweiten Paars von Biomarkerwerten, wobei der zweite hergeleitete Biomarkerwert auf ein Verhältnis von Konzentrationen der dritten und vierten HVaSIRS-Biomarker hinweist; (c) Bestimmen eines dritten hergeleiteten Biomarkerwertes unter Verwendung eines dritten Paars von Biomarkerwerten, wobei der dritte hergeleitete Biomarkerwert auf ein Verhältnis von Konzentrationen der fünften und sechsten HVaSIRS-Biomarker hinweist, wobei der erste HVaSIRS-Biomarker aus Gruppe A-HVaSIRS-Biomarkern ausgewählt ist, der zweite HVaSIRS-Biomarker aus Gruppe B-HVaSIRS-Biomarkern ausgewählt ist, der dritte HVaSIRS-Biomarker aus Gruppe C-HVaSIRS-Biomarkern ausgewählt ist, der vierte HVaSIRS-Biomarker aus Gruppe D-HVaSIRS-Biomarken ausgewählt ist, der fünfte HVaSIRS-Biomarker aus Gruppe E-HVaSIRS-Biomarkern ausgewählt ist und der sechste HVaSIRS-Biomarker aus Gruppe F-HVaSIRS-Biomarkern ausgewählt ist, wobei ein einzelner Gruppe C-HVaSIRS-Biomarker ein Expressionsprodukt eines Gens ist, das ausgewählt ist aus der Gruppe bestehend aus: **PPP2R2B,** *RUNX3, GPR56, LBH, POP5, SYNE2, TPST2, RPL36, GALNT10, GFI1, MZF1, SREBF1, AKAP11, CAMK1D, CYB561, BTG3, CST7, SVIL, EIF2AK2, ARNTL, ZBP1, ADCY7, SYPL1* und *HERC6,* wobei ein einzelner Gruppe D-HVaSIRS-Biomarker ein Expressionsprodukt eines Gens ist, das ausgewählt ist aus der Gruppe bestehend aus: *GNG7, CD79B, CD79A, VPREB3, TCL1A, LAMA5, BCL11A, TCF4, DPEP2, RPAIN, RAB3GAP1, MED25* und *LRMP,* wobei ein einzelner Gruppe E-HVaSIRS-Biomarker ein Expressionsprodukt eines Gens ist, das ausgewählt ist aus der Gruppe bestehend aus: *CASZ1, TMUB2, ARSB, C1QB, PFKL, EHD1, PRDM1, IKBKG, TAP1, TANK, PARP3, OASL, ADCY3, FAM129A, SQRDL, UBR2, IFITM1, CYLD, ACSL1, PXN, UBE2L6, IL4R, COL17A1, TXN, CD300A, LDLR, TBK1, HERC5, TDRD7, KPNB1, IFI44, CASP4* und *IFI16,* und wobei ein einzelner Gruppe F-HVaSIRS-Biomarker ein Expressionsprodukt eines Gens ist, das ausgewählt ist aus der Gruppe bestehend aus: *VASH1, GPR162, NAALADL1, LTBP3, REC8, CD37, TPK1, GDPD5, SAC3D1, SPINT2* und *CD93;* und (d) Bestimmen des Indikators durch Kombinieren der ersten, zweiten und dritten hergeleiteten Biomarkerwerte.

4. Verfahren nach Anspruch 3, wobei die hergeleiteten Biomarkerwerte unter Verwendung einer Kombinationsfunktion kombiniert werden, wobei die Kombinationsfunktion mindestens eine ist aus: ein additives Modell; ein lineares Modell; eine Support-Vektor-Maschine; ein neurales Netzwerkmodell; Tree-Learning-Verfahren (z.B. ein Random-Forest-Modell); ein Regressionsmodell; ein genetischer Algorithmus; ein Annealing-Algorithmus; eine gewichtete Summe; ein Nearest-Neighbor-Modell; ein Ensemble-Verfahren (z.B. Bagging, Boosting, gewichtete Mittelwertbildung); und ein probabilistisches Modell.

5. Verfahren nach Anspruch 3 oder Anspruch 4, wobei der Gruppe A-HVaSIRS-Biomarker ein Expressionsprodukt von *CCL5* ist, der Gruppe B-HVaSIRS-Biomarker ein Expressionsprodukt von *FLNB* ist, der Gruppe C-HVaSIRS-Biomarker ein Expressionsprodukt von *PPP2R2B* ist, der Gruppe D-HVaSIRS-Biomarker ein Expressionsprodukt von *GNG7* ist, der Gruppe E-HVaSIRS-Biomarker ein Expressionsprodukt von *CASZ1* ist und der Gruppe F-HVaSIRS-Biomarker ein Expressionsprodukt von *VASH1* ist.

6. Verfahren nach einem der Ansprüche 1 bis 5, wobei das Virus, das mit dem HVaSIRS assoziiert ist, ausgewählt ist aus humanen Herpesviren 1-8 (HHV1-8) (*Herpes Simplex Virus* 1 und 2, *Varicella Zoster Virus, Epstein-Barr Virus, Cytomegalovirus, Roseolovirus, Herpes Simplex Virus* 7 und *Kaposi's-Sarkom-assoziiertes Virus).*

7. Verwendung eines Geräts zur Bestimmung eines Indikators, der bei der Bewertung einer Wahrscheinlichkeit verwendet wird, dass ein Individuum eine Anwesenheit, Abwesenheit oder einen Grad von HVaSIRS aufweist, wobei das Gerät mindestens eine elektronische Bearbeitungsvorrichtung umfasst, die:

• ein Paar von Biomarkerwerten bestimmt, wobei jeder Biomarkerwert ein Wert ist, der für mindestens einen entsprechenden HVaSIRS-Biomarker in einer von dem Individuum entnommenen Probe gemessen oder hergeleitet wird und der zumindest teilweise auf eine Konzentration des HVaSIRS-Biomarkers in der Probe hinweist, wobei ein HVaSIRS-Biomarker des Biomarkerpaares ausgewählt ist aus Gruppe A-HVaSIRS-Biomarkern und der andere aus Gruppe B-HVaSIRS-Biomarkern ausgewählt ist;
• ein hergeleiteten Biomarker unter Verwendung des Paars von Biomarkerwerten bestimmt, wobei der hergeleitete Biomarkerwert auf ein Verhältnis von Konzentrationen des Paars von HVaSIRS-Biomarkerwerten hinweist; und
• den Indikator unter Verwendung des hergeleiteten Biomarkerwertes bestimmt;

wobei die Gruppe A-HVaSIRS-Biomarker und die Gruppe-B-HVaSIRS-Biomarker wie in Anspruch 1 definiert sind.

8. Verwendung eines Kits in einem Verfahren nach einem der Ansprüche 1 bis 6, wobei der Kit mindestens ein erstes Paar von Reagenzien und gegebenenfalls ein zweites Paar von Reagenzien und/oder ein drittes Paar von Reagenzien umfasst; wobei das erste Paar von Reagenzien umfasst: (i) ein Reagens, dass die Quantifizierung eines Gruppe A-HVaSIRS-Biomarkers ermöglicht und (ii) ein Reagens, dass die Quantifizierung eines Gruppe B-HVaSIRS-Biomarkers ermöglicht; wobei das zweite Paar von Reagenzien umfasst: (iii) ein Reagens, das die Quantifizierung eines Gruppe C-HVaSIRS-Biomarkers ermöglicht und (iv) ein Reagens, das die Quantifizierung eines Gruppe D-HVaSIRS-Biomarkers ermöglicht; und wobei das dritte Paar von Reagenzien umfasst: (v) ein Reagens, dass die Quantifizierung eines Gruppe E-HVaSIRS-Biomarkers ermöglicht und (vi) ein Reagens, dass die Quantifizierung eines Gruppe F-HVaSIRS-Biomarkers ermöglicht.

9. Verwendung nach Anspruch 8, wobei die Reagenzien für eine Echtzeit-PCR sind.

**Revendications**

1. Procédé de détermination d'un indicateur utilisé dans l'évaluation d'une probabilité pour un sujet d'avoir une présence, une absence ou un degré de syndrome de réponse inflammatoire systémique associé à un virus de l'herpès (HVaSIRS), dans lequel le sujet présente au moins un signe clinique de SIRS, le procédé comprenant : (a) la détermination d'une paire de valeurs de biomarqueur, chaque valeur de biomarqueur étant une valeur mesurée ou dérivée pour au moins un biomarqueur de HVaSIRS correspondant et qui est au moins partiellement indicative d'une concentration du biomarqueur de HVaSIRS dans un échantillon prélevé du sujet ; (b) la détermination d'une valeur de biomarqueur dérivée à l'aide de la paire de valeurs de biomarqueur, la valeur de biomarqueur dérivée étant indicative d'un rapport de concentrations de la paire de biomarqueurs de HVaSIRS ; et (c) la détermination de l'indicateur à l'aide de la valeur de biomarqueur dérivée, dans lequel un biomarqueur de HVaSIRS de la paire de biomarqueurs est choisi dans le groupe A de biomarqueurs de HVaSIRS et l'autre est choisi dans le groupe B de biomarqueurs de HVaSIRS, dans lequel un biomarqueur de HVaSIRS de groupe A individuel est un produit d'expression d'un gène choisi dans le groupe constitué par : *CCL5, GZMA, TGFBR3, CD8A, PABPC4, CHST12, NKG7, SYT11, RARRES3, RPL12, KIF13B, ICAM2, ADRB2, MRPS18B, RPL8, RPS11, SLAMF7, PRKD2, FDFT1, ATP2B4, RPL38, BTG1, DNPEP, NAP1L1, RABGAP1L et ACAT1* et dans lequel un biomarqueur de HVaSIRS de groupe B individuel est un produit d'expression d'un gène choisi dans le groupe constitué par : *FLNB, EPHX2, CCR7, MAL, LEF1, TRAF3IP3, PIK3IP1, ITM2C, LDLRAP1, NOSIP, ARHGEF18, SATB1, ITPKB, TBC1D4, FLT3LG, DHRS3, RBM4B, HSD17B8, MLLT11, TMC6, PLEKHB1, ALDOC, RPL22, EEF1G, TOP2B, BACH2, BNIP3, ADSL, GRWD1, RPL10A, TMEM134, ETS1, CRY2, SERBP1, RPS5, ZW10, EIF4B, PEX11B, PHB2, SERPINE2, SRM, LSM7, RPL15, RBM17, DGKA, PTOV1, HIC2, NET1, XPC, SLC5A6, SERTAD2, TMEM39B, RPL27, RPL11, C6orf48, WDR61, PFKP, EIF2S3, NECAP2, RPL30, NACA, NGFRAP1, RPS9, RALA, C0X4I1, UBE2D2 et VPS35.*

2. Procédé selon la revendication 1, dans lequel l'échantillon est choisi parmi un échantillon de leucocytes et un échantillon de sang, de préférence un échantillon de sang périphérique.

3. Procédé selon la revendication 1 ou la revendication 2, comprenant : (a) la détermination d'une première valeur de biomarqueur dérivée à l'aide d'une première paire de valeurs de biomarqueur, la première valeur de biomarqueur dérivée étant indicative d'un rapport de concentrations de premier et deuxième biomarqueurs de HVaSIRS ; (b) la détermination d'une deuxième valeur de biomarqueur dérivée à l'aide d'une deuxième paire de valeurs de biomarqueur, la deuxième valeur de biomarqueur dérivée étant indicative d'un rapport de concentrations de troisième et quatrième biomarqueurs de HVaSIRS ; (c) la détermination d'une troisième valeur de biomarqueur dérivée à l'aide d'une troisième paire de valeurs de biomarqueur, la troisième valeur de biomarqueur dérivée étant indicative d'un rapport de concentrations de cinquième et sixième biomarqueurs de HVaSIRS, dans lequel le premier biomarqueur de HVaSIRS est choisi parmi les biomarqueurs de HVaSIRS de groupe A, le deuxième biomarqueur de HVaSIRS est choisi parmi les biomarqueurs de HVaSIRS de groupe B, le troisième biomarqueur de HVaSIRS est choisi parmi les biomarqueurs de HVaSIRS de groupe C, le quatrième biomarqueur de HVaSIRS est choisi parmi les biomarqueurs de HVaSIRS de groupe D, le cinquième biomarqueur de HVaSIRS est choisi parmi les biomarqueurs de HVaSIRS de groupe E, et le sixième biomarqueur de HVaSIRS est choisi parmi les biomarqueurs de HVaSIRS de groupe F, dans lequel un biomarqueur de HVaSIRS de groupe C individuel est un produit d'expression d'un gène choisi dans le groupe constitué par : *PPP2R2B, RUNX3, GPR56, LBH, POP5, SYNE2, TPST2, RPL36, GALNT10, GFI1, MZF1, SREBF1, AKAP11, CAMK1D, CYB561, BTG3, CST7, SVIL, EIF2AK2, ARNTL, ZBP1, ADCY7, SYPL1 et HERC6,* dans lequel un biomarqueur de HVaSIRS de groupe D individuel est un produit d'expression d'un gène choisi dans le groupe constitué par : GNG7, CD79B, *CD79A, VPREB3, TCL1A, LAMA5, BCL11A, TCF4, DPEP2,*

*RPAIN, RAB3GAP1, MED25* et *LRMP,* dans lequel un biomarqueur de HVaSIRS de groupe E individuel est un produit d'expression d'un gène choisi dans le groupe constitué par : *CASZ1, TMUB2, ARSB, C1QB, PFKL, EHD1, PRDM1, IKBKG, TAP1, TANK, PARP3, OASL, ADCY3, FAM129A, SQRDL, UBR2, IFITM1, CYLD, ACSL1, PXN, UBE2L6, IL4R, COL17A1, TXN, CD300A, LDLR, TBK1, HERC5, TDRD7, KPNB1, IFI44, CASP4 et IFI16* et dans lequel un biomarqueur de HVaSIRS de groupe F individuel est un produit d'expression d'un gène choisi dans le groupe constitué par: *VASH1, GPR162, NAALADL1, LTBP3, REC8, CD37, TPK1, GDPD5, SAC3D1, SPINT2* et CD93; et (d) la détermination de l'indicateur par combinaison des première, deuxième et troisième valeurs de biomarqueur dérivées.

4. Procédé selon la revendication 3, dans lequel les valeurs de biomarqueur dérivées sont combinées à l'aide d'une fonction de combinaison, dans lequel la fonction de combinaison est au moins l'une parmi : un modèle additif ; un modèle linéaire ; une machine à vecteurs de support ; un modèle de réseau neuronal ; une méthode d'apprentissage par arborescence (par exemple un modèle de forêt d'arbres décisionnels) ; un modèle de régression ; un algorithme génétique ; un algorithme de recuit ; une somme pondérée ; un modèle du plus proche voisin ; une méthode d'ensemble (par exemple bagging, boosting de moyenne pondérée) ; un modèle probabiliste.

5. Procédé selon la revendication 3 ou la revendication 4, dans lequel le biomarqueur de HVaSIRS de groupe A est un produit d'expression de CCL5, le biomarqueur de HVaSIRS de groupe B est un produit d'expression de *FLNB,* le biomarqueur de HVaSIRS de groupe C est un produit d'expression de *PPP2R2B,* le biomarqueur de HVaSIRS de groupe D est un produit d'expression de *GNG7,* le biomarqueur de HVaSIRS de groupe E est un produit d'expression de *CASZ1,* et le biomarqueur de HVaSIRS de groupe F est un produit d'expression de *VASH1.*

6. Procédé selon l'une quelconque des revendications 1 à 5, dans lequel le virus associé au HVaSIRS est choisi parmi les virus herpès humains 1 à 8 (HHV1 à 8) (*virus Herpes simplex* 1 et 2, virus Varicella Zoster, *virus Epstein-Barr, Cytomegalovirus, Roseolovirus, virus Herpes simplex* 7, et *virus associé au sarcome de Kaposi).*

7. Utilisation d'un appareil de détermination d'un indicateur utilisé dans l'évaluation d'une probabilité pour un sujet d'avoir une présence, une absence ou un degré de HVaSIRS, l'appareil comprend au moins un dispositif de traitement électronique qui :

   • détermine une paire de valeurs de biomarqueur, chaque valeur de biomarqueur étant une valeur mesurée ou dérivée pour au moins un biomarqueur de HVaSIRS d'un échantillon prélevé d'un sujet et étant au moins partiellement indicative d'une concentration du biomarqueur de HVaSIRS dans l'échantillon, dans laquelle un biomarqueur de HVaSIRS de la paire de biomarqueurs est choisi dans les biomarqueurs de HVaSIRS de groupe A et l'autre est choisi dans les biomarqueurs de HVaSIRS de groupe B;
   • détermine une valeur de biomarqueur dérivée à l'aide de la paire de valeurs de biomarqueur, la valeur de biomarqueur dérivée étant indicative d'un rapport de concentrations de la paire de biomarqueurs de HVaSIRS ; et
   • détermine l'indicateur à l'aide de la valeur de biomarqueur dérivée ;

   dans laquelle les biomarqueurs de HVaSIRS de groupe A et les biomarqueurs de HVaSIRS de groupe B sont tels que définis dans la revendication 1.

8. Utilisation d'un kit dans un procédé selon l'une quelconque des revendications 1 à 6, le kit comprenant au moins une première paire de réactifs, et optionnellement une deuxième paire de réactifs et/ou une troisième paire de réactifs ; dans laquelle la première paire de réactifs comprend (i) un réactif qui permet une quantification d'un biomarqueur de HVaSIRS de groupe A, et (ii) un réactif qui permet une quantification d'un biomarqueur de HVaSIRS de groupe B ; dans laquelle la deuxième paire de réactifs comprend : (iii) un réactif qui permet une quantification d'un biomarqueur de HVaSIRS de groupe C, et (iv) un réactif qui permet une quantification d'un biomarqueur de HVaSIRS de groupe D, et dans laquelle la troisième paire de réactifs comprend : (v) un réactif qui permet une quantification d'un biomarqueur de HVaSIRS de groupe E, et (vi) un réactif qui permet une quantification d'un biomarqueur de HVaSIRS de groupe F.

9. Utilisation selon la revendication 8, dans laquelle les réactifs sont destinés à une PCR en temps réel.

FIGURE 1

FIGURE 2

FIGURE 3

FIGURE 4

FIGURE 5

FIGURE 6

FIGURE 7

FIGURE 8

**PATIENT REPORT**

**Immunexpress**

SepatiCyte® Herpes

| Patient Name | Medical Report # | Blood Collection Date | Sample ID |
|---|---|---|---|
| Jane Doe | 1000000 | 28-Jul-2014 | IXP100000 |

6
Score

0        5        10

**Probability of Herpes Infection**
>80%
73-86% (80% CI)

**Score Interpretation Bands**

| BAND | LIKELIHOOD | RATIO |
|---|---|---|
| Band 1 | Herpes <10% likely | 0.05 |
| Band 2 | Herpes >40% likely | 0.46 |
| Band 3 | Herpes >80% likely | 6 |
| Band 4 | Herpes >90% likely | 9 |

**TEST DESCRIPTION:**
The SeptiCyte® Herpes test is an aid in the diagnosis of active herpes virus infection in patients with signs and symptoms consistent with a systemic inflammatory response to herpes virus infections, including Herpes Simplex Virus (HSV1, 2), Varicella Zoster Virus (VZV), Epstein-Barr Virus (EBV), and Cytomegalovirus (CMV). Increasing values of the SeptiCyte® Herpes Score is associated with an increased likelihood that systemic inflammation is attributable to an active herpes infection (in contrast to a latent infection) and relative to other causes. The SeptiCyte® Herpes Score is a quantitative blood test that combines the results of six RNA transcripts (CCL5, FLNB, PPP2R2B, GNG7, CASZ1 and VASH2) as a single numerical Patient Score.

**TEST RESULT INFORMATION:**
The SeptiCyte® Herpes test should only be used in patients when active herpes virus infection is suspected. Individual patient values should be interpreted only in the context of all other clinical and laboratory information.

**LIMITATIONS:**
The reported probability of herpes virus infection for the the SeptiCyte® Herpes test was determined in a sample of XX European and US patients aged X – X assessed for herpes virus infection. Patients were classified by retrospective physician assessment in conjunction with specific virus detection or antibody assays (PCR, serum IgG, IgM).

**PHYSICIAN COMMENTS:**

**ACTUAL PATIENT SCORE IN RELATION TO HISTORICAL DATA**

Probability of Infection

0        3,1# 4,5# 6        10#
BAND 1    2  3      4

SeptiCyte® Score

**Immunexpress**

425 PONTIUS AVE N, SUITE 430
SEATTLE, WA 98104
P: 206.273.7975
www.immunexpress.com

## FIGURE 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2014201516 A
- WO 2015117204 A
- WO 2015121605 A
- WO 2014209238 A
- WO 2006015452 A
- US 4683195 A
- US 4683202 A
- US 4800159 A
- WO 9007641 A
- US 20070190540

- US 5143854 A, Pirrung
- US 5925525 A, Fodor
- US 20020055186
- US 20030003599
- WO 03062444 A
- WO 03077851 A
- WO 0259601 A
- WO 0239120 A
- WO 0179849 A
- WO 9939210 A

### Non-patent literature cited in the description

- **RANGEL-FRAUSTO et al.** The natural history of the systemic inflammatory response syndrome (SIRS). A prospective study. *JAMA : The Journal of the American Medical Association,* 1995, vol. 273 (2), 117-123
- **MCGOWAN et al.** Fever in hospitalized patients. With special reference to the medical service. *The American Journal of Medicine,* 1987, vol. 82 (3), 580-586
- **BOR et al.** Fever in hospitalized medical patients: characteristics and significance. *Journal of General Internal Medicine,* 1988, vol. 3 (2), 119-125
- **FINKELSTEIN et al.** Fever in pediatric primary care: occurrence, management, and outcomes. *Pediatrics,* 2000, vol. 105 (1), 260-266
- **LEVY et al.** 2001 SCCM/ESICM/ACCP/ATS/SIS International Sepsis Definitions Conference. *Critical Care Medicine,* 2003, vol. 31 (4), 1250-1256
- **MUNRO, N.** Fever in acute and critical care: a diagnostic approach. *AACN Adv Crit Care,* 2014, vol. 25, 237-248
- **MCGEOCH DJ. ; COOK S. ; DOLAN A. ; JAMIESON FE. ; TELFORD EA.** Molecular phylogeny and evolutionary timescale for the family of mammalian herpesviruses. *Journal of Molecular Biology,* 1995, vol. 247, 443
- **HILL, AB.** Mechanisms of interference with the MHC class I-restricted pathway of antigen presentation by herpesviruses. *Immunology and Cell Biology,* 1996, vol. 74, 523-526
- **ROSS SA ; NOVAK Z ; PATI S ; BOPPANA SB.** Diagnosis of Cytomegalovirus Infections. *Infectious disorders drug targets,* 2011, vol. 11 (5), 466-474

- **BUSTAMANTE, C. I. ; WADE, J. C.** Herpes simplex virus infection in the immunocompromised cancer patient. *Journal of Clinical Oncology : Official Journal of the American Society of Clinical Oncology,* 1991, vol. 9 (10), 1903-1915
- **WALTON, A. H. ; MUENZER, J. T. ; RASCHE, D. ; BOOMER, J. S. ; SATO, B. ; BROWNSTEIN, B. H. et al.** Reactivation of multiple viruses in patients with sepsis. *PLoS ONE,* 2014, vol. 9 (2), e98819
- **BLYTH, W. A. ; HARBOUR, D. A. ; HILL, T. J.** Effect of immunosuppression on recurrent herpes simplex in mice. *Infection and Immunity,* 1980, vol. 29 (3), 902-907
- **MULLER, W. ; JONES, C. ; KOELLE, D.** Immunobiology of Herpes Simplex Virus and Cytomegalovirus Infections of the Fetus and Newborn. *Current Immunology Reviews,* 2010, vol. 6 (1), 38-55
- **MOSSMAN KL.** Activation and inhibition of virus and interferon: The herpesvirus story. *Viral Immunology,* 2002, vol. 15 (1), 3-15
- The role of interferon in immunity and prophylaxis. **LEBEL F ; HIRSCH MS.** The Herpesviruses. Plenum Press, 1985, vol. 4, 371-393
- **DE VEER MJ. ; HOLKO M. ; FREVEL M. ; WALKER E. ; DER S. ; PARANJAPE JM. ; SILVERMAN RH. ; WILLIAMS BRG.** Functional classification of interferon-stimulated genes identified using microarrays. 22001. *Journal of Leukocyte Biology,* vol. 69, 912-920
- **HERRERA, V. ; PERRY, S. ; PARSONNET, J. ; BANAEI, N.** Clinical Application and Limitations of Interferon- Release Assays for the Diagnosis of Latent Tuberculosis Infection. *Clinical Infectious Diseases : An Official Publication of the Infectious Diseases Society of America,* 2011, vol. 52 (8), 1031-1037

- **YAROVINSKY, F.** Innate immunity to Toxoplasma gondii infection. *Nature Reviews Immunology,* 2014, vol. 14 (2), 109-121
- **HU, X. ; YU, J. ; CROSBY, S. D. ; STORCH, G. A.** Gene expression profiles in febrile children with defined viral and bacterial infection. *Proceedings of the National Academy of Sciences,* 2013, vol. 110 (31), 12792-12797
- **HALMINEN, M. ; ILONEN, J. ; JULKUNEN, I. ; RU-USKANEN, O. ; SIMELL, O. ; MAKELA, M. J.** Expression of MxA protein in blood lymphocytes discriminates between viral and bacterial infections in febrile children. *Pediatric Research,* 1997, vol. 41 (5), 647-650
- **NEEDLEMAN ; WÜNSCH.** *J. Mol. Biol.,* 1970, vol. 48, 444-453
- **E. MEYERS ; W. MILLER.** *Cabios,* 1989, vol. 4, 11-17
- **ALTSCHUL et al.** *J Mol Biol.,* 1990, vol. 215, 403-10
- **ALTSCHUL et al.** *Nucleic Acids Res,* 1997, vol. 25, 3389-3402
- **INNIS et al.** PCR Protocols. Academic Press, Inc, 1990
- **HIGUCHI et al.** *Biotechnology,* 1992, vol. 10, 413-417
- **HACIA et al.** *Nature Genetics,* 1996, vol. 14, 441-447
- **SHOEMAKER et al.** *Nature Genetics,* 1996, vol. 14, 450-456
- **PEASE et al.** *Proc. Natl. Acad. Sci. U.S.A.,* 1994, vol. 91, 5022-5026
- **FODOR et al.** *Science,* 1991, vol. 251, 767-773
- NUCLEIC ACID HYBRIDIZATION, A PRACTICAL APPROACH. IRL press, 1985
- **HARRIS et al.** *Science,* 2008, vol. 320, 106-109
- **MARGULIES et al.** *Nature,* 2005, vol. 437, 376-380
- **SONI ; MELLER.** *Clin Chem,* 2007, vol. 53, 1996-2001
- **GE.** *Nucleic Acids Res.,* 2000, vol. 28 (2), e3
- **LOPEZ et al.** *J. Chromatogram. B,* 2003, vol. 787, 19-27
- **CAHILL.** *Trends in Biotechnology,* 2000, vol. 7, 47-51
- **TROENDLE ATKINS, J. ; DEMMLER, G. J. ; WILLIAMSON, W. D. ; MCDONALD, J. M. ; ISTAS, A. S. ; BUFFONE, G. J.** Polymerase chain reaction to detect cytomegalovirus DNA in the cerebrospinal fluid of neonates with congenital infection. *The Journal of Infectious Diseases,* 1994, vol. 169 (6), 1334-1337
- **REVELLO, M. G. ; FURIONE, M. ; ROGNONI, V. ; AROSSA, A. ; GERNA, G.** Cytomegalovirus DNAemia in pregnant women. *Journal of Clinical Virology : The Official Publication of the Pan American Society for Clinical Virology,* 2014, vol. 61 (4), 590-592
- What is the Role of Respiratory Viruses in Community-Acquired Pneumonia?. *Infectious Disease Clinics of North America,* 2013, vol. 27, 157-175
- **BRUN-BUISSON C.** The epidemiology of the systemic inflammatory response. *Intensive Care Med,* 2000, vol. 26 (1), S64-S74
- **CONSTET P ; STORGAARD M ; LASSEN AT.** The Systemic Inflammatory Response Syndrome (SIRS) in acutely hospitalised medical patients: a cohort study. *Scand J Trauma Resusc Emerg Med,* 2009, vol. 17, 67
- **WALTON, A. H. ; MUENZER, J. T. ; RASCHE, D. ; BOOMER, J. S. ; SATO, B.** Reactivation of multiple viruses in patients with sepsis. *PLoS ONE,* 2014
- **RIVERS EP.** Point: Adherence to Early Goal-Directed Therapy: Does It Really Matter? Yes. After a Decade, the Scientific Proof Speaks for Itself. *Chest,* 2010, vol. 138, 476-480
- **VERBOON-MACIOLEK, M. A. ; KREDIET, T. G. ; GERARDS, L. J. ; FLEER, A. ; VAN LOON, T. M.** Clinical and epidemiologic characteristics of viral infections in a neonatal intensive care unit during a 12-year period. *The Pediatric Infectious Disease Journal,* 2005, vol. 24 (10), 901-904
- **MCGOWAN JEJ ; ROSE RC ; JACOBS NF ; SCHABERG DR ; HALEY RW.** Fever in hospitalized patients. With special reference to the medical service. *Am J Med,* 1987, vol. 82, 580-586
- **NISKA R ; BHUIYA F ; XU J.** National hospital ambulatory medical care survey: 2007 emergency department summary. *Natl Health Stat Report,* 2010, vol. 26, 1-31
- **LIMPER M ; EEFTINCK SCHATTENKERK D ; DE KRUIF MD ; VAN WISSEN M ; BRANDJES DPM et al.** One-year epidemiology of fever at the Emergency Department. *Neth J Med,* 2011, vol. 69, 124-128
- **SANTAMARIA, C. ; URUENA, A. ; VIDELA, C. ; SUAREZ, A. ; GANDUGLIA, C. ; CARBALLAL, G. et al.** Epidemiological study of influenza virus infections in young adult outpatients from Buenos Aires, Argentina. *Influenza and Other Respiratory Viruses,* 2008, vol. 2 (4), 131-134
- **BOURGEOIS, F. T. ; VALIM, C. ; WEI, J. C. ; MCADAM, A. J. ; MANDL, K. D.** Influenza and other respiratory virus-related emergency department visits among young children. *Pediatrics,* 2006, vol. 118 (1), e1-8

- **ADCOCK, P. M. ; STOUT, G. G. ; HAUCK, M. A. ; MARSHALL, G. S.** Effect of rapid viral diagnosis on the management of children hospitalized with lower respiratory tract infection. *The Pediatric Infectious Disease Journal,* 1997, vol. 16 (9), 842-846
- **WALTON AH ; MUENZER JT ; RASCHE D ; BOOMER JS ; SATO B et al.** Reactivation of multiple viruses in patients with sepsis. *PLoS ONE,* 2014, vol. 9, e98819
- **ANDERSEN, H. K. ; E. S. SPENCER.** Cytomegalovirus infection among renal allograft recipients. *Acta Med. Scand.,* 1969, vol. 186, 7-19
- **BUSTAMANTE CI ; WADE JC.** Herpes simplex virus infection in the immunocompromised cancer patient. *J Clin Oncol,* 1991, vol. 9, 1903-1915
- **LJUNGMAN P ; GRIFFITHS P ; PAYA C.** Definitions of cytomegalovirus infection and disease in transplant recipients. *Clin. Infect. Dis.,* 2002, vol. 34 (8), 1094-1097
- **FISCHER, J. E. ; HARBARTH, S. ; AGTHE, A. G. ; BENN, A. ; RINGER, S. A. ; GOLDMANN, D. A. ; FANCONI, S.** Quantifying uncertainty: physicians' estimates of infection in critically ill neonates and children. *Clinical Infectious Diseases : An Official Publication of the Infectious Diseases Society of America,* 2004, vol. 38 (10), 1383-1390
- **BRAYKOV, N. P. ; MORGAN, D. J. ; SCHWEIZER, M. L. ; USLAN, D. Z. ; KELESIDIS, T. ; WEISENBERG, S. A. et al.** Assessment of empirical antibiotic therapy optimisation in six hospitals: an observational cohort study. *The Lancet Infectious Diseases,* 2014, vol. 14 (12), 1220-1227
- **DE VRIES RD ; MESMAN AW ; GEIJTENBEEK TBH ; DUPREX WP ; DE SWART RL.** The pathogenesis of measles. *Curr Opin Virol,* 2012, vol. 2, 248-255
- **ROHWEDDER A ; KEMINER O ; FORSTER J ; SCHNEIDER K ; SCHNEIDER E et al.** Detection of respiratory syncytial virus RNA in blood of neonates by polymerase chain reaction. *J Med Virol,* 1998, vol. 54, 320-327
- **WOOTTON SH ; AGUILERA EA ; WANGER A ; JEWELL A ; PATEL K et al.** Detection of NH1N1 influenza virus in nonrespiratory sites among children. *Pediatr Infect Dis J,* 2014, vol. 33, 95-96
- **PRIPUZOVA N ; WANG R ; TSAI S ; LI B ; HUNG G-C et al.** Development of Real-Time PCR Array for Simultaneous Detection of Eight Human Blood-Borne Viral Pathogens. *PLoS ONE,* 2012, vol. 7, e43246
- **JOHNSON G ; NELSON S ; PETRIC M ; TELLIER R.** Comprehensive PCR-based assay for detection and species identification of human herpesviruses. *Journal of Clinical Microbiology,* 2000, vol. 38, 3274-3279
- **XATZIPSALTI M ; KYRANA S ; TSOLIA M ; PSARRAS S ; BOSSIOS A et al.** Rhinovirus viremia in children with respiratory infections. *American Journal of Respiratory and Critical Care Medicine,* 2005, vol. 172, 1037-1040
- **DUNN JJ ; MILLER MB.** Emerging respiratory viruses other than influenza. *Clin Lab Med,* 2014, vol. 34, 409-430
- **EVANDER M ; ERIKSSON I ; PETTERSSON L ; JUTO P ; AHLM C et al.** Puumala Hantavirus Viremia Diagnosed by Real-Time Reverse Transcriptase PCR Using Samples from Patients with Hemorrhagic Fever and Renal Syndrome. *Journal of Clinical Microbiology,* 2007, vol. 45, 2491-2497
- **CHENG H-Y ; HUANG Y-C ; YEN T-Y ; HSIA S-H ; HSIEH Y-C et al.** The correlation between the presence of viremia and clinical severity in patients with enterovirus 71 infection: a multi-center cohort study. *BMC Infect Dis,* 2014, vol. 14, 417
- **ABED Y ; BOIVIN G.** Human parechovirus infections in Canada. *Emerging Infectious Diseases,* 2006, vol. 12, 969
- **ERARD V ; STORER B ; COREY L ; NOLLKAMPER J ; HUANG M-L et al.** BK virus infection in hematopoietic stem cell transplant recipients: frequency, risk factors, and association with postengraftment hemorrhagic cystitis. *Clin Infect Dis,* 2004, vol. 39, 1861-1865
- **GERKOWICZ T ; SZAJNER-MILART I ; SZCZYGIELSKA J ; BLASZYNSKA M ; KARSKA M et al.** Clinical manifestations of viremia during infections caused by adenoviruses and parainfluenza viruses. *Pediatr Pol,* 1979, vol. 54, 41-45
- **WANG WK ; FANG CT ; CHEN HL ; YANG CF ; CHEN YC et al.** Detection of Severe Acute Respiratory Syndrome Coronavirus RNA in Plasma during the Course of Infection. *Journal of Clinical Microbiology,* 2005, vol. 43, 962-965
- **CLEM AL ; SIMS J ; TELANG S ; EATON JW ; CHESNEY J.** Virus detection and identification using random multiplex (RT)-PCR with 3'-locked random primers. *Virol J,* 2007, vol. 4, 65
- **JUHL D ; STEPPAT D ; GÖRG S ; HENNIG H.** Parvovirus B19 Infections and Blood Counts in Blood Donors. *Transfus Med Hemother,* 2014, vol. 41, 6-6
- **LA CRUZ HERNANDEZ DE SI ; FLORES-AGUILAR H ; GONZALEZ-MATEOS S ; LOPEZ-MARTINEZ I ; ORTIZ-NAVARRETE V et al.** Viral load in patients infected with dengue is modulated by the presence of anti-dengue IgM antibodies. *J Clin Virol,* 2013, vol. 58, 258-261